# EUROPEAN PATENT APPLICATION

(11) **EP 4 375 270 A1**
(43) Date of publication of application: **29.05.2024**
(21) Application number: 22845430.2
(22) Date of filing: 22.07.2022
(51) Int. Cl.: C07C 233/47, C08G 83/00, C07C 231/02

(54) **SUPRAMOLECULAR AMINO ACID OR SALT THEREOF, AND PREPARATION METHOD THEREFOR AND APPLICATION THEREOF**

(30) Priority: 23.07.2021 CN 202110837472
(71) Applicant: Suzhou Oulit Biopharm Co., Ltd., Suzhou, Jiangsu 215127 (CN)
(72) Inventor: ZHANG, Jian, Suzhou, Jiangsu 215127 (CN)
(74) Representative: Winger
(86) International application number: PCT/CN2022/107270
(87) International publication number: WO 2023/001267

(57) **Abstract**

A preparation method for an N-long-chain acyl amino acid dipeptide, supramolecular amino acid and corresponding salts thereof, capable of controlling the occurrence of structural reconstruction and controlling the content of long-chain fatty acid. Further provided are a supramolecular amino acid and salts thereof, as well as an application in the fields of daily chemicals and the like.

## Description

### TECHNICAL FIELD

The present invention relates to the technical field of amino acid-based surfactant preparation, and particularly, to an amino acid supramolecule or a salt thereof, a method for preparing same, and use thereof.

### BACKGROUND

Surfactants compose an essential part in many fields such as chemistry, agriculture, pharmaceuticals, and the like. There are dozens of surfactants currently used in the market, among which sodium dodecylbenzenesulfonate (SLS), sodium lauryl polyoxyethylene ether sulfate (AES), and sodium lauryl sulfate (K12) are commonly used. Since the three prominent surfactants have been used for decades or even hundreds of years, their negative effects have been gradually revealed during the use, and such effects on health and the environment are frequently reported.

Other surfactants include saccharide surfactants such as alkyl polyglycosides (APGs), and amino acid surfactants such as lauroyl-L-glutamic acid, lauroyl glycine, lauroyl sarcosine, and the like. Although they are biological substance-based surfactants possessing high safety, good biodegradability and excellent skin feel and are receiving increasing attention, such surfactants are rarely used as a primary surfactant due to their moderate detergency. They are often used in combination with other primary surfactants, and fail to solve the safety and biodegradability problems of household surfactant products.

Some studies on long-chain acyl amino acids such as lauroyl alanine and the like have been reported. In terms of the synthesis process, the reaction of fatty acid chloride with the amino group of amino acids is the main pathway of industrial production at present. This reaction is also known as the Schotten-Baumann reaction, and a classical process of this reaction may be accompanied by several side reactions, such as hydrolysis of the fatty acid chloride, anhydride formation, etc.

In one aspect, higher fatty acids produced by hydrolysis of the acid chloride in this process have a structure close to that of the long-chain acyl amino acid product and little difference in length of the carbon chain, and can hardly be removed efficiently and cost-efficiently by existing separation methods. The presence of higher fatty acid impurities may affect the product quality, and the removal or reduction of such impurities has an important significance.

"Study on Synthesis and Properties of Sodium Lauroyl Alanine", Chen Lili, et al., Textile Auxiliaries, Vol. 26, No. 4, April 2009, reports a detailed study on the synthesis conditions, and states that lauroyl chloride is prone to hydrolysis, and various conditions such as solvent, pH, reaction temperature, etc. have certain influence on the hydrolysis of lauroyl chloride. The yield was about 85% despite the optimization of such conditions.

Previous studies have shown that the hydrolysis of long-chain acid chlorides is difficult to avoid and that long-chain fatty acids such as lauric acid and the like formed by hydrolysis are difficult to remove in Schotten-Baumann reactions. In addition, the Schotten-Baumann reaction produces various impurities other than a long-chain fatty acid, including impurities produced by the anhydride-forming reaction, acetone and its aldol reaction products such as diacetone alcohol and isopropylidene acetone, esters (when an alcohol solvent is used) and the like. Though the purity may be increased through procedures of elution, filtration, recrystallization, and the like, how to effectively and cost-effectively control the content of long-chain fatty acids is still a research orientation of interest.

In the 1990s, Kao modified the Schotten-Baumann reaction for the synthesis of N-long-chain acyl-β-alanine. β-alanine and fatty acid halide are reacted in water at 25 to 60 °C in the presence of potassium hydroxide, and the obtained salt and a strong acid are reacted at 60 to 90 °C to give the N-long-chain acyl-β-alanine. However, this method has two problems. One is that it produces N-long-chain acyl-β-alanine rather than N-long-chain acyl-L-alanine (or N-long-chain acyl α-alanine). β-alanine has no side chain, and α-alanine has a methyl side chain. The two possess different steric hindrances and require different reaction conditions. Therefore, the method may have problems when applied to amino acids having a side chain and is thus limited. The other problem is that the acid addition procedures of the method are conducted at 60 to 90 °C in water, leading to serious hydrolysis of acid chloride and difficulties in effective control. In a conventional Schotten-Baumann reaction, a lower temperature is usually selected to reduce the hydrolysis of the acid chloride.

Similarly, Ajinomoto corporation has also attempted to modify the Schotten-Baumann reaction, and has disclosed a method for preparing a detergent composition comprising an N-acyl amino acid-based anionic surfactant comprising the following steps (1) to (3): (1) reacting a halogenated fatty acid with an amino acid, (2) adding an acid to the reaction mixture and adjusting to pH 1 to 5 and a temperature of 50 to 100 °C, and (3) separating the organic layer and the aqueous layer and neutralizing the organic layer with a base. The method mainly solves the problems in desalting by the separation of the organic layer and aqueous layer. However, this method uses anionic surfactants, and also possesses the problem of acid chloride hydrolysis and difficulties in effective control.

The product prepared by the method contains a high level of higher fatty acid impurities. However, since higher fatty acids such as lauric acid do not produce peaks in a high-performance liquid chromatographic system (equipped with an ultraviolet detector), the higher fatty acid impurities are not detected by conventional detection methods, which further contributes to the unawareness of the higher fatty acid impurities of high levels in the product and errors in product purity calculation (in which the higher fatty acid impurities are accounted in the final product). Such problems have been recognized and described in, for example, CN105675749B, CN106442829B, CN106596768B, etc. However, those disclosures mainly focus on how to detect the residual higher fatty acids, and do not relate to the removal of higher fatty acids.

In another aspect, long-chain acyl amino acid dipeptides are known substances. Long-chain acyl glycyl glycine, long-chain acyl glutamyl glutamic acid, and the like have been produced, and the introduction of N-long-chain acyl amino acid dipeptides or salts thereof into detergent compositions has been attempted in the prior art. For example, CN100448968C discloses a related detergent composition. However, according to the disclosure, amino acids and lauric acid are mixed in an alkali solution, and heated in a nitrogen atmosphere at 180 °C for 1.5 hours, which requires a very high temperature and strict conditions and is thus unfavorable for industrial production. CN105683151B discloses an aqueous solution comprising an N-long-chain acyl acidic amino acid and/or a salt thereof and a method for producing same. The method, despite relatively mild conditions, requires the use of Celite^{®} and leaves large amounts of sodium chloride and sodium glutamate in the product, thus having a narrow applicability range and applying only to the production of N-long-chain acyl acidic amino acids.

Theoretically, N-long-chain acyl amino acid dipeptides or salts thereof can be prepared from an acid chloride derived from an N-long-chain acyl amino acid and an amino acid by the Schotten-Baumann reaction or the like, or by a reaction of an amino acid dipeptide (such as glutamyl glutamic acid) and an acid chloride. The above method has at least two problems, one being the cost-inefficient preparation and low yield and the other being the limited application where, for amino acid containing a side chain or having higher steric hindrance such as L-alanine, N-long-chain acyl-L-alanine dipeptides can not be easily prepared by the Schotten-Baumann reaction.

Therefore, the method for preparing a dipeptide product with high or even excellent purity with high efficiency, suitability for industrial production, and cost-efficiency, is an urgent research project.

Moreover, though amino acid-based surfactants are known, such surfactants have mild detergency and are rarely used alone as the primary surfactants. How to prepare an amino acid-based surfactant with strong detergency and how to prepare an amino acid surfactant with a novel characteristic structure are also subjects in the related art.

Prior Application Nos. CN108752228A, CN110804188A, WO2019233375A1, WO2019233377A1, etc., of the inventor describe in detail amino acid self-assembly supramolecules or salts thereof having novel characteristic structures, which are incorporated herein by reference in their entireties. The preparation processes of the prior applications demand catalysts and a reaction pressure of 5-50 kg, but give products with low dipeptide purity. Thus, the present invention will explore other solutions more suitable for industrial production.

In industrial production, a simpler method with capabilities of controlling the self-assembly, promoting structural reconstruction, and controlling the content of long-chain fatty acids and/or dipeptides (for example, lauroyl alanyl alanine in a reaction of lauroyl chloride and sodium alaninate) is desired. It is also expected to give a novel amino acid-based surfactant that has high detergency and is capable of serving as a primary surfactant.

### SUMMARY

In view of the above, in one aspect, the present invention is intended to provide a preparation method with a simple process, cost-efficiency and ease to achieve industrial production. The method can readily give an N-long-chain acyl amino acid dipeptide and/or a salt thereof (or a long-chain acyl amino acid dipeptide and/or a salt thereof) and a composition comprising same, and is capable of controlling the content of the long-chain acyl amino acid dipeptide and/or the salt thereof in the composition.

In another aspect, the present invention is intended to provide a simple method for controlling a long-chain fatty acid impurity level produced in the Schotten-Baumann reaction, or a simple method for controlling a long-chain fatty acid impurity level produced in a reaction of an amino acid with a long-chain acid halide. In this process, various water-soluble impurities such as salts, amino acids, etc., are also removed.

In yet another aspect, the present invention provides a method for separating components in a solid mixture using the difference in melting point on the basis of a principle for separating long-chain fatty acids from N-long-chain acyl amino acids.

In still another aspect, the present invention is intended to provide a simple method for preparing an amino acid supramolecule having properties different from those of existing commercially available long-chain acyl amino acids. The amino acid supramolecule or the salt thereof is an amino acid-based product prepared by the method provided by the present invention, and is defined as an amino acid supramolecule or a salt thereof according to its characteristics due to the specific structure formed under given conditions.

In the last aspect, after treatment with the specific process of the present invention, the contents of impurities (especially the long-chain fatty acid, or higher fatty acid, generally with a skeletal carbon atom number of 8-22), sodium halides (such as sodium chloride), and amino acids in the product can be controlled at extremely low levels.

Specifically, the present invention is as follows.
[1] A method for preparing an N-long-chain acyl amino acid dipeptide and/or a salt thereof or a composition comprising the N-long-chain acyl amino acid dipeptide and/or the salt thereof, comprising: reacting an amino acid and/or a salt thereof with a long-chain acid halide, wherein the pH value of the system after the reaction is less than 8, preferably 7.5 or less, more preferably 7 or less, and most preferably 5-6.5.
[2] A method for preparing an N-long-chain acyl amino acid dipeptide and/or a salt thereof or a composition comprising the N-long-chain acyl amino acid dipeptide and/or the salt thereof, comprising: reacting an amino acid and/or a salt thereof with a long-chain acid halide in the presence of a base, wherein throughout the reaction, the molar ratio of the amino acid to the base is 3:1 to 1:2, preferably 2:1 to 1:1.8, more preferably 1.7:1 to 1:1.7, and most preferably 1.5:1 to 1:1.5.
[3] The method according to item [1] or [2], wherein the long-chain acid halide is added to the amino acid and/or the salt thereof without controlling the reaction solution at an alkaline pH, preferably without controlling the reaction solution at pH 8 or greater;
   alternatively, the long-chain acid halide is added without simultaneously adding the base or controlling the dripping speed and amount of the base to maintain the pH value of the system; or alternatively, the difference between the pH values of the system before and after the addition of the long-chain acid halide is 2 or higher, preferably 3 or higher, and more preferably 4 or higher.
[4] The method according to any one of items [1]-[3], wherein after the reaction of the amino acid and/or salt thereof with the long-chain acid halide, the percentage content by weight of the N-long-chain acyl amino acid dipeptide and/or the salt thereof in the product is 3% or greater, preferably 5% or greater, more preferably 8% or greater, and most preferably 10% or greater.
[5] The method according to any one of items [1]-[4], comprising: (1) reacting a raw material comprising the amino acid and the base to give an amino acid salt solution; and (2) adding the long-chain acid halide into the resultant amino acid salt solution or adding the long-chain acid halide and the base into the resultant amino acid salt solution, wherein the method meets one or more of the following conditions:
   a, the pH value of the amino acid salt solution prepared in step (1) is 7.5-14, preferably 8-12, and more preferably 9-11, and the pH value of the system after the reaction in step (2) is less than 8, preferably 7.5 or less, more preferably 7 or less, and most preferably 5-6.5;
   b, the molar ratio of the amino acid to the base in the reaction system throughout step (1) and step (2) is 3:1 to 1:2, preferably 2:1 to 1:1.8, more preferably 1.7:1 to 1:1.7, and most preferably 1.5:1 to 1:1.5; and
   c, the pH value of the amino acid salt solution prepared in step (1) is greater than that of the system prepared after the reaction of the amino acid salt and the long-chain acid halide in step (2), and the difference between the two is 2 or greater, preferably 3 or greater, and more preferably 4 or greater.
[6] The method according to any one of items [1]-[5], wherein the reaction of the amino acid and/or the salt thereof with the long-chain acid halide meets one or more of the following conditions:
   a, the reaction is conducted in the presence of water or a mixed solution of water and a hydrophilic organic solvent; the hydrophilic organic solvent is selected from one or more of acetone, methanol, ethanol, isopropanol, sec-butyl alcohol, tert-butyl alcohol, acetonitrile, and tetrahydrofuran, and preferably acetone; preferably, the volume ratio of the water to the hydrophilic organic solvent is 1:(0-2);
   b, the temperature of the reaction is 35 °C or lower, and preferably 30 °C or lower; and
   c, the molar ratio of the amino acid and/or the salt thereof to the long-chain acid halide is greater than 1, preferably 2:1 to 1.1:1, and more preferably 1.5:1 to 1.2:1.
[7] The method according to any one of items [1]-[6], further comprising: acidifying a product prepared by the reaction of the amino acid and/or the salt thereof with the long-chain acid halide to give a crude N-long-chain acyl amino acid product; preferably, the pH after the acidification is 1 to 4, and more preferably 1 to 2.
[8] The method according to any one of items [1]-[7], wherein the method meets one or more of the following conditions:
   a, the amino acid is selected from one or more of glycine, alanine, glutamic acid, sarcosine, aspartic acid, leucine, isoleucine, valine, threonine, proline, phenylalanine, arginine, and lysine;
   b, the long-chain acyl in the long-chain acid halide is derived from a saturated or unsaturated linear or branched fatty acid with 8-22 carbon atoms; and
   c, the base is selected from one or more of sodium hydroxide, potassium hydroxide, sodium carbonate, potassium carbonate, and ammonia.
[9] The method according to item [8], wherein the method meets one or more of the following conditions:
   a, the amino acid is selected from alanine, glycine, glutamic acid, sarcosine, arginine, or lysine, and preferably L-alanine;
   b, the long-chain acid halide is selected from one or more of octanoyl chloride, caprinoyl chloride, undecanoyl chloride, lauroyl chloride, myristoyl chloride, pentadecanoyl chloride, palmitoyl chloride, stearoyl chloride, oleoyl chloride, linoleoyl chloride, isostearoyl chloride, coconut oil fatty acid chloride, or palm oil fatty acid chloride, preferably coconut oil fatty acid chloride or lauroyl chloride, and most preferably lauroyl chloride; and
   c, the base is selected from sodium hydroxide or potassium hydroxide.
[10] A method for removing an impurity from a crude N-long-chain acyl amino acid product, comprising: mixing the crude N-long-chain acyl amino acid product with a solvent, optionally stirring, and controlling the temperature T of the mixture system in a range of the melting point of a long-chain fatty acid to the melting point of the N-long-chain acyl amino acid, wherein the solvent is water, an organic solvent, or a mixed solution of water and an organic solvent; and after the temperature control in the system, separating the solid and liquid phases.
[11] The method according to item [10], wherein the solid-liquid separation is conducted under the action of centrifugation or pressure; preferably, the solid-liquid separation is promoted by using a solvent having a certain temperature as a medium that allows temperature T to be controlled in a range of the melting point of the long-chain fatty acid to the melting point of the N-long-chain acyl amino acid, and the solvent is water, an organic solvent, or a mixed solution of water and an organic solvent.
[12] The method according to item [11], wherein the solid-liquid separation meets one or more of the following conditions:
   a, during the solid-liquid separation, the solvent used as the medium is in contact with the crude product, and under the action of centrifugation or pressure, the solvent removes the impurity to promote the separation;
   b, the solvent used as the medium in the solid-liquid separation is provided by spraying;
   c, the amount of the solvent used as the medium during the solid-liquid separation is greater than 0.5 times the mass of the crude N-long-chain acyl amino acid product; and
   d, the solid-liquid separation is conducted using an industrial centrifuge or a filter press, and preferably a filter centrifuge equipped with a filter screen or a filter cloth.
[13] The method according to item [11] or [12], wherein the temperature T of the solvent as the medium is present in a plurality of temperature stages, and preferably the temperature in a next stage is equal to or higher than the temperature in a previous stage;
   preferably, the temperature in the first stage is controlled in a range of the melting point of the long-chain fatty acid to the melting point of the long-chain fatty acid + 15 °C, and the temperature in at least one subsequent stage is controlled in a range of the melting point of the long-chain fatty acid + 15 °C to the melting point of the N-long-chain acyl amino acid;
   more preferably, the temperature in the first stage is controlled in a range of the melting point of the long-chain fatty acid to the melting point of the long-chain fatty acid + 10 °C, and the temperature in at least one subsequent stage is controlled in a range of the melting point of the long-chain fatty acid + 20 °C to the melting point of the N-long-chain acyl amino acid.
[14] The method according to item [11] or [12], wherein if water is used as the solvent in the preparation of the crude N-long-chain acyl amino acid product or the content of the long-chain fatty acid impurity in the crude N-long-chain acyl amino acid product is 10 wt% or greater, the temperature T of the solvent as the medium is present in a plurality of temperature stages, and the temperature in the first stage is controlled in a range of the melting point of the long-chain fatty acid to the melting point of the long-chain fatty acid + 6 °C, and the temperature in at least one subsequent stage is controlled in a range of the melting point of the long-chain fatty acid + 15 °C to the melting point of the N-long-chain acyl amino acid;
   more preferably, the temperature in the first stage is controlled in a range of the melting point of the long-chain fatty acid to the melting point of the long-chain fatty acid + 3 °C, and the temperature in at least one subsequent stage is controlled in a range of the melting point of the long-chain fatty acid + 20 °C to the melting point of the N-long-chain acyl amino acid.
[15] The method according to item [11] or [12], wherein the temperature T of the solvent as the medium is present in a plurality of temperature stages; the temperature in the first stage is controlled at 60 °C or lower, and the temperature in at least one subsequent stage is controlled at 60 °C or higher; more preferably the temperature in the first stage is controlled at 50-60 °C, and the temperature in at least one subsequent stage is controlled at 65-70 °C.
[16] The method according to any one of items [10]-[15], wherein after the first solid-liquid separation, n solid-liquid separations are conducted, wherein n is not less than 1, and preferably the temperature in a next solid-liquid separation is equal to or higher than the temperature in a previous solid-liquid separation;
   each solid-liquid separation comprises: mixing a solid obtained from a previous solid-liquid separation with a solvent, optionally stirring, controlling the temperature Tn of the mixture system in a range of the melting point of the long-chain fatty acid to the melting point of the N-long-chain acyl amino acid, and conducting the solid-liquid separation, wherein the solvent is water, an organic solvent, or a mixed solution of water and an organic solvent; or
   alternatively: mixing a solid obtained from a previous solid-liquid separation with a solvent, optionally stirring, controlling the temperature Tn of the system in a range of the melting point of the long-chain fatty acid to the melting point of the N-long-chain acyl amino acid, and conducting the solid-liquid separation, wherein the solid-liquid separation is promoted by using a solvent having a certain temperature as a medium that allows temperature Tn to be controlled in a range of the melting point of the long-chain fatty acid to the melting point of the N-long-chain acyl amino acid, and the solvent is water, an organic solvent, or a mixed solution of water and an organic solvent.
[17] The method according to item [16], wherein the temperature T in the first solid-liquid separation is controlled in a range of the melting point of the long-chain fatty acid to the melting point of the long-chain fatty acid + 15 °C, and the temperature Tn in at least one solid-liquid separation of the n subsequent solid-liquid separations is controlled in a range of the melting point of the long-chain fatty acid + 15 °C to the melting point of the N-long-chain acyl amino acid;
   more preferably, the temperature T in the first solid-liquid separation is controlled in a range of the melting point of the long-chain fatty acid to the melting point of the long-chain fatty acid + 10 °C, and the temperature Tn in at least one solid-liquid separation of the n subsequent solid-liquid separations is controlled in a range of the melting point of the long-chain fatty acid + 20 °C to the melting point of the N-long-chain acyl amino acid.
[18] The method according to item [16], wherein three or more solid-liquid separations are conducted; the temperature T in the first solid-liquid separation is controlled in a range of the melting point of the long-chain fatty acid to the melting point of the long-chain fatty acid + 8 °C, the temperature Tn in at least one intermediate solid-liquid separation is controlled in a range of the melting point of the long-chain fatty acid + 8 °C to the melting point of the long-chain fatty acid + 18 °C, and the temperature Tn in the last solid-liquid separation is controlled in a range of the melting point of the long-chain fatty acid + 24 °C to the melting point of the N-long-chain acyl amino acid.
[19] The method according to item [16], wherein if water is used as the solvent in the preparation of the crude N-long-chain acyl amino acid product or the content of the long-chain fatty acid in the crude N-long-chain acyl amino acid product is 10% or greater, the temperature T in the first solid-liquid separation is controlled in a range of the melting point of the long-chain fatty acid to the melting point of the long-chain fatty acid + 6 °C, and the temperature Tn in at least one solid-liquid separation of the n subsequent solid-liquid separations is controlled in a range of the melting point of the long-chain fatty acid + 15 °C to the melting point of the N-long-chain acyl amino acid;
   more preferably, the temperature T in the first solid-liquid separation is controlled in a range of the melting point of the long-chain fatty acid to the melting point of the long-chain fatty acid + 3 °C, and the temperature Tn in at least one solid-liquid separation of the n subsequent solid-liquid separations is controlled in a range of the melting point of the long-chain fatty acid + 20 °C to the melting point of the N-long-chain acyl amino acid.
[20] The method according to item [16], wherein the temperature T in the first solid-liquid separation is controlled at 60 °C or lower, and the temperature Tn in at least one solid-liquid separation of the n subsequent solid-liquid separations is controlled at 60 °C or higher; more preferably, the temperature T in the first solid-liquid separation is controlled at 50-60 °C, and the temperature Tn in at least one solid-liquid separation of the n subsequent solid-liquid separations is controlled at 65-70 °C.
[21] The method according to any one of items [10]-[20], wherein the crude N-long-chain acyl amino acid product is a commercially available N-long-chain acyl amino acid product;
   alternatively, the crude N-long-chain acyl amino acid product described in any one of items [7]-[9];
   alternatively, an N-long-chain acyl amino acid product with a long-chain fatty acid percentage content by weight of 5% or greater;
   alternatively, a crude N-long-chain acyl amino acid product prepared by a method comprising: (1) reacting a raw material comprising an amino acid and a base to give an amino acid salt solution; (2) adding a long-chain acid halide and optionally a base into the resultant amino acid salt solution to give an N-long-chain acyl amino acid salt; and (3) acidifying the resultant N-long-chain acyl amino acid salt; or
   alternatively, a crude N-long-chain acyl amino acid product prepared by a method comprising: reacting an amino acid and/or a salt thereof with a long-chain acid halide in the presence of a base to give an N-long-chain acyl amino acid salt, acidifying the resultant N-long-chain acyl amino acid salt, gradually precipitating a solid, standing, separating solid and liquid phases, and optionally washing and drying to give the crude N-long-chain acyl amino acid product.
[22] The method according to any one of items [10]-[21], wherein the long-chain fatty acid is a saturated or unsaturated linear or branched fatty acid having 8-22 carbon atoms; the N-long-chain acyl group in the N-long-chain acyl amino acid is derived from the saturated or unsaturated linear or branched fatty acid having 8-22 carbon atoms; the amino acid in the N-long-chain acyl amino acid is derived from one or more of glycine, alanine, glutamic acid, sarcosine, aspartic acid, leucine, isoleucine, valine, threonine, proline, phenylalanine, arginine, and lysine; the organic solvent is an organic solvent in which the long-chain fatty acid and the N-long-chain acyl amino acid are slightly soluble, practically insoluble, or insoluble, wherein slightly soluble, practically insoluble, or insoluble refers to that the solubility of the long-chain fatty acid and the N-long-chain acyl amino acid in the organic solvent at 20 °C is less than 1 g/100 g, preferably less than 0.01 g/100 g, and more preferably less than 0.001 g/100 g.
[23] The method according to item [22], wherein the long-chain fatty acid is selected from one or more of octylic acid, capric acid, undecanoic acid, lauric acid, myristic acid, pentadecanoic acid, palmitic acid, stearic acid, oleic acid, linoleic acid, isostearic acid, coconut oil fatty acid, or palm oil fatty acid, preferably coconut oil fatty acid or lauric acid, and most preferably lauric acid;
   correspondingly, the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
   the amino acid in the N-long-chain acyl amino acid is derived from alanine, glycine, glutamic acid, sarcosine, arginine, or lysine, and preferably L-alanine.
[24] A method for separating components in a solid mixture using the difference in melting point, comprising: (a) adding a solvent to the solid mixture, (b) after the addition of the solvent, controlling the temperature T of the system in a range of the melting point of a low-melting-point component to the melting point of a high-melting-point component, (c) after the temperature control in the system, separating the solid and liquid phases, wherein the solvent is a solvent in which the components to be separated (i.e., the high-melting-point component and the low-melting-point component to be separated) are slightly soluble, practically insoluble, or insoluble, wherein slightly soluble, practically insoluble, or insoluble refers to that the solubility of the components to be separated in the solvent at 20 °C is less than 1 g/100 g, preferably less than 0.01 g/100 g, and more preferably less than 0.001 g/100 g; the boiling point of the solvent is greater than the melting point of the low-melting-point component, and the temperature T of the system is not higher than the boiling point of the solvent.
[25] The method according to item [24], wherein the difference between the melting points of the components to be separated is 10 °C or greater, preferably 20 °C or greater, and more preferably 30 °C or greater;
   and/or the percentage content by weight of the low-melting-point component is 50% or less, preferably 40% or less, and more preferably 30% or less.
[26] The method according to item 24 or 25, wherein the solid-liquid separation is conducted under the action of centrifugation or pressure; preferably, the solid-liquid separation is promoted by using a solvent having a certain temperature as a medium that allows temperature T to be controlled in a range of the melting point of the low-melting-point component to the melting point of the high-melting-point component, and the solvent is a solvent in which the components to be separated are slightly soluble, practically insoluble, or insoluble.
[27] The method according to any one of items [24]-[26], wherein the solid-liquid separation meets one or more of the following conditions:
   a, during the solid-liquid separation, the solvent used as the medium is in contact with the mixture to be separated, and under the action of centrifugation or pressure, the solvent removes the low-melting-point component to promote the separation;
   b, the solvent used as the medium in the solid-liquid separation is provided by spraying;
   c, the amount of the solvent used as the medium during the solid-liquid separation is greater than 0.5 times the mass of the mixture to be separated; and
   d, the solid-liquid separation is conducted using an industrial centrifuge or a filter press, and preferably a filter centrifuge equipped with a filter screen or a filter cloth.
[28] The method according to item [26] or [27], wherein the temperature T of the solvent as the medium is present in a plurality of temperature stages, and preferably the temperature in a next stage is equal to or higher than the temperature in a previous stage;
   preferably, the temperature in the first stage is controlled in a range of the melting point of the low-melting-point component to the melting point of the low-melting-point component + 10 °C, and the temperature in at least one subsequent stage is controlled in a range of the melting point of the low-melting-point component + 10 °C to the melting point of the high-melting-point component; and
   more preferably, the temperature in the first stage is controlled in a range of the melting point of the low-melting-point component to the melting point of the low-melting-point component + 10 °C, and the temperature in at least one subsequent stage is controlled in a range of the melting point of the low-melting-point component + 20 °C to the melting point of the high-melting-point component.
[29] The method according to item [26] or [27], wherein the percentage content by weight of the low-melting-point component is 10%-40%, and preferably 15%-30%; the temperature T of the solvent as the medium is present in a plurality of temperature stages, and the temperature in the first stage is controlled in a range of the melting point of the low-melting-point component to the melting point of the low-melting-point component + 6 °C, and the temperature in at least one subsequent stage is controlled in a range of the melting point of the low-melting-point component + 15 °C to the melting point of the high-melting-point component;
   more preferably, the temperature in the first stage is controlled in a range of the melting point of the low-melting-point component to the melting point of the low-melting-point component + 3 °C, and the temperature in at least one subsequent stage is controlled in a range of the melting point of the low-melting-point component + 20 °C to the melting point of the high-melting-point component.
[30] The method according to any one of items [24]-[29], wherein after the first solid-liquid separation, n solid-liquid separations are conducted, wherein n is not less than 1, and preferably the temperature in a next solid-liquid separation is equal to or higher than the temperature in a previous solid-liquid separation;
   each solid-liquid separation comprises: mixing a solid obtained from a previous solid-liquid separation with a solvent, optionally stirring, controlling the temperature Tn of the mixture system in a range of the melting point of the low-melting-point component to the melting point of the high-melting-point component, and conducting the solid-liquid separation, wherein the solvent is a solvent in which the components to be separated are slightly soluble, practically insoluble, or insoluble; or
   alternatively: mixing a solid obtained from a previous solid-liquid separation with a solvent, optionally stirring, controlling the temperature Tn of the system in a range of the melting point of the low-melting-point component to the melting point of the high-melting-point component, and conducting the solid-liquid separation, wherein the solid-liquid separation is promoted by using a solvent having a certain temperature as a medium that allows temperature Tn to be controlled in a range of the melting point of the low-melting-point component to the melting point of the high-melting-point component, and the solvent is a solvent in which the components to be separated are slightly soluble, practically insoluble, or insoluble.
[31] The method according to item [30], wherein the temperature T in the first solid-liquid separation is controlled in a range of the melting point of the low-melting-point component to the melting point of the low-melting-point component + 10 °C, and the temperature Tn in at least one solid-liquid separation of the n subsequent solid-liquid separations is controlled in a range of the melting point of the low-melting-point component + 10 °C to the melting point of the high-melting-point component;
   preferably, the temperature T in the first solid-liquid separation is controlled in a range of the melting point of the low-melting-point component to the melting point of the low-melting-point component + 10 °C, and the temperature Tn in at least one solid-liquid separation of the n subsequent solid-liquid separations is controlled in a range of the melting point of the low-melting-point component + 20 °C to the melting point of the high-melting-point component.
[32] The method according to item [30], wherein three or more solid-liquid separations are conducted; the temperature T in the first solid-liquid separation is controlled in a range of the melting point of the low-melting-point component to the melting point of the low-melting-point component + 8 °C, the temperature Tn in at least one intermediate solid-liquid separation is controlled in a range of the melting point of the low-melting-point component + 8 °C to the melting point of the low-melting-point component + 18 °C, and the temperature Tn in the last solid-liquid separation is controlled in a range of the melting point of the low-melting-point component + 24 °C to the melting point of the high-melting-point component.
[33] The method according to item [30], wherein the percentage content by weight of the low-melting-point component is 10%-40%, and preferably 15%-30%; the temperature T in the first solid-liquid separation is controlled in a range of the melting point of the low-melting-point component to the melting point of the low-melting-point component + 6 °C, and the temperature Tn in at least one solid-liquid separation of the n subsequent solid-liquid separations is controlled in a range of the melting point of the low-melting-point component + 15 °C to the melting point of the high-melting-point component;
   more preferably, the temperature T in the first solid-liquid separation is controlled in a range of the melting point of the low-melting-point component to the melting point of the low-melting-point component + 3 °C, and the temperature Tn in at least one solid-liquid separation of the n subsequent solid-liquid separations is controlled in a range of the melting point of the low-melting-point component + 20 °C to the melting point of the high-melting-point component.
[34] A method for preparing an amino acid supramolecule, comprising the step of removing an impurity from a crude N-long-chain acyl amino acid product described in any one of items [10]-[23], wherein a structural reconstruction occurs during the removal of the impurity.
[35] An amino acid supramolecule prepared by the method according to item [34].
[36] The amino acid supramolecule according to item [35], wherein the percentage content by weight of a long-chain fatty acid is 5% or less, preferably 3% or less, and most preferably 0.5%-3%;
   and/or, the percentage content by weight of an N-long-chain acyl amino acid dipeptide is 3% or greater, preferably 5% or greater, more preferably 8% or greater, and most preferably 10% or greater.
[37] An amino acid supramolecule, comprising a supramolecular structure self-assembled by an N-long-chain acyl amino acid and an N-long-chain acyl amino acid dipeptide, wherein the percentage content by weight of the N-long-chain acyl amino acid dipeptide is 3% or greater, preferably 5% or greater, more preferably 8% or greater, and most preferably 10% or greater.
[38] The amino acid supramolecule according to item [37], wherein the amino acid supramolecule is an amino acid supramolecule having a medium content of the dipeptide, and the percentage content by weight of the N-long-chain acyl amino acid dipeptide is 5% or greater and preferably 10% or greater and less than 15% by weight; or
   alternatively, the amino acid supramolecule is an amino acid supramolecule having a high content of the dipeptide, and the percentage content by weight of the N-long-chain acyl amino acid dipeptide is 15% or greater, and preferably 20% or greater.
[39] The amino acid supramolecule according to item [37] or [38], wherein the percentage content by weight of the long-chain fatty acid is 5% or less, preferably 3% or less, and most preferably 0.5%-3%.
[40] The amino acid supramolecule according to any one of items [35]-[39], wherein the amino acid supramolecule has a characteristic ion peak in a range of 541-545 in a mass spectrum detected on a mass spectrometer AB4500 with a scanning mode Q1SCAN, an ionization mode ESI (-), and a scanning range m/z = 200-600;
   and/or, the amino acid supramolecule has 3 or 4 group peaks in the retention time range of 30-45 min in a chromatogram detected on a high-performance liquid chromatograph equipped with an ultraviolet detector with a column of ODS-2 HYPERSIL C18 250 × 4.6 mm 5 µm, a wavelength of 210 nm, and a mobile phase of methanol:20 mmol/L potassium dihydrogen phosphate buffer (pH 3.0) = 70:30 (v/v).
[41] The amino acid supramolecule according to any one of items [35]-[40], wherein the amino acid supramolecule meets one or more of the following conditions: a, the solid powder of the amino acid supramolecule has a columnar, rod-shaped, thread-shaped, or rope-shaped micromorphology; b, the amino acid supramolecule has an initial melting temperature of 78 °C or higher and a final melting temperature of 87 °C or higher, and preferably an initial melting temperature of 80 °C or higher and a final melting temperature of 90 °C or higher, as measured by a capillary method; c, the amino acid supramolecule has a DSC peak temperature of 86 °C or higher, preferably 88 °C or higher, and more preferably 90 °C or higher; d, the sodium salt of the amino acid supramolecule has a number-average molecular weight of 5,000-250,000, preferably 10,000-150,000, and more preferably 15,000-100,000.
[42] The amino acid supramolecule according to any one of items [35]-[41], wherein the amino acid supramolecule meets one or more of the following conditions:
   a, an arm washed with an 8% aqueous solution of the amino acid supramolecule neutralized by arginine for 60 minutes has a moisture content in the stratum corneum larger than that before the washing;
   b, the aqueous solution of the amino acid supramolecule salt is easy to rinse and is not pseudo-slippery; and
   c, according to the GB/T 29679-2013 test, a salt solution of the amino acid supramolecule with an amino acid supramolecule percentage content by weight of 0.5% has a foam quantity at 0 min of more than 130 mm, preferably greater than 150 mm, and more preferably greater than 160 mm; a salt solution of the amino acid supramolecule with an amino acid supramolecule percentage content by weight of 0.05% has a foam quantity at 0 min of more than 40 mm, preferably greater than 60 mm, and more preferably greater than 80 mm.
[43] The amino acid supramolecule according to any one of items [35]-[42], wherein the amino acid supramolecule meets one or more of the following conditions:
   a, the N-long-chain acyl group in the N-long-chain acyl amino acid and the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
   b, the amino acid in the N-long-chain acyl amino acid and the N-long-chain acyl amino acid dipeptide is derived from one or more of glycine, alanine, glutamic acid, sarcosine, aspartic acid, leucine, isoleucine, valine, threonine, proline, phenylalanine, arginine, and lysine, preferably alanine, glycine, glutamic acid, sarcosine, arginine, or lysine, and most preferably L-alanine;
   c, the long-chain fatty acid is selected from one or more of octylic acid, capric acid, undecanoic acid, lauric acid, myristic acid, pentadecanoic acid, palmitic acid, stearic acid, oleic acid, linoleic acid, isostearic acid, coconut oil fatty acid, or palm oil fatty acid, preferably coconut oil fatty acid or lauric acid, and most preferably lauric acid.
[44] The amino acid supramolecule according to any one of items [35]-[43], wherein the N-long-chain acyl amino acid is N-lauroyl-L-alanine, the N-long-chain acyl amino acid dipeptide is N-lauroyl-L-alanyl-L-alanine, and the long-chain fatty acid is lauric acid.
[45] An amino acid supramolecule salt, formed from the amino acid supramolecule according to any one of items [35]-[44] and a base.
[46] The amino acid supramolecule salt according to item [45], wherein the base is selected from one or more of inorganic bases, organic amines, and basic amino acids.
[47] The amino acid supramolecule salt according to item [46], wherein the inorganic base is selected from one or more of sodium hydroxide, potassium hydroxide, sodium carbonate, and potassium carbonate, and preferably sodium hydroxide or potassium hydroxide; the organic amine is selected from amine and alkanolamine; the basic amino acid is selected from one or more of arginine, lysine, and histidine, preferably arginine or lysine.
[48] Use of the amino acid supramolecule and/or the salt thereof according to any one of items [35]-[47], in a cleaning composition, a washing composition, a cosmetic composition, or a healthcare composition.
[49] Use of the amino acid supramolecule and/or the salt thereof according to any one of items [35]-[47], as a surfactant or emulsifier.
[50] Use of the amino acid supramolecule and/or the salt thereof according to any one of items [35]-[47], in adsorbing oil stains or microorganisms, or in sterilizing, deodorizing or removing pesticide residues.
[51] Use of the amino acid supramolecule and/or the salt thereof according to any one of items [35]-[47], in a detergent, a toothpaste, a laundry liquid, a soap, a laundry powder, a dish detergent, a facial mask, a shampoo, a body wash, a facial cleanser, a makeup remover, a mouth wash, a shaving product, a hand sanitizer, a cleaning lotion, or a cleansing cream.
[52] A cleaning composition, comprising the amino acid supramolecule and/or the salt thereof according to any one of items [35]-[47], and preferably comprising an arginine salt or a lysine salt of the amino acid supramolecule.
[53] The cleaning composition according to item [52], wherein the cleaning composition is a detergent, a laundry liquid, a soap, a laundry powder, a dish detergent, a facial mask, a shampoo, a body wash, a facial cleanser, a makeup remover, a mouth wash, a shaving product, a hand sanitizer, a cleaning lotion, or a cleansing cream.
[54] A toothpaste, comprising the amino acid supramolecule and/or the salt thereof according to any one of items [35]-[47], and preferably comprising an arginine salt or a lysine salt of the amino acid supramolecule.
[55] A cosmetic composition, comprising the amino acid supramolecule and/or the salt thereof according to any one of items [35]-[47], and preferably comprising an arginine salt or a lysine salt of the amino acid supramolecule.
[56] A toothpaste, comprising a friction agent, a humectant, a thickening agent, and a surfactant in the following percentages by weight of the toothpaste:
   0.1-25% of the surfactant,
   10-50% of the friction agent,
   5-40% of the humectant,
   0.1-6% of the thickening agent;
   wherein the surfactant comprises the amino acid supramolecule and/or the salt thereof according to any one of items [35]-[47]; preferably, the surfactant comprises 50 wt% or greater, more preferably 80 wt% or greater, and even more preferably 100 wt% of the amino acid supramolecule and/or the salt thereof according to any one of items [35]-[47].
[57] A skin care composition, comprising an oil, a surfactant, and a suspended particle in the following percentages by weight:
   50-95% of the oil,
   0.5-30% of the surfactant,
   0-45% of the suspended particle,
   wherein the surfactant comprises the amino acid supramolecule and/or the salt thereof according to any one of items [35]-[47]; preferably, the surfactant comprises 50 wt% or greater, more preferably 80 wt% or greater, and even more preferably 100 wt% of the amino acid supramolecule and/or the salt thereof according to any one of items [35]-[47].
[58] A laundry liquid, comprising a surfactant, a softening agent, a chelating agent, deionized water, a preservative, and an essence in the following percentages by weight:
   5-50% of the surfactant,
   0.1-3% of the softening agent,
   0.1-5% of the chelating agent,
   50-90% of deionized water,
   0.1-6% of the preservative,
   0.1-2% of the essence,
   wherein the surfactant comprises the amino acid supramolecule and/or the salt thereof according to any one of items [35]-[47]; preferably, the surfactant comprises 50 wt% or greater, more preferably 80 wt% or greater, and even more preferably 100 wt% of the amino acid supramolecule and/or the salt thereof according to any one of items [35]-[47].
[59] A laundry powder, comprising a surfactant and a friction agent in the following percentages by weight:
   10-50% of the surfactant,
   50-90% of the friction agent,
   wherein the surfactant comprises the amino acid supramolecule and/or the salt thereof according to any one of items [35]-[47]; preferably, the surfactant comprises 50 wt% or greater, more preferably 80 wt% or greater, and even more preferably 100 wt% of the amino acid supramolecule and/or the salt thereof according to any one of items [35]-[47].
[60] A dish detergent, comprising a surfactant, deionized water, a thickening agent, glycerol, a preservative, and an essence in the following percentages by weight:
   5-20% of the surfactant,
   70-90% of deionized water,
   1-2% of the thickening agent,
   5-10% of glycerol,
   0.1-6% of the preservative, and
   0.1-2% of the essence,
   wherein the surfactant comprises the amino acid supramolecule and/or the salt thereof according to any one of items [35]-[47]; preferably, the surfactant comprises 50 wt% or greater, more preferably 80 wt% or greater, and even more preferably 100 wt% of the amino acid supramolecule and/or the salt thereof according to any one of items [35]-[47].

Compared with the prior art, the present invention has the following beneficial technical effects:
1. The inventors have surprisingly found that long-chain acyl amino acid dipeptides and/or salts thereof and compositions comprising the same can be prepared at low costs by controlling conditions such as pH, addition amount of base, and the like.
2. The present invention provides a creative method for efficiently removing impurities in the long-chain acyl amino acid, and the method does not need a catalyst and pressurization and has mild conditions. Based on the principle, the present invention achieves the separation of the components in a solid mixture by utilizing the difference in melting point of the components to be separated and the solvent with a specific temperature.
3. The temperature of the solvent is controlled in a range of the melting point of the long-chain fatty acid to the melting point of the long-chain acyl amino acid, which is beneficial to removing impurities and controlling the structural reconstruction of the long-chain acyl amino acid to form a product with a specific structure. The method for screening the proper separation temperature according to the melting points of the long-chain fatty acid and the long-chain acyl amino acid is proposed for the first time and serves the separation well.
4. In the conventional purification operation, solvents at normal or lower temperatures are often used for washing a product. It has not been realized that, when some low-melting-point impurities are removed at a lower temperature, the subsequent solid-liquid separation can endure a higher temperature, and therefore, the impurities can be better removed by adopting multiple solid-liquid separations and/or treatments with temperature gradients.
5. The solid-liquid separation is promoted by using a medium solvent with a certain temperature controlled in a range from the melting point of the long-chain fatty acid to the melting point of the N-long-chain acyl amino acid. By spraying/washing with the medium solvent (especially spraying under the action of centrifugation or pressure) and separation, impurities are effectively removed, and the structural reconstruction of the N-long-chain acyl amino acid can be promoted to give a product with a specific structure. The solution is centrifuged continuously in a filter centrifuge with a filter screen or filter cloth like a laundry machine, and a product with a reconstructed structure is obtained with the coordination of a medium solvent at a certain temperature.
6. In conventional Schotten-Baumann reactions, post-treatments must be conducted with an organic solvent such as petroleum ether, isopropanol, ethanol, etc., for washing or recrystallization. The proper control of treatment temperature according to the present invention and/or the centrifugation allows treatment using water only, which is of great significance in terms of both environmental preservation and cost efficiency.
7. The method of the present invention can properly control the content of the long-chain fatty acid and the content of dipeptide (for example, lauroyl alanyl alanine in a reaction of lauroyl chloride and sodium alaninate), and can even conveniently give a product with a dipeptide content of greater than 20%.
8. The amino acid supramolecule or the salt thereof obtained by the method has a special structure, can adsorb organic matters such as oil stains and the like, and has strong degreasing capability.
9. The amino acid supramolecule or the salt thereof prepared by the method has stable structural performance, supramolecular property, and special spatial structure, thus having properties of physical sterilization, deodorization, pesticide residue removal, and the like. The supramolecule also has good microorganism inhibitory rates of up to 100% on Escherichia coli, Staphylococcus aureus, and Candida albicans, high removal rates of methamidophos and acephate, and good deodorizing performance.
10. Due to the special structure, the amino acid supramolecule can be combined with grease to give a non-sticky "solid"/paste which is easy to remove and has good cleaning capacity within a pH range of 5-14 and a broad application range.

### BRIEF DESCRIPTION OF THE DRAWINGS

FIG. 1 is a high-performance liquid chromatogram of the Example #33 sample;
FIG. 2 is a high-performance liquid chromatogram of the Example #503 sample;
FIG. 3 is a high-performance liquid chromatogram of the Example #604 sample;
FIG. 4 is a spectrum of ESI negative ion mode and full scan mode;
FIG. 5 is a standard curve of lauric acid in method 1;
FIG. 6 is an SIR spectrum of the sample solution in Example 1;
FIG. 7 is a standard curve of lauric acid in method 2;
FIG. 8 is a PDA channel spectrum and integral of the sample solution in Example 1;
FIG. 9 is a QDa full-scan channel spectrum and integral of the sample solution in Example 1;
FIG. 10 illustrates a DSC analysis of the sample in Example 1;
FIG. 11 illustrates a DSC analysis of Example #502 sample;
FIG. 12 illustrates a DSC analysis of Example #501 sample after the first solid-liquid separation;
FIG. 13 illustrates a DSC analysis of Example #501 sample after the second solid-liquid separation;
FIG. 14 illustrates a DSC analysis of Example #501 sample after the third solid-liquid separation;
FIG. 15 illustrates a DSC analysis of Example #503 sample after the first solid-liquid separation;
FIG. 16 illustrates a DSC analysis of Example #503 sample after the second solid-liquid separation;
FIG. 17 illustrates a DSC analysis of Example #503 sample after the third solid-liquid separation;
FIG. 18 illustrates scanning electron micrographs of the crude product of Example 502#, wherein 18a is at 200× magnification, 18b is at 500× magnification, and 18c is at 2000× magnification;
FIG. 19 illustrates scanning electron micrographs of the crude product of Example 501# after washing, wherein 19a is at 200× magnification, 19b is at 1000× magnification, and 19c is at 2000× magnification;
FIGs. 20a and 20b are double quantum-filtered hydrogen spectra (DQ-filtered), 20c is a 2D ¹H-¹H DQ-SQ two-dimensional spectrum, 20d is a ¹³C CP spectrum, and 20e is a 2D ¹³C-¹H FSLG-HETCOR spectrum;
FIG. 21 is a mass spectrum of the sample in Example 6;
FIG. 22 is a diagram showing the effects of removing color cosmetics; and
FIG. 23 is a change profile in moisture content of the stratum corneum.

### DETAILED DESCRIPTION

In one embodiment, provided are a cost-efficient method for preparing a long-chain acyl amino acid dipeptide and/or a salt thereof, and a method for preparing a composition comprising the dipeptide. The long-chain acyl amino acid dipeptide refers to a long-chain acyl aminoacyl amino acid, typically, for example, a long-chain acyl glycyl glycine, a long-chain acyl glutamyl glutamic acid, a long-chain acyl alanyl alanine, and the like.

Specifically, the method comprises the steps of reacting an amino acid and/or a salt thereof with a long-chain acid halide; the pH of the system after the reaction (only referring to the system after the reaction of the amino acid and/or the salt thereof with the long-chain acid halide) is less than 8, preferably 7.5, 7 or less, more preferably within a range of 3, 3.5, 4, 4.5 or 5 to 7, 6.9, 6.8, 6.7, 6.6, 6.5 or 6. Preferably, the pH is 5-7, and most preferably 5-6.5.

A conventional Schotten-Baumann reaction requires a pH of 8 or greater in the system throughout the reaction process because according to the conventional reaction mechanism, it is believed that for the condensation of the acid halide with the amine, the amine first serves as a nucleophile of the nucleophilic addition/substitution to the acid halide to give a cationic amide intermediate, which is subsequently deprotonated under basic conditions to yield the amide product. The reactivity of the amine depends on the alkalinity, where a stronger alkalinity indicates a faster reaction rate. If the alkalinity in the system is not controlled, the amine substrate can be protonated and deactivated by Bronsted acid by-products generated in the system.

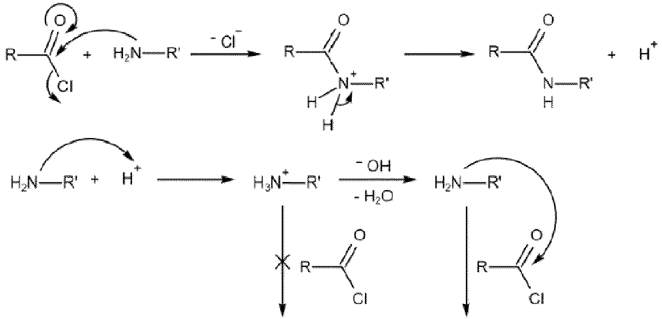

The inventors have surprisingly found that if the pH of the reaction system is not controlled at 8 or greater, the pH of the reaction system will decrease with the continuous addition of the acid halide, and if the final pH after the reaction is less than 8, for example, 7.5 or less, particularly 7 or less or less than 7, the formation of the long-chain acyl amino acid dipeptide and/or the salt thereof is facilitated.

In general, the final pH of the reaction system can be controlled by controlling the addition amount of the base. It has been investigated that in the step of reacting an amino acid and/or a salt thereof with a long-chain acid halide in the presence of a base, the molar ratio of the amino acid to the base throughout the reaction is 3:1 to 1:2 (for the amino acid salt, the molar ratio of the amino acid to the base is also counted), preferably 2:1 to 1:2, and more preferably in a range of 1.9:1, 1.8:1, 1.7:1, 1.6:1, 1.5:1, 1.4:1, 1.3:1, 1.2:1 or 1.1:1 to 1:1.9, 1:1.8, 1:1.7, 1:1.6, 1:1.5, 1:1.4, 1:1.3, 1:1.2, 1:1.1 or 1:1; for example, amolar ratio of 2:1 to 1:1.8, 1.8:1 to 1:1.8 or 1.7:1 to 1:1.7, more preferably 1.5:1 to 1:1.5, and most preferably 1.4:1 to 1:1.4. The molar ratio of the amino acid to the base refers to the molar ratio of the overall amount of the amino acid to the overall amount of the base.

The base can be added once or in batches, and can be added into the reaction system independently or together with the long-chain acid halide. The long-chain acid halide can be added into the reaction system once or in batches. However, even if the base and/or the acid halide are added in batches, it is no required that the pH of the reaction system should be maintained at an alkaline level.

Furthermore, according to the method of the present invention, the long-chain acid halide is added to the amino acid and/or the salt thereof without controlling the reaction solution at an alkaline pH, preferably without controlling the reaction solution at pH 8 or greater; alternatively, the long-chain acid halide is added to the amino acid and/or the salt thereof without adding the base to maintain the pH value, or without controlling the dripping speed and amount of the base such as sodium hydroxide to maintain the pH value of the system; alternatively, the difference between the pH values of the system before and after the addition of the long-chain acid halide is 2 or higher, and preferably 3, 4, 5, 6 or higher. That is, the present invention does not control the pH value like conventional Schotten-Baumann reactions, but lets the pH value of the reaction system gradually decrease over time.

By controlling the addition amount of the specific base (the molar ratio of the amino acid to the base) and/or the pH, the present invention can conveniently obtain a large amount of dipeptide product. Preferably, the percentage content by weight of the N-long-chain acyl amino acid dipeptide in the product is 3% or greater, preferably 4%, 5%, 6%, 7%, 8%, 9%, 10% or greater after the reaction of the amino acid and/or the salt thereof with the long-chain acid halide.

In one embodiment, after the condensation reaction of the acid halide and the amino acid, the dipeptide has a moderate content, i.e., the percentage content by weight of the N-long-chain acyl amino acid dipeptide and/or the salt thereof is 5% or greater, preferably 8% or greater, and more preferably 10% or greater, and less than 15%.

In one embodiment, after the condensation reaction of the acid halide and the amino acid, the dipeptide has a high content, i.e., the percentage content by weight of the N-long-chain acyl amino acid dipeptide and/or the salt thereof is 15% or greater, and preferably 20% or greater.

In one preferred embodiment, the method for preparing the long-chain acyl amino acid dipeptide and/or the salt thereof or the related composition comprises: (1) reacting a raw material comprising the amino acid and the base to give an amino acid salt solution; and (2) adding the long-chain acid halide into the resultant amino acid salt solution or adding the long-chain acid halide and the base into the resultant amino acid salt solution, wherein the method meets one or more of the following conditions:
a, the pH value of the amino acid salt solution prepared in step (1) is 7.5-14, preferably in a range of 8, 8.5, 9 or 9.5 to 13.5, 13, 12.5, 12, 11.5 or 11, preferably 8-12, and more preferably 9-11, and the pH value of the system after the reaction in step (2) is less than 8, for example, 7.5 or less, preferably 7 or less or less than 7, more preferably in a range of 3, 3.5, 4, 4.5 or 5 to 7, 6.9, 6.8, 6.7, 6.5 or 6. Preferably, the pH is 5-7, and most preferably 5-6.5, for example, about 6.
b, the molar ratio of the amino acid to the base in the reaction system throughout step (1) and step (2) is 3:1 to 1:2, preferably 2:1 to 1:1.8, and more preferably in a range of 1.9:1, 1.8:1, 1.7:1, 1. 6:1, 1.5:1, 1.4:1, 1.3:1, 1.2: 1 or 1. 1: 1 to 1: 1. 9, 1:1.8, 1:1.7, 1:1.6, 1:1.5, 1:1.4, 1:1.3, 1:1.2, 1: 1. 1 or 1:1. The molar ratio of the amino acid to the base is further preferably 1.8:1 to 1:1.8, preferably 1.7:1 to 1:1.7, and most preferably 1.5:1 to 1:1.5 or 1.4:1 to 1:1.4.
c, the pH value of the amino acid salt solution prepared in step (1) is greater than that of the system prepared after the reaction of the amino acid salt and the long-chain acid halide in step (2), and the difference between the two is 2 or greater, and preferably 3, 4, 5 or 6 or greater.

If the addition amount of the base or the pH value after the reaction is excessive, the reaction product may have a composition similar to that of a conventional Schotten-Baumann reaction, i.e., the prominent component is a long-chain acyl amino acid (monopeptide) and the dipeptide content of the long-chain acyl amino acid is very low (generally less than 2%, or even 0). If the addition amount of the base is insufficient, a precipitate may be generated soon after the acid halide is added dropwise, and the hydrolysis of the acid halide is accelerated, which may affect the reaction and reduce the yield. Therefore, it is advantageous to contain a specific amount of the base as described above, and it is preferable to satisfy the above conditions of a, b, and c at the same time.

In one preferred embodiment, the reaction of the amino acid and/or the salt thereof with the long-chain acid halide meets one or more of the following conditions:
a, the reaction is conducted in the presence of water or a mixed solution of water and a hydrophilic organic solvent; the hydrophilic organic solvent is selected from one or more of acetone, methanol, ethanol, isopropanol, sec-butyl alcohol, tert-butyl alcohol, acetonitrile, and tetrahydrofuran, and preferably acetone; preferably, the volume ratio of the water to the hydrophilic organic solvent is 1:(0-2), and preferably 1:(0.8-1.5);
b, the temperature of the reaction is 35 °C or lower, and preferably 30 °C or lower; and
c, the molar ratio of the amino acid and/or the salt thereof to the long-chain acid halide is greater than 1, preferably 2:1 to 1.1:1, and more preferably 1.5:1 to 1.2:1.

It is preferable to satisfy the conditions a, b, and c.

If water is used as a medium for the reaction of the amino acid and/or the salt thereof with the long-chain acid halide, for example, when the amino acid and the base are dissolved in water and uniformly stirred before the long-chain acid halide is added, the reaction system is more environment-friendly, but the hydrolysis of the long-chain acid halide is promoted, leading to increased reaction impurities and reduced yield. If a mixed solution of water and a hydrophilic organic solvent is used as a medium, the hydrolysis of the long-chain acid halide can be well controlled by increasing the amount of the hydrophilic organic solvent. However, an excessive amount of the hydrophilic organic solvent is unfavorable for environmental protection, cost-efficiency and the like, and is undesirable for the reaction, thus reducing the yield and the like.

The temperature of the reaction is preferably controlled at a relatively lower temperature, e.g., 40 °C or lower, preferably 35 °C or 30 °C or 25 °C or lower, for example, the temperature can be controlled at 20-30 °C throughout the reaction. If the temperature is too high, the acid halide hydrolysis may be increased. The temperature should not be too low, or otherwise, the reactivity may be poor. It is preferable to control the temperature at 10 °C or higher.

To facilitate the formation of the dipeptide, it is preferred that the molar ratio of the amino acid and/or salt thereof to the long-chain acid halide is greater than 1. Certainly, excessive amino acids may be unnecessary. The amino acid excess cannot be reacted sufficiently and may increase the burden of the post-treatment. The molar ratio of the amino acid and/or the salt thereof to the long-chain acid halide is preferably 1, 1.1, 1.2, 1.3 or greater to 2, 1.9, 1.8, 1.7, 1.6, 1.5 or 1.4 or less; for example, 2:1 to 1.1:1, and more preferably 1.5:1 to 1.2:1.

Furthermore, the method also comprises: acidifying a product prepared by the reaction of the amino acid and/or the salt thereof with the long-chain acid halide to give a crude N-long-chain acyl amino acid product; preferably, the pH after the acidification is 1 or 2 or greater to 3 or 4 or less, and more preferably 1 to 2. The crude product refers to a product containing impurities such as long-chain fatty acid, sodium chloride, and the like. For the composition, the crude product contains both the long-chain acyl amino acid and the long-chain acyl amino acid dipeptide.

The above-mentioned product can adopt a known process or the following impurity-removing process of the present invention to further remove the impurities, so as to give a composition containing the long-chain acyl amino acid and the long-chain acyl amino acid dipeptide and the related amino acid supramolecule. The composition can also be separated to give the long-chain acyl amino acid dipeptide.

In one preferred embodiment, the amino acid is selected from one or more of glycine, alanine, glutamic acid, sarcosine, aspartic acid, leucine, isoleucine, valine, threonine, proline, phenylalanine, arginine, and lysine. More preferably, the amino acid is selected from alanine, glycine, glutamic acid, sarcosine, arginine, or lysine, and most preferably L-alanine.

In one preferred embodiment, the long-chain acyl in the long-chain acid halide is derived from a saturated or unsaturated linear or branched fatty acid with 8-22 carbon atoms. More preferably, the long-chain acid halide is selected from one or more of octanoyl chloride, caprinoyl chloride, undecanoyl chloride, lauroyl chloride, myristoyl chloride, pentadecanoyl chloride, palmitoyl chloride, stearoyl chloride, oleoyl chloride, linoleoyl chloride, isostearoyl chloride, coconut oil fatty acid chloride, or palm oil fatty acid chloride, preferably coconut oil fatty acid chloride or lauroyl chloride, and most preferably lauroyl chloride.

In one preferred embodiment, the base is selected from one or more of sodium hydroxide, potassium hydroxide, sodium carbonate, potassium carbonate, and ammonia. More preferably, the base is selected from sodium hydroxide or potassium hydroxide.

In one embodiment, provided is a method for removing an impurity from an N-long-chain acyl amino acid, wherein during the removal of the impurity according to the method, a structural reconstruction occurs, forming an amino acid supramolecule with a specific structure.

The method comprises: mixing the crude N-long-chain acyl amino acid product with a solvent (the solvent can be added to the crude N-long-chain acyl amino acid product, or the crude N-long-chain acyl amino acid product can be added into the solvent, and the mixture after mixing is referred to as the system), optionally stirring, and controlling the temperature T of the system in a range of the melting point of a long-chain fatty acid to the melting point of the N-long-chain acyl amino acid, wherein the solvent is water, an organic solvent, or a mixed solution of water and an organic solvent. It is more preferred to use only water as the solvent from the viewpoints of cost-efficiency for industrial production and environmental protection.

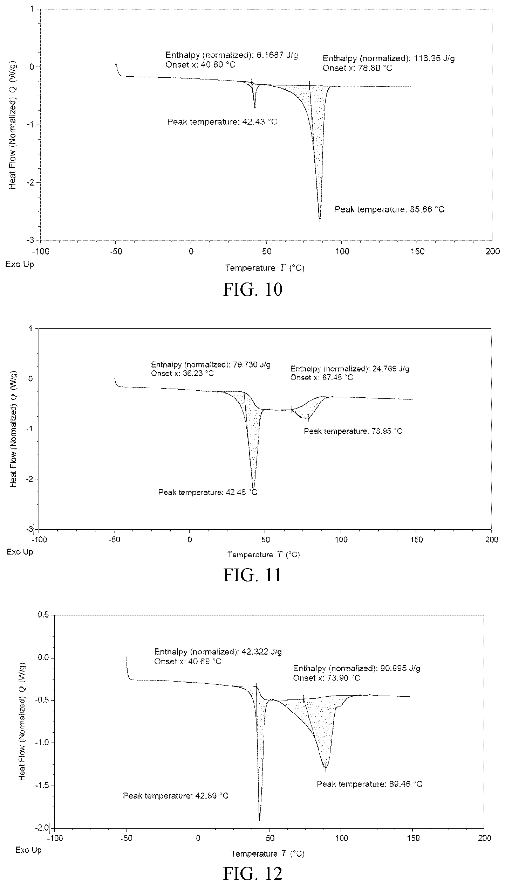

The selection of the specific temperature is attributed to the difference between the melting points of the N-long-chain acyl amino acid and that of the long-chain fatty acid. As shown in FIG. 11, for example, for lauroyl alanine, the corresponding peak value of lauric acid in the figure is 42.46 °C, and the peak value of lauroyl alanine (crude product) is 78.95 °C. If the temperature is controlled at the melting point of the long-chain fatty acid such as lauric acid or higher, the long-chain fatty acid impurity can be melted, thus facilitating the removal of the long-chain fatty acid; if the temperature is controlled at the melting point of the N-long-chain acyl amino acid or lower, the melting of the N-long-chain acyl amino acid can be avoided. An excessive temperature may lead to the melting of the solid and thus invalid solid-liquid separation, and the two phases can only be formed only by selecting a specific temperature. The method for screening the proper impurity-removing temperature according to the melting points of the long-chain fatty acid and the long-chain acyl amino acid is proposed for the first time and well serves the effective removal of impurities, the formation of specific spatial structures, and the performance of the product.

The crude N-long-chain acyl amino acid product is mixed with a solvent, and besides the additional addition of the solvent, the situation that the crude product contains a solvent is also included. For example, a typical situation where the crude product contains a solvent is that no solid-liquid separation is conducted after acidification with hydrochloric acid/sulfuric acid. In this case, no additional solvent well be added, but instead, the solvent may be optionally supplemented, which still satisfies the condition of "mixing the crude N-long-chain acyl amino acid product with a solvent".

Preferably, the impurity removal mainly refers to the removal of the long-chain fatty acid (such as lauric acid) and water-soluble impurities such as salts, unreacted amino acids, and the like.

The crude N-long-chain acyl amino acid product is a commercially available N-long-chain acyl amino acid, a crude N-long-chain acyl amino acid product acquired from a Schotten-Baumann reaction of an amino acid and/or a salt thereof and a long-chain acid halide, or the aforementioned crude product containing the dipeptide. The crude product refers to a product containing impurities such as long-chain fatty acid, sodium chloride, and the like.

The N-long-chain acyl amino acid described in "the melting point of the N-long-chain acyl amino acid" is identical to the N-long-chain acyl amino acid described in "the crude N-long-chain acyl amino acid product", and both are all derived from the long-chain fatty acid, i.e., the N-long-chain acyl amino acid, the crude N-long-chain acyl amino acid product, and the long-chain fatty acid have the same "long chain". For example, in the case of a crude lauroyl alanine product, controlling the temperature T of the system "in a range of the melting point of the long-chain fatty acid to the melting point of the N-long-chain acyl amino acid" refers to controlling the temperature T of the system "in a range of the melting point of lauric acid to the melting point of lauroyl alanine". Similarly, for a crude lauroyl glutamic acid product, it refers to controlling the temperature T of the system "in a range of the melting point of lauric acid to the melting point of lauroyl glutamic acid"; for a crude lauroyl sarcosine product, it refers to controlling the temperature T of the system "in a range of the melting point of lauric acid to the melting point of lauroyl sarcosine".

For controlling the temperature T of the system, the general purpose is to control the temperature, and the form may be various. For example, the solvent is added into a reaction kettle/container before the crude N-long-chain acyl amino acid product is added, and then the system is heated to raise the temperature to a range of the melting point of the long-chain fatty acid to the melting point of the N-long-chain acyl amino acid. Alternatively, the crude N-long-chain acyl amino acid product is added into a reaction kettle/container before the solvent or a solvent with a certain temperature such as hot water. Optionally the system is heated, and the temperature is controlled in a range of the melting point of the long-chain fatty acid to the melting point of the N-long-chain acyl amino acid.

Stirring can make the mixture more homogeneous, and certainly, other procedures that achieve the same mixing effect can also be adopted. Stirring or a similar procedure is preferred in view of the effect of mixing.

Optionally, the amino acid is added simultaneously when the solvent is added to the crude N-long-chain acyl amino acid product, or an amino acid solution, such as an aqueous amino acid solution, is added to the crude N-long-chain acyl amino acid product, and then the temperature of the system is controlled in a range of the melting point of the long-chain fatty acid to the melting point of the N-long-chain acyl amino acid.

After the temperature control of the system is completed, the solid-liquid separation procedure (the first solid-liquid separation) is conducted.

In one preferred embodiment, the solid-liquid separation is performed under the action of centrifugation or pressure. Preferably, the solid-liquid separation is further promoted by using a solvent having a certain temperature as a medium that allows temperature T to be controlled in a range of the melting point of the long-chain fatty acid to the melting point of the N-long-chain acyl amino acid, and the solvent is water, an organic solvent, or a mixed solution of water and an organic solvent. It is more preferred to use only water as the solvent from the viewpoints of cost-efficiency for industrial production and environmental protection.

In the present invention, the solvent as a medium is also referred to as "medium solvent". For the temperature of the medium solvent, for example, for a crude lauroyl sarcosine product, the temperature T of the medium solvent should be controlled in a range of the melting point of lauric acid to the melting point of lauroyl sarcosine; for a crude lauroyl alanine product, the temperature T of the medium solvent should be controlled in a range of the melting point of lauric acid to the melting point of lauroyl alanine.

For ease of understanding, the "promoted by using a solvent having a certain temperature as a medium" may be analogous to, but different from, elution in a conventional chemical separation. Specifically, at the described temperature, the crude product is separated into solid and liquid phases (at a temperature higher than the melting point of the long-chain fatty acid, the long-chain fatty acid impurity is melted to form the liquid phase, and the N-long-chain acyl amino acid is the solid phase), wherein the liquid phase can be gradually removed (preferably under the action of centrifugation or pressure) after the medium solvent is brought into contact with the crude product. That is, the medium solvent treatment refers to contacting the medium solvent with the crude product, e.g., in a manner similar to washing the product/crude product or rinsing the product/crude product, etc.

Preferably, during the solid-liquid separation, the solvent used as the medium is in continuous contact with the crude product, and under the action of centrifugation or pressure, the solvent carrying the impurity is removed to promote the separation.

Preferably, the medium solvent treatment is conducted simultaneously with the solid-liquid separation. The "medium solvent treatment simultaneous with the solid-liquid separation" refers to that the treatment is performed while the solid-liquid separation is performed in at least one stage of the solid-liquid separation. For example, in a solid-liquid separation operation on a centrifuge, after the mixed solution of the crude product and the solvent is transferred into the centrifuge, the centrifuge is started to separate the liquid first, and then a spraying device is turned on to spray the medium solvent such as hot water while the centrifuge is still working; alternatively, the centrifuge can be started after the mixed solution is transferred into the centrifuge, and the spraying device is turned on to spray the medium solvent such as hot water at the start of the centrifugation, while the centrifuge is working. It is surprisingly found in the present invention that in the case that the centrifugal separation and medium solvent treatment procedures are conducted simultaneously in at least one stage, the amino acid supramolecule is more likely to form a specific spatial structure, and the product may exhibit excellent performance.

The mode of adding the medium solvent during the solid-liquid separation is preferably spraying, i.e., the medium solvent is sprayed on the crude product to achieve the purpose of continuously contacting the crude product, and the amount of the medium solvent is greater than 0.5 times, preferably 0.5-3 times, and preferably 1-2 times the mass of the crude product each time. An insufficient amount may lead to poor treatment effects, while an excessive amount may lead to a waste of water and electricity resources and loss of products.

Furthermore, the medium solvent treatment is conducted under the action of centrifugation or pressure. The treatment under the action of centrifugation refers to that during the solvent treatment (e.g., spraying/washing), the solid-liquid separation is conducted under the action of centrifugation, most typically, in a centrifuge. The simultaneous operation of the centrifuge during the medium solvent treatment (e.g., spraying/washing) imparts a centrifugal environment. The treatment under the action of pressure refers to that during the solvent treatment (e.g., spraying/washing) the solid-liquid separation is conducted under the action of pressure, for example, in various apparatuses/devices that may impart a pressure, most typically, in a filter press. The simultaneous operation of the filter press during the treatment creates a pressed environment.

Furthermore, the solid-liquid separation is conducted using a centrifuge or a filter press. For the centrifuge, an industrial centrifuge is particularly preferred. More particularly preferred is a filter centrifuge equipped with a filter screen or a filter cloth.

In view of better removing the impurities, facilitating the structural reconstruction and the like, a mode of multiple temperature stages/temperature gradient treatment is preferably adopted, or alternatively, after the first solid-liquid separation, n solid-liquid separations can be conducted, wherein n is ≥ 1.

In one preferred embodiment, the temperature T of the medium solvent is present in a plurality of temperature stages (referred to as "medium solvent temperature gradient treatment" or "temperature gradient treatment"). Preferably, the temperature in the first stage is controlled in a range of the melting point of the long-chain fatty acid to the melting point of the long-chain fatty acid + 15 °C, and the temperature in at least one subsequent stage is controlled in a range of the melting point of the long-chain fatty acid + 15 °C to the melting point of the N-long-chain acyl amino acid; more preferably, the temperature in the first stage is controlled in a range of the melting point of the long-chain fatty acid to the melting point of the long-chain fatty acid + 10 °C, and the temperature in at least one subsequent stage is controlled in a range of the melting point of the long-chain fatty acid + 20 °C to the melting point of the N-long-chain acyl amino acid.

Preferably, three or more temperature stages are adopted; the temperature in the first stage is controlled in a range of the melting point of the long-chain fatty acid to the melting point of the long-chain fatty acid + 8 °C; the temperature in at least one intermediate stage is controlled in a range of the melting point of the long-chain fatty acid + 8 °C to the melting point of the long-chain fatty acid + 18 °C, and the temperature in the last stage is controlled in a range of the melting point of the long-chain fatty acid + 24 °C to the melting point of the N-long-chain acyl amino acid.

Preferably, if water is used as the solvent in the preparation of the crude N-long-chain acyl amino acid product or the content of the long-chain fatty acid impurity in the crude N-long-chain acyl amino acid product is 10% or greater, the temperature T of the medium solvent is present in a plurality of temperature stages, and the temperature in the first stage is controlled in a range of the melting point of the long-chain fatty acid to the melting point of the long-chain fatty acid + 6 °C, and the temperature in at least one subsequent stage is controlled in a range of the melting point of the long-chain fatty acid + 15 °C to the melting point of the N-long-chain acyl amino acid; more preferably, the temperature in the first stage is controlled in a range of the melting point of the long-chain fatty acid to the melting point of the long-chain fatty acid + 3 °C, and the temperature in at least one subsequent stage is controlled in a range of the melting point of the long-chain fatty acid + 20 °C to the melting point of the N-long-chain acyl amino acid.

The gradient treatment with multiple temperature stages is used because the temperature tolerance of the entire crude product gradually increases with the decrease of impurity level (e.g., as can be seen in FIGs. 11-14, the peak shifts gradually to the right after each treatment). If a higher temperature is used early, most of the product may be melted, and if a lower temperature is used, the long-chain fatty acid impurity may not be melted and will not be removed effectively. When a low temperature that is higher than the melting point of the long-chain fatty acid is adopted, part of the impurities can be removed first, and the temperature tolerance of the treated system is improved to a certain extent. Then, the treatment at a higher temperature is adopted, and more impurities can be removed, before the treatment temperature further rises. When the crude product has a high impurity content, the system may possess poor temperature tolerance. An excessively high temperature may lead to a pasty product and even the gradual melting of the solid, which are unfavorable for separation by filtration. Accordingly, it is preferred that the initial treatment temperature is close to the melting point of the long-chain fatty acid. Otherwise, when the temperature is improperly adjusted, the excessively high temperature may cause an excessive amount of melted long-chain fatty acid. Such melted long-chain fatty acid may serve as a solvent to further dissolve the N-long-chain acyl amino acid, thus eventually resulting in reduced product yields.

When the long-chain fatty acid involved in the long-chain acyl amino acid is lauric acid, which has a melting point of about 44 °C, in some related embodiments, the temperature T of the medium solvent is present in a plurality of temperature stages; the temperature in the first stage is controlled at 60 °C or lower, preferably, in a range of 44-60 °C, more preferably in a range of 50-60 °C, and still more preferably in a range of 55-58 °C. The temperature in at least one subsequent stage is controlled at 60 °C or higher, preferably at 65 °C or higher or in a range of 60-95 °C, 62-90 °C, 65-80 °C, 65-77 °C, 65-75 °C, and more preferably 65-70 °C and 66-68 °C.

The temperature controlled at 60 °C or higher in at least one subsequent stage can be interpreted as follows: for example, there are 3 temperature stages in total; the temperature in the first stage can be controlled in a range of 50-60 °C such as 50 °C, the temperature in the second stage can be controlled at 60 °C or higher such as 65 °C, and the temperature in the third stage can be controlled at 60 °C or higher such as 70 °C; alternatively, the temperature in the first stage can be controlled in a range of 50-60 °C such as 50 °C, the temperature in the second stage can be controlled in a range of 50-60 °C such as 50 °C, and the temperature in the third stage can be controlled at 60 °C or higher such as 65 °C; alternatively, the temperature in the first stage can also be controlled in a range of 50-60 °C such as 50 °C, the temperature in the second stage can be controlled at 60 °C or higher such as 65 °C, and the temperature in the third stage can be controlled in a range of 50-60 °C such as 60 °C. Preferably, the temperature in the first stage is controlled in a range of 50-60 °C, followed by a gradually ascending temperature.

For another example, there are 4 temperature stages in total; the temperature in the first stage can be controlled in a range of 50-60 °C, the temperature in the second stage can be controlled at 60 °C or higher, the temperature in the third stage can be controlled at 60 °C or higher, and the temperature in the fourth stage can be controlled at 60 °C or higher; alternatively, the temperature in the first stage can be controlled in a range of 50-60 °C, the temperature in the second stage can be controlled in a range of 50-60 °C, the temperature in the third stage can be controlled at 60 °C or higher, and the temperature in the fourth stage can be controlled at 60 °C or higher; alternatively, the temperature in the first stage can also be controlled in a range of 50-60 °C, the temperature in the second stage can be controlled at 60 °C or higher, the temperature in the third stage can be controlled in a range of 50-60 °C, and the temperature in the fourth stage can be controlled at 60 °C or higher. Preferably, the temperature in the first 1-2 stage is in a range of 50-60 °C, and the temperature in the subsequent stage(s) is 60 °C or higher.

For all of the above embodiments, it is preferred that the temperature ascends or generally ascends (substantially identical temperatures in some intermediate stages are permitted) among all stages. That is, the treatment temperature in a next stage is equal to or higher than the treatment temperature in a previous stage. Preferably, there are more than 3, 4, 5 or 6 or more temperature stages. Generally, in terms of the content of impurities and the convenience, cost-efficiency and efficiency of treatment, 3 temperature stages are preferred.

In order to achieve an adequate effect of removing impurities, the medium solvent can be stirred at random during the medium solvent treatment, such that the medium solvent is in sufficient contact with the solid as much as possible.

In another preferred embodiment, after the first solid-liquid separation, n solid-liquid separations can be conducted, wherein n is ≥ 1. Each solid-liquid separation comprises: mixing a solid obtained from a previous solid-liquid separation with a solvent, optionally stirring, controlling the temperature Tn of the mixture system in a range of the melting point of the long-chain fatty acid to the melting point of the N-long-chain acyl amino acid, and conducting the solid-liquid separation, wherein the solvent is water, an organic solvent, or a mixed solution of water and an organic solvent.

Alternatively, the solid-liquid separation specifically comprises: mixing a solid obtained from a previous solid-liquid separation with a solvent, optionally stirring, controlling the temperature Tn of the system in a range of the melting point of the long-chain fatty acid to the melting point of the N-long-chain acyl amino acid, and conducting the solid-liquid separation, wherein the solid-liquid separation is promoted by using a solvent having a certain temperature as a medium that allows temperature Tn to be controlled in a range of the melting point of the long-chain fatty acid to the melting point of the N-long-chain acyl amino acid. The medium solvent is water, an organic solvent, or a mixed solution of water and an organic solvent. It is more preferred to use only water as the medium solvent from the viewpoints of cost-efficiency for industrial production and environmental protection. For the related content on the medium solvent, reference may be made to the foregoing description, which is not repeated here.

The solid-liquid separation operations are independent of each other. That is, the two solid-liquid separation modes described above may be selected independently and combined. For example, in some solid-liquid separation operations, a solvent having a certain temperature is used as the medium to promote the separation, and in other solid-liquid separation operations, no medium solvent is used.

Preferably, in all solid-liquid separation, the medium solvent treatment is conducted simultaneously; it is also preferred that the solvent used as the medium for the solid-liquid separation is applied by spraying.

The temperature T or Tn of the mixture system of the solid and the solvent and the temperature T or Tn of the medium solvent may be identical or different as long as they meet the defined temperature range. For example, in the first solid-liquid separation, the temperature T of the mixture system of the solid and the solvent may be different from the temperature T of the subsequent medium solvent, but identical temperatures are preferred from the viewpoints of ease to operate and control and the like.

The temperature T in the first solid-liquid separation is controlled in a range of the melting point of the long-chain fatty acid to the melting point of the long-chain fatty acid + 15 °C, and the temperature Tn in at least one solid-liquid separation of the n subsequent solid-liquid separations is controlled in a range of the melting point of the long-chain fatty acid + 15 °C to the melting point of the N-long-chain acyl amino acid; more preferably, the temperature T in the first solid-liquid separation is controlled in a range of the melting point of the long-chain fatty acid to the melting point of the long-chain fatty acid + 10 °C, and the temperature Tn in at least one solid-liquid separation of the n subsequent solid-liquid separations is controlled in a range of the melting point of the long-chain fatty acid + 20 °C to the melting point of the N-long-chain acyl amino acid.

Unless otherwise specified, in the present invention, the temperature of the system and the temperature of the medium (if present) solvent are in the same temperature range in each solid-liquid separation. For example, if the temperature Tn of the system is controlled in a range of the melting point of the long-chain fatty acid to the melting point of the long-chain fatty acid + 15 °C, the temperature Tn of the medium solvent is similarly controlled in a range of the melting point of the long-chain fatty acid to the melting point of the long-chain fatty acid + 15 °C. From the viewpoint of ease to operate, it is preferred that the two Tn are not only in the same temperature range, but also have substantially identical values (temperature errors are permitted).

Preferably, three or more solid-liquid separations are conducted; the temperature in the first solid-liquid separation is controlled in a range of the melting point of the long-chain fatty acid to the melting point of the long-chain fatty acid + 8 °C, the temperature in at least one intermediate solid-liquid separation is controlled in a range of the melting point of the long-chain fatty acid + 8 °C to the melting point of the long-chain fatty acid + 18 °C, and the temperature in the last solid-liquid separation is controlled in a range of the melting point of the long-chain fatty acid + 24 °C to the melting point of the N-long-chain acyl amino acid.

Preferably, if water is used as the solvent in the preparation of the crude N-long-chain acyl amino acid product or the content of the long-chain fatty acid in the crude N-long-chain acyl amino acid product is 10% or greater, the temperature T in the first solid-liquid separation is controlled in a range of the melting point of the long-chain fatty acid to the melting point of the long-chain fatty acid + 6 °C, and the temperature Tn in at least one solid-liquid separation of the n subsequent solid-liquid separations is controlled in a range of the melting point of the long-chain fatty acid + 15 °C to the melting point of the N-long-chain acyl amino acid; more preferably, the temperature T in the first solid-liquid separation is controlled in a range of the melting point of the long-chain fatty acid to the melting point of the long-chain fatty acid + 3 °C, and the temperature Tn in at least one solid-liquid separation of the n subsequent solid-liquid separations is controlled in a range of the melting point of the long-chain fatty acid + 20 °C to the melting point of the N-long-chain acyl amino acid.

In some embodiments, when the long-chain fatty acid is lauric acid, the temperature T in the first solid-liquid separation is controlled at 60 °C or lower, and the temperature Tn in at least one solid-liquid separation of the n subsequent solid-liquid separations is controlled at 60 °C or higher; more preferably, the temperature T in the first solid-liquid separation is controlled at 50-60 °C, and the temperature Tn in at least one solid-liquid separation of the n subsequent solid-liquid separations is controlled at 65-70 °C.

For all of the above embodiments, it is preferred that the temperature substantially ascends among all solid-liquid separations. That is, the temperature in a next solid-liquid separation is equal to or higher than that of a previous solid-liquid separation. Preferably, 3, 4, 5 or 6 or more solid-liquid separations are conducted. Generally, in terms of the content of impurities and the convenience, cost-efficiency and efficiency of treatment, 3 solid-liquid separations are preferred.

In all of the above embodiments for removing impurities, when the solvent is an organic solvent or a mixed solution of water and an organic solvent, the organic solvent is preferably an organic solvent in which the long-chain fatty acid and the long-chain acyl amino acid are slightly soluble, practically insoluble, or insoluble, wherein slightly soluble, practically insoluble, or insoluble refers to that the solubility of the long-chain fatty acid and the N-long-chain acyl amino acid in the organic solvent at 20 °C is less than 1 g/100 g, preferably less than 0.01 g/100 g, and more preferably less than 0.001 g/100 g. For example, the organic solvent may be selected from petroleum ether and acetone.

It is surprisingly found that, since the temperature of the first solid-liquid separation is properly controlled, some impurities (such as low-melting-point impurities) can be selectively removed by filtration. When such impurities are removed before the temperature is raised, the system will not become pasty and can endure a higher temperature. Thus, for a better impurity removal effect, the temperature of the subsequent solid-liquid separation operations can be properly raised (this is not conceived in conventional purification operations, which usually use solvents at a normal or lower temperature for washing/elution). However, an excessively high temperature is also unnecessary, or otherwise, the solid may be melted. Specifically, the preferred temperature range may be slightly different depending on the kind of the long-chain fatty acid and the long-chain acyl amino acid.

For at least one solid-liquid separation, controlling the temperature Tn of the solvent/system at 60 °C or higher shall be interpreted as follows. For example, when 3 solid-liquid separations are conducted, the temperature in the first separation can be controlled in a range of 50-60 °C such as 50 °C, the temperature in the second separation can be controlled at 60 °C or higher, and the temperature in the third separation can be controlled at 60 °C or higher; alternatively, the temperature in the first separation can be controlled in a range of 50-60 °C such as 50 °C, the temperature in the second separation can be controlled in a range of 50-60 °C such as 50 °C, and the temperature in the third separation can be controlled at 60 °C or higher; alternatively, the temperature in the first separation can also be controlled in a range of 50-60 °C, the temperature in the second separation can be controlled at 60 °C or higher, and the temperature in the third separation can be controlled in a range of 50-60 °C. Preferably, the temperature in the first separation is controlled in a range of 50-60 °C, and preferably, followed by a gradually ascending temperature. Similar definitions and related principles are detailed in the medium solvent temperature gradient treatment section.

The n solid-liquid separations and the medium solvent temperature gradient treatment can be conducted simultaneously, or only one of the two may be conducted. From the viewpoint of ease of operation, only the n solid-liquid separations may be conducted.

In one embodiment, considering that the present invention utilizes the difference between the melting points of the long-chain fatty acid and the N-long-chain acyl amino acid, that the use of solvents with a certain temperature can promote the formation of solid and liquid phases in the mixture, and that the principle is widely applicable to the separation of components in solid mixtures, a method for separating components in a solid mixtures by using the difference in melting point is provided. This is a novel separation method different from conventional separation methods such as evaporation, distillation, crystallization, filtration, solvent extraction, absorption, adsorption, column chromatography, dialysis, permeation, ultrafiltration, and the like.

For separating a solid mixture, when a component having a lower content is an impurity, the separation of the component is equivalent to the process of removing an impurity.

The solid mixture described herein is not limited to a completely dry solid mixture, and may contain an organic solvent or water, for example, a crude product after a chemical reaction.

The present invention is particularly useful for separating a mixture comprising multiple components having similar physical properties. For example, for components with close solubilities, components that may form an azeotrope or components that are miscible after melting, separations with existing methods are difficult, but the method disclosed herein can achieve the purpose of separation.

Separations by utilizing the difference in melting point have been proposed in the prior art, which, however, mainly focus on separation by crystallization (such as CN102423542B), separation by crystallization and solubility (such as CN106590939B), and separation by direct discharge after melting (such as CN111039776A), and does not relate to the treatment with a solvent having a certain temperature to form solid and liquid phases in the mixture before a direct solid-liquid separation or a solid-liquid separation with the assistance of a medium solvent.

Specifically, the present invention provides a method for separating a high-melting-point component and a low-melting-point component in a solid mixture via a melting point difference, comprising: (a) adding a solvent to the mixture, (b) after the addition of the solvent, controlling the temperature T of the system in a range of the melting point of a low-melting-point component to the melting point of a high-melting-point component, (c) after the temperature control in the system, separating the solid and liquid phases, wherein the solvent is a solvent in which the components to be separated (i.e., the high-melting-point component and the low-melting-point component to be separated) are slightly soluble, practically insoluble, or insoluble, wherein slightly soluble, practically insoluble, or insoluble refers to that the solubility of the components to be separated in the solvent at 20 °C is less than 1 g/100 g, preferably less than 0.01 g/100 g, and more preferably less than 0.001 g/100 g; the boiling point of the solvent is greater than the melting point of the low-melting-point component, and the temperature T of the system is not greater than the boiling point of the solvent.

The mixture, which consists of the high-melting component and the low-melting component, is not limited to 2 components. For example, there may be 2-3 high-melting-point components and 2-3 low-melting-point components. The "temperature T in a range of the melting point of the low-melting-point component to ..." refers to that the temperature T is greater than the melting points of all of the low-melting-point components, and "temperature T in a range of ... to the melting point of the high-melting-point component" refers to that the temperature T is less than the melting points of all of the high-melting-point components. For ease of understanding, assuming that the mixture has 4 components A, B, C and D with melting points of 34 °C, 44 °C, 54 °C and 64 °C, respectively, if D is to be separated from the other components, A, B and C are the low-melting-point components, D is the high-melting-point component, and the temperature T should be set in a range of 54 °C to 64 °C. If it is desired to separate A and B from C and D, A and B are the low-melting-point components, and C and D are the high-melting-point components, and the temperature T should be set in a range of 44 °C to 54 °C. Certainly, after the separation of A and B from C and D is complete, the mixture of A and B can be separated into A and B and the mixture of C and D can be separated into C and D according to the method disclosed herein. Preferably, the method is used for separating two components.

Preferably, the difference between the melting points of the components to be separated is 10 °C or greater, more preferably 15 °C, 20 °C, 25 °C, 30 °C or 35 °C or greater. If there are a plurality of low-melting-point components and a plurality of high-melting-point components, the difference between the melting points of the components to be separated is the difference between the highest melting point of the low-melting-point components and the lowest melting point of the high-melting-point components.

Preferably, the percentage content by weight of the low-melting-point component is 50% or less, and more preferably 45%, 40%, 35%, 30%, 25%, 20%, 15%, 10% or 5% or less. The percentage content by weight is based on the entire mixture.

Preferably, the solid-liquid separation is conducted under the action of centrifugation or pressure; more preferably, the solid-liquid separation is promoted by using a solvent having a certain temperature as a medium that allows temperature T to be controlled in a range of the melting point of the low-melting-point component to the melting point of the high-melting-point component, and the solvent is a solvent in which the components to be separated are slightly soluble, practically insoluble, or insoluble, wherein slightly soluble, practically insoluble, or insoluble refers to that the solubility of the components to be separated in the solvent at 20 °C is less than 1 g/100 g, preferably less than 0.01 g/100 g, and more preferably less than 0.001 g/100 g. The medium solvent may be referred to in the related discussion in other embodiments.

Preferably, the solid-liquid separation meets one or more of the following conditions:
a, during the solid-liquid separation, the solvent used as the medium is contacted with the mixture to be separated, and under the action of centrifugation or pressure, the solvent removes the low-melting-point component to promote the separation;
b, the solvent used as the medium in the solid-liquid separation is provided by spraying;
c, the amount of the solvent used as the medium during the solid-liquid separation is greater than 0.5 times the mass of the solid mixture to be separated; and
d, the solid-liquid separation is conducted using an industrial centrifuge or a filter press, and preferably a filter centrifuge equipped with a filter screen or a filter cloth.

In one embodiment, the temperature T of the solvent as the medium is present in a plurality of temperature stages, and preferably the temperature in a next stage is equal to or higher than the temperature in a previous stage.

Preferably, the temperature in the first stage is controlled in a range of the melting point of the low-melting-point component to the melting point of the low-melting-point component + 10 °C or + 15 °C (for a great difference between the melting points of the high- and low-melting-point components, + 15 °C is preferred), and the temperature in at least one subsequent stage is controlled in a range of the melting point of the low-melting-point component + 10 °C or + 15 °C (for a great difference between the melting points of the high- and low-melting-point components, + 15 °C is preferred) to the melting point of the high-melting-point component; more preferably, the temperature in the first stage is controlled in a range of the melting point of the low-melting-point component to the melting point of the low-melting-point component + 10 °C, and the temperature in at least one subsequent stage is controlled in a range of the melting point of the low-melting-point component + 20 °C to the melting point of the high-melting-point component.

Preferably, if the percentage content by weight of the low-melting-point component is 10%-40%, and particularly 15%-30%, the temperature T of the solvent as the medium is present in a plurality of temperature stages, the temperature in the first stage is controlled in a range of the melting point of the low-melting-point component to the melting point of the low-melting-point component + 6 °C, and the temperature in at least one subsequent stage is controlled in a range of the melting point of the low-melting-point component + 15 °C to the melting point of the high-melting-point component; more preferably, the temperature in the first stage is controlled in a range of the melting point of the low-melting-point component to the melting point of the low-melting-point component + 3 °C, and the temperature in at least one subsequent stage is controlled in a range of the melting point of the low-melting-point component + 20 °C to the melting point of the high-melting-point component.

In one embodiment, after the first solid-liquid separation, n solid-liquid separations are conducted, wherein n is not less than 1, and preferably the temperature in a next solid-liquid separation is equal to or higher than the temperature in a previous solid-liquid separation.

Each solid-liquid separation comprises: mixing a solid obtained from a previous solid-liquid separation with a solvent, optionally stirring, controlling the temperature Tn of the mixture system in a range of the melting point of the low-melting-point component to the melting point of the high-melting-point component, and conducting the solid-liquid separation; or
alternatively: mixing a solid obtained from a previous solid-liquid separation with a solvent, optionally stirring, controlling the temperature Tn of the system in a range of the melting point of the low-melting-point component to the melting point of the high-melting-point component, and conducting the solid-liquid separation, wherein the solid-liquid separation is promoted by using a solvent having a certain temperature as a medium that allows temperature Tn to be controlled in a range of the melting point of the low-melting-point component to the melting point of the high-melting-point component.

In each solid-liquid separation, the solvent (including the medium solvent) is a solvent in which the components to be separated are slightly soluble, practically insoluble, or insoluble, wherein slightly soluble, practically insoluble, or insoluble refers to that the solubility of the components to be separated in the solvent at 20 °C is less than 1 g/100 g, preferably less than 0.01 g/100 g, and more preferably less than 0.001 g/100 g.

Preferably, the temperature T in the first solid-liquid separation is controlled in a range of the melting point of the low-melting-point component to the melting point of the low-melting-point component + 10 °C or + 15 °C (for a great difference between the melting points of the high- and low-melting-point components, + 15 °C is preferred), and the temperature Tn in at least one solid-liquid separation of the n subsequent solid-liquid separations is controlled in a range of the melting point of the low-melting-point component + 10 °C or + 15 °C to the melting point of the high-melting-point component; preferably, the temperature T in the first solid-liquid separation is controlled in a range of the melting point of the low-melting-point component to the melting point of the low-melting-point component + 10 °C, and the temperature Tn in at least one solid-liquid separation of the n subsequent solid-liquid separations is controlled in a range of the melting point of the low-melting-point component + 20 °C to the melting point of the high-melting-point component.

Preferably, three or more solid-liquid separations are conducted; the temperature T in the first solid-liquid separation is controlled in a range of the melting point of the low-melting-point component to the melting point of the low-melting-point component + 8 °C, the temperature Tn in at least on intermediate solid-liquid separation is controlled in a range of the melting point of the low-melting-point component + 8 °C to the melting point of the low-melting-point component + 18 °C, and the temperature Tn in the last solid-liquid separation is controlled in a range of the melting point of the low-melting-point component + 24 °C to the melting point of the high-melting-point component.

Preferably, if the percentage content by weight of the low-melting-point component is 10%-40%, and particularly 15%-30%, the temperature T in the first solid-liquid separation is controlled in a range of the melting point of the low-melting-point component to the melting point of the low-melting-point component + 6 °C, and the temperature Tn in at least one solid-liquid separation of the n subsequent solid-liquid separations is controlled in a range of the melting point of the low-melting-point component + 15 °C to the melting point of the high-melting-point component; more preferably, the temperature T in the first solid-liquid separation is controlled in a range of the melting point of the low-melting-point component to the melting point of the low-melting-point component + 3 °C, and the temperature Tn in at least one solid-liquid separation of the n subsequent solid-liquid separations is controlled in a range of the melting point of the low-melting-point component + 20 °C to the melting point of the high-melting-point component.

The "multiple solid-liquid separations" and the "temperature T of the solvent as the medium present in a plurality of temperature stages" may be satisfied simultaneously, or one of them may be satisfied.

In the above embodiments, the crude N-long-chain acyl amino acid product may be a commercially available N-long-chain acyl amino acid, and specific specifications and the like are not limited as long as it is any commercially available N-long-chain acyl amino acid. Some of the commercially available N-long-chain acyl amino acid products have high labeled purity, but actually contain a high content of impurities, because lauric acid does not absorb ultraviolet light and cannot be detected by a conventional liquid chromatograph (equipped with an ultraviolet detector). The purity is probably inaccurate, and the impurities must be analyzed by adopting high-performance liquid chromatography-mass spectrometry, high-performance liquid chromatography with a specific detector and the like. Commercially available N-long-chain acyl amino acid products can be further purified by the treatment according to the above embodiments and undergo structural reconstruction to give a product having a specific structure (an amino acid supramolecule with a specific structure).

In the above embodiments, the crude N-long-chain acyl amino acid product can be prepared by a method comprising the following steps: (1) reacting a raw material comprising an amino acid and a base to give an amino acid salt solution; (2) adding a long-chain acid halide and optionally a base into the resultant amino acid salt solution to give an N-long-chain acyl amino acid salt; and (3) acidifying the resultant N-long-chain acyl amino acid salt.

After the acidification in step (3), a solid-liquid separation operation such as filtration/centrifugal separation can be conducted to give the crude product, or the product can be directly used as the crude product without solid-liquid separation. A solid-liquid separation is preferred.

Alternatively, the crude N-long-chain acyl amino acid product is prepared by a method comprising the following steps: reacting an amino acid and/or a salt thereof with a long-chain acid halide in the presence of a base to give an N-long-chain acyl amino acid salt, acidifying the resultant N-long-chain acyl amino acid salt, gradually precipitating a solid, standing, separating solid and liquid phases, and optionally washing and drying to give the crude N-long-chain acyl amino acid product.

Alternatively, the crude N-long-chain acyl amino acid product is prepared by a method comprising the following steps: (1) dissolving an amino acid and a base in a mixed solution of water and an organic solvent and uniformly stirring to give an amino acid salt solution; (2) adding a long-chain acid halide and a base into the resultant amino acid salt solution and stirring to give an N-long-chain acyl amino acid salt; and (3) acidifying the resultant N-long-chain acyl amino acid salt, gradually precipitating a solid, standing, separating solid and liquid phases, and optionally washing and drying to give crude N-long-chain acyl amino acid product.

Furthermore, the crude N-long-chain acyl amino acid product is prepared by the following steps:
(1) dissolving an amino acid and a metal inorganic base in a mixed solution of water and an organic solvent and uniformly stirring to give an amino acid salt solution;
(2) sequentially adding a long-chain acid chloride and a metal inorganic base into the resultant amino acid salt solution and stirring to give an N-long-chain acyl amino acid salt; and
(3) acidifying the resultant N-long-chain acyl amino acid salt, gradually precipitating a solid, standing, and separating solid and liquid phases (e.g., by filtration, centrifugal separation and the like), to give crude N-long-chain acyl amino acid product.

From the viewpoint of the complete reaction of the long-chain acid chloride, the molar ratio of the amino acid to the long-chain acid chloride is 1:1 or greater, and preferably 1.2:1 or greater.

Furthermore, the crude N-long-chain acyl amino acid product is prepared by the following steps:
(1) dissolving an amino acid and a metal inorganic base in a mixed solution of water and an organic solvent and uniformly stirring to give an amino acid salt solution;
(2) sequentially adding a long-chain acid chloride and a metal inorganic base into the resultant amino acid salt solution and stirring at 0-50 °C (preferably a lower temperature, e.g., 0-25 °C) to give an N-long-chain acyl amino acid salt; and
(3) acidifying the resultant N-long-chain acyl amino acid salt, gradually precipitating a solid, standing for 1-5 hours at 0-30 °C (e.g., in an ice bath), and separating solid and liquid phases (e.g., by filtration, centrifugal separation and the like), to give crude N-long-chain acyl amino acid product.

In step (1), the molar ratio of the amino acid to the metal inorganic base is 1:(1-1.5). The metal inorganic base is selected from one or more of sodium hydroxide, potassium hydroxide, sodium carbonate, and potassium carbonate.

In step (1), the volume ratio of water to the organic solvent is 1:(1-1.5). In step (2), the concentration of the metal inorganic base is 30-80%.

In step (2), the metal inorganic base is selected from one or more of sodium hydroxide, potassium hydroxide, sodium carbonate, and potassium carbonate. The reaction system is controlled at pH 8-10 by controlling the addition amount of the metal inorganic base.

In step (2), the long-chain acid chloride is selected from one or more of octanoyl chloride, caprinoyl chloride, undecanoyl chloride, lauroyl chloride, myristoyl chloride, pentadecanoyl chloride, palmitoyl chloride, stearoyl chloride, oleoyl chloride, linoleoyl chloride, isostearoyl chloride, coconut oil fatty acid chloride, or palm oil fatty acid chloride, preferably coconut oil fatty acid chloride or lauroyl chloride, and most preferably lauroyl chloride.

In one embodiment, a method for preparing an amino acid supramolecule and a method for preparing a composition comprising an amino acid supramolecule are provided, wherein the amino acid supramolecules prepared by the above methods have structures and properties different from common commercially available long-chain acyl amino acids.

Specifically, the crude N-long-chain acyl amino acid product (whether comprising the dipeptide or not) is subjected to an impurity removal step according to the above embodiments, and a structural reconstruction occurs during the impurity removal process to form the amino acid supramolecule with a specific structure.

The percentage content by weight of the long-chain fatty acid in the amino acid supramolecule is 5% or less, preferably 4%, 3% or 2% or less and 0.1%, 0.2% or 0.5% or greater, and most preferably 0.5%-3%, in view of the difficulties in completely removing long-chain fatty acids by mild processes and the lack of cost advantages.

The present invention further provides an amino acid supramolecule, comprising a supramolecular structure self-assembled by an N-long-chain acyl amino acid and an N-long-chain acyl amino acid dipeptide. The percentage content by weight of an N-long-chain acyl amino acid dipeptide is 3% or greater, and preferably 4%, 5%, 6%, 7%, 8%, 9%, 10%, 11%, 12%, 13%, 14% or 15% or greater.

In one embodiment, the dipeptide has a moderate content, i.e., the percentage content by weight of the N-long-chain acyl amino acid dipeptide is 5% or greater, preferably 8% or greater, and more preferably 10% or greater, and less than 15%.

In one embodiment, the dipeptide has a high content, i.e., the percentage content by weight of the N-long-chain acyl amino acid dipeptide is 15% or greater, and preferably 20% or greater.

The percentage content by weight of the long-chain fatty acid in the amino acid supramolecule is 5% or less, preferably 4%, 3% or 2% or less and 0.1%, 0.2%, 0.5% or 1% or greater, and most preferably 0.5%-3%, in view of the difficulties in completely removing long-chain fatty acids by mild processes and the lack of cost advantages.

Preferably, the amino acid supramolecule of the present invention has a characteristic ion peak in a range of 541-545 in a mass spectrum detected on a mass spectrometer AB4500 with a scanning mode Q1SCAN, an ionization mode ESI (-), and a scanning range m/z = 200-600.

Preferably, the amino acid supramolecule of the present invention has 3 or 4 group peaks in the retention time range of 30-45 min in a high-performance liquid chromatogram detected on a high-performance liquid chromatograph equipped with an ultraviolet detector with a column of ODS-2 HYPERSIL C18 250 × 4.6 mm 5 µm, a wavelength of 210 nm, and a mobile phase of methanol:20 mmol/L potassium dihydrogen phosphate buffer (pH 3.0) = 70:30 (v/v).

Preferably, the solid powder of the amino acid supramolecule has a columnar, rod-shaped, thread-shaped, or rope-shaped micromorphology.

Preferably, the amino acid supramolecule has an initial melting temperature of 75 °C or higher, preferably 78 °C or higher, and more preferably 80 °C or higher, and a final melting temperature of 87 °C or higher, preferably 90 °C or higher, and more preferably 92 °C or higher, as measured by a capillary method. The final melting temperature of a conventional Schotten-Baumann reaction product is 77-84 °C, while the final melting temperature of the amino acid supramolecule of the present invention may reach 87 °C or higher and may increase along with the increase in dipeptide content.

Preferably, the amino acid supramolecule has a DSC peak temperature of 86 °C or higher, preferably 88 °C or higher, and more preferably 90 °C or higher, and the DSC peak shifts to the right with the increase in dipeptide content.

Preferably, the sodium salt of the amino acid supramolecule has a number-average molecular weight of 5,000-250,000, preferably in a range of 6,000, 7,000, 8,000, 9,000, 10,000, 11,000, 12,000, 13,000, 14,000, 15,000, 16,000, 17,000, 18,000, 19,000 or 20,000 to 240,000, 230,000, 220,000, 210,000, 200,000, 190,000, 180,000, 170,000, 160,000, 150,000, 140,000, 130,000, 120,000 or 110,000, more preferably 10,000-150,000, and most preferably 15,000-100,000.

In the present invention, unless otherwise specified, the long-chain fatty acid is a saturated or unsaturated linear or branched fatty acid with 8-22 carbon atoms. Preferred specific examples include one or more of octylic acid, capric acid, undecanoic acid, lauric acid, myristic acid, pentadecanoic acid, palmitic acid, stearic acid, oleic acid, linoleic acid, isostearic acid, coconut oil fatty acid, or palm oil fatty acid, preferably coconut oil fatty acid or lauric acid, and most preferably lauric acid.

The amino acid is selected from one or more of glycine, alanine, glutamic acid, sarcosine, aspartic acid, leucine, isoleucine, valine, threonine, proline, phenylalanine, arginine, and lysine, preferably alanine, glycine, glutamic acid, sarcosine, arginine or lysine, and most preferably L-alanine. The described amino acid also refers to the amino acids from which the crude long-chain acyl amino acid product/long-chain acyl amino acid is derived (i.e., the amino acids used to synthesize the crude long-chain acyl amino acid product/long-chain acyl amino acid).

The N-long-chain acyl in the crude N-long-chain acyl amino acid product/N-long-chain acyl amino acid is derived from a saturated or unsaturated linear or branched fatty acid with 8-22 carbon atoms. Preferably, the N-long-chain acyl group is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl.

The long chain in the crude N-long-chain acyl amino acid product/N-long-chain acyl amino acid is identical to the long chain in the long-chain fatty acid.

In all embodiments, the amino acid supramolecule salt is formed from the amino acid supramolecule and a base. The base is not particularly limited, and includes inorganic bases and organic bases, specifically, inorganic bases represented by those of alkali metals such as sodium and potassium or alkaline earth metals such as magnesium and calcium, and organic bases represented by organic amines such as amines and alkanolamines or basic amino acids such as lysine, arginine and histidine. These bases may be used alone, or in combination of 2 or more.

For the inorganic base, sodium hydroxide and potassium hydroxide are preferred and particularly sodium hydroxide. For the organic base, basic amino acids are preferred, particularly arginine or lysine.

It is also surprisingly found that salts formed by the reaction of basic amino acids such as arginine with the amino acid supramolecule in practical application produce a higher amount of foam with higher density, good duration, softer feel, elasticity, and superior detergency performance. In addition, salts formed by the reaction of lysine and the amino acid supramolecule possess good solubility and system stability, and produce the most stable foam.

The present invention further provides an amino acid supramolecule salt prepared from the amino acid supramolecule described above and a basic amino acid. The basic amino acid is selected from arginine, lysine, and histidine, and preferably arginine and lysine. The amino acid supramolecule salt can be used in a detergent, an emulsifier, a healthcare composition, or a cosmetic.

The present invention further provides an amino acid supramolecule or a salt thereof prepared by the above embodiments. Also provided is use of the amino acid supramolecule or the salt thereof described above as a surfactant or emulsifier.

Also provided is use of the amino acid supramolecule or the salt thereof described above in preparing a detergent composition, a toothpaste, a healthcare composition, a laundry liquid, a soap, a laundry powder, a dish detergent, a facial mask, a shampoo, a body wash, a facial cleanser, a makeup remover, a mouth wash, a shaving product, a hand sanitizer, a cleaning lotion, a cleansing cream, or the like.

Due to the special spatial structure, also provided is use of the amino acid supramolecule or the salt thereof described above in absorbing oil stains or microorganisms, or in sterilizing, deodorizing or removing pesticide residues.

The present invention provides an amino acid-based dish detergent, an amino acid-based laundry liquid, an amino acid-based toothpaste, a skin care composition, an amino acid-based soap, an amino acid-based laundry powder, an amino acid-based facial mask, an amino acid-based shampoo, an amino acid-based body wash, or an amino acid-based facial cleanser, comprising the amino acid supramolecule or salt thereof described above. The amino acid-based products described in the present invention are named after the inclusion of the amino acid supramolecule and/or the salt thereof, and are not particularly limited by the amino acids.

The present invention provides a toothpaste, comprising a friction agent, a humectant, a thickening agent, and a surfactant. The surfactant comprises the amino acid supramolecule and/or the salt thereof described above. The toothpaste comprises, by weight of the toothpaste, 0.1-25 wt% of the surfactant, 10-50 wt% of the friction agent, 5-40 wt% of the humectant, and 0.1-6 wt% of thickening agent. Alternatively, the surfactant is completely composed of the amino acid supramolecule and/or the salt thereof disclosed herein, or an amino acid surfactant is completely composed of the amino acid supramolecule and/or the salt thereof disclosed herein. Preferably, the surfactant comprises 20 wt% or greater, more preferably 30, 40, 50, 60, 70, 80 or 90 wt% or greater, even more preferably 100 wt% of the amino acid supramolecule and/or the salt thereof disclosed herein.

The friction agent is selected from one or a mixture of more of hydrated silica, calcium carbonate, and calcium hydrophosphate. The humectant is one or a mixture of more of sorbitol, polyethylene glycol-400, glycerol, and propylene glycol. The thickening agent is selected from one or a mixture of more of carboxymethyl cellulose, xanthan gum, carrageenan, carbomer, poloxamer 407, and magnesium aluminum silicate.

The toothpaste also comprises the following substances in percentage by weight: 0.1-0.3% of a sweetener, 0.5-1.5% of an essence, 5-10% of water, 0.3-0.5% of a Chinese herbal medicine extract, 0.3-0.5% of a preservative, and 0.05-0.15% of a colorant. The sweetener is selected from one or a mixture of more of sodium saccharin, xylitol, and erythritol. The Chinese herbal medicine extract is selected from one or a mixture of more of a *Chondrus crispus* extract, a *Glycyrrhiza uralensis* extract, and a *Portulaca oleracea* extract. The preservative is selected from one or a mixture of more of sodium benzoate, a paraben, triclosan/copolymer, and a biological lysozyme. The colorant is selected from one or a mixture of more of CI77019, CI77891, CI42090, CI19140, mica, titanium dioxide, and brilliant blue. The amino acid-based toothpaste does not comprise sodium dodecyl sulfate.

The present invention provides a method of preparing the toothpaste described above, comprising: (1) preparing an aqueous solution from water, a sweetener, a preservative and a humectant, and adding the aqueous solution into a paste-making machine; (2) mixing a thickening agent, a friction agent and a Chinese herbal medicine extract, adding the mixture into the paste-making machine, stirring and milling until the paste is uniform, and defoaming in vacuum; and (3) sequentially adding a surfactant, an essence and a colorant into the paste-making machine, stirring and milling until the paste is uniform, and defoaming to give the toothpaste.

The present invention provides a skin care composition comprising, by weight:
50-95 wt% of an oil,
0.5-30 wt% of a surfactant, and
0-45 wt% of a suspended particle.

The surfactant comprises the amino acid supramolecule and/or the salt thereof described above. Alternatively, the surfactant is completely composed of the amino acid supramolecule and/or the salt thereof disclosed herein, or an amino acid surfactant is completely composed of the amino acid supramolecule and/or the salt thereof disclosed herein. Preferably, the surfactant comprises 20 wt% or greater, more preferably 30, 40, 50, 60, 70, 80 or 90 wt% or greater, even more preferably 100 wt% of the amino acid supramolecule and/or the salt thereof disclosed herein.

The oil is selected from a natural oil, a synthetic oil, or a mixture thereof with a freezing point of -50 °C to 6 °C. The natural oil includes vegetable oils and animal oils. The vegetable oil includes grape seed oil, sunflower seed oil, jojoba oil, aloe oil, olive oil, linseed oil, safflower seed oil, soybean oil, almond oil, tea oil, or any mixture thereof. The animal oil includes horse oil and sheep oil. The synthetic oil includes isodecyl neodecanoate and neopentyl glycol diheptanoate.

The content of the oil is 65 wt% or 85 wt%.

The suspended particle has a particle size of less than 30 µm, preferably less than 15 µm, and more preferably less than 5 µm.

The suspended particle is selected from an oil-insoluble solid particle or an oil-immiscible liquid. The oil-insoluble solid particle includes mica, starch, zinc oxide, titanium dioxide, talc powder, and a silicone elastomer. The oil-immiscible liquid includes glycerol, water, and a polyol.

The present invention provides a method for preparing the skin care composition described above, comprising: (1) mixing an oil, a surfactant and a suspended particle in percentages by weight of (50-95%):(0.5-30%):(0-45%), stirring at 82-87 °C for reaction, and cooling to 65-72 °C by stirring the mixture after the surfactant is completely dissolved in the oil; and (2) cooling the resultant mixture to room temperature to give the skin care composition.

The present invention provides a laundry liquid, comprising a surfactant, a softening agent, a chelating agent, deionized water, a preservative, and an essence in the following percentages by weight:
5-50% of the surfactant,
0.1-3% of the softening agent,
0.1-5% of the chelating agent,
50-90% of deionized water,
0.1-6% of the preservative,
0.1-2% of the essence,

The surfactant comprises the amino acid supramolecule and/or the salt thereof described above. Alternatively, the surfactant is completely composed of the amino acid supramolecule and/or the salt thereof disclosed herein, or an amino acid surfactant is completely composed of the amino acid supramolecule and/or the salt thereof disclosed herein. Preferably, the surfactant comprises 20 wt% or greater, more preferably 30, 40, 50, 60, 70, 80 or 90 wt% or greater, even more preferably 100 wt% of the amino acid supramolecule and/or the salt thereof disclosed herein.

The present invention provides a soap, comprising a surfactant, a fatty acid, glycerol, a softening agent, a chelating agent, a filler, and deionized water in the following percentages by weight:
10-50% of the surfactant,
0.1-7% of the fatty acid,
0.1-5% of glycerol,
0.1-6% of the softening agent,
0.1-1% of the chelating agent,
10-40% of the filler, and
1-5% of deionized water.

The surfactant comprises the amino acid supramolecule and/or the salt thereof described above. Alternatively, the surfactant is completely composed of the amino acid supramolecule and/or the salt thereof disclosed herein, or an amino acid surfactant is completely composed of the amino acid supramolecule and/or the salt thereof disclosed herein. Preferably, the surfactant comprises 20 wt% or greater, more preferably 30, 40, 50, 60, 70, 80 or 90 wt% or greater, even more preferably 100 wt% of the amino acid supramolecule and/or the salt thereof disclosed herein.

The present invention provides a laundry powder, comprising a surfactant and a friction agent in the following percentages by weight:
10-50% of the surfactant, and
50-90% of the friction agent,

The surfactant comprises the amino acid supramolecule and/or the salt thereof described above. Alternatively, the surfactant is completely composed of the amino acid supramolecule and/or the salt thereof disclosed herein, or an amino acid surfactant is completely composed of the amino acid supramolecule and/or the salt thereof disclosed herein. Preferably, the surfactant comprises 20 wt% or greater, more preferably 30, 40, 50, 60, 70, 80 or 90 wt% or greater, even more preferably 100 wt% of the amino acid supramolecule and/or the salt thereof disclosed herein.

The present invention provides a dish detergent, comprising a surfactant, deionized water, a thickening agent, glycerol, a preservative, and an essence in the following percentages by weight:
5-20% of the surfactant,
70-90% of deionized water,
1-2% of the thickening agent,
5-10% of glycerol,
0.1-6% of the preservative, and
0.1-2% of the essence,

The surfactant comprises the amino acid supramolecule and/or the salt thereof described above. Alternatively, the surfactant is completely composed of the amino acid supramolecule and/or the salt thereof disclosed herein, or an amino acid surfactant is completely composed of the amino acid supramolecule and/or the salt thereof disclosed herein. Preferably, the surfactant comprises 20 wt% or greater, more preferably 30, 40, 50, 60, 70, 80 or 90 wt% or greater, even more preferably 100 wt% of the amino acid supramolecule and/or the salt thereof disclosed herein.

The present invention provides a facial mask, comprising a surfactant, deionized water, glycerol, a preservative, and an essence in the following percentages by weight:
0.1-5% of the surfactant,
50-90% of deionized water,
1-10% of glycerol,
0.1-2% of the preservative, and
0.1-2% of the essence.

The surfactant comprises the amino acid supramolecule and/or the salt thereof described above. Alternatively, the surfactant is completely composed of the amino acid supramolecule and/or the salt thereof disclosed herein, or an amino acid surfactant is completely composed of the amino acid supramolecule and/or the salt thereof disclosed herein. Preferably, the surfactant comprises 20 wt% or greater, more preferably 30, 40, 50, 60, 70, 80 or 90 wt% or greater, even more preferably 100 wt% of the amino acid supramolecule and/or the salt thereof disclosed herein.

The present invention will be further illustrated by the following examples in conjunction with the accompanying drawings. It will be appreciated that these examples are merely intended to illustrate the present invention rather than limit the scope of the present invention. Furthermore, it will be appreciated that various changes or modifications of the present invention can be made by those skilled in the art after reading the teachings of the present invention, and such equivalents also fall within the scope of the appended claims of the present application.

The present invention will be further illustrated with reference to the following specific examples.

### Preparation Examples Synthesis of crude N-lauroyl-L-alanine product

### Preparation Example 1

At room temperature, in a 1000-L reaction kettle, 89 kg (1 kmol) of L-alanine and 40 kg (1 kmol) of sodium hydroxide were dissolved in a mixed solution of 150 L of distilled water and 150 L of acetone, and the mixture was uniformly stirred to give a sodium L-alaninate solution.

At 20 °C, 175 kg (0.8 kmol) of lauroyl chloride was slowly added dropwise to the sodium L-alaninate solution, and a 50% sodium hydroxide solution was added dropwise to adjust the reaction system to pH 9. After the addition, the system was stirred at 20 °C for 1.5 h to give a pasty N-lauroyl-L-alanine salt.

Hydrochloric acid was added into the pasty N-lauroyl-L-alanine salt for acidification to pH 3-4.

A white solid was gradually precipitated. The system was incubated for 3 h in an ice bath, and then filtered to give a crude N-lauroyl-L-alanine product.

### Preparation Example 2

At room temperature, in a 1000-L reaction kettle, 89 kg (1 kmol) of L-alanine and 56 kg (1 kmol) of potassium hydroxide were dissolved in a mixed solution of 150 L of distilled water and 150 L of acetone, and the mixture was uniformly stirred to give a potassium L-alaninate solution.

At 20 °C, 218.7 kg (1 kmol) of lauroyl chloride was slowly added dropwise to the L-alanine salt solution, and a 50% potassium hydroxide solution was added dropwise to adjust the reaction system to pH 9. After the addition, the system was stirred at 20 °C for 2 h to give a pasty N-lauroyl-L-alanine salt.

Hydrochloric acid was added into the pasty N-lauroyl-L-alanine salt for acidification to pH 3-4. A white solid was gradually precipitated. The system was incubated for 2 h in an ice bath, and then filtered to give a crude N-lauroyl-L-alanine product.

### Preparation Example 3

At room temperature, in a 1000-L three-necked flask, 89 kg (1 kmol) of L-alanine and 106 kg (1 kmol) of sodium carbonate were dissolved in a mixed solution of 150 L of distilled water and 150 L of acetone, and the mixture was uniformly stirred to give a sodium L-alaninate solution.

At 20 °C, 218.7 kg (1 kmol) of lauroyl chloride was slowly added dropwise to the L-alanine salt solution, and a 30% potassium hydroxide solution was added dropwise to adjust the reaction system to pH 8. After the addition, the system was stirred at 20 °C for 3.5 h to give a pasty N-lauroyl-L-alanine salt.

Hydrochloric acid was added into the pasty N-lauroyl-L-alanine salt for acidification to pH 3-4. A white solid was gradually precipitated. The system was incubated for 3 h in an ice bath, and then filtered to give a crude N-lauroyl-L-alanine product.

### Examples Compositions/crude products comprising N-lauroyl alanyl alanine (N-lauroyl alanine dipeptide)

### Example #14

100 g of water, 79 g of acetone, 60 g of alanine, and 20 g of caustic soda flakes were added into a reaction flask and stirred uniformly. 100 g of lauroyl chloride was dropwise added with the reaction temperature controlled at 20-30 °C. After the addition, the system was about pH 6-7 and incubated for 20 min. Hydrochloric acid was dropwise added to adjust the mixture to pH 1-2, and the mixture was filtered.

### Example #20

100 g of water, 79 g of acetone, 77 g of alanine, and 20 g of caustic soda flakes were added into a reaction flask and stirred uniformly. 100 g of lauroyl chloride was dropwise added with the reaction temperature controlled at 20-30 °C. After the addition, the system was about pH 6-7 and incubated for 20 min. Hydrochloric acid was dropwise added to adjust the mixture to pH 1-2, and the mixture was filtered.

### Example #27

100 g of water, 79 g of acetone, 53 g of alanine, and 20 g of caustic soda flakes were added into a reaction flask and stirred uniformly. 100 g of lauroyl chloride was dropwise added with the reaction temperature controlled at 20-30 °C. After the addition, the system was about pH 6-7 and incubated for 20 min. Hydrochloric acid was dropwise added to adjust the mixture to pH 1-2, and the mixture was filtered.

### Example #31

275 g of water, 216 g of acetone, 122.2 g of alanine, and 20 g of caustic soda flakes were added into a reaction flask and stirred uniformly. 100 g of lauroyl chloride was dropwise added with the reaction temperature controlled at 20-30 °C. After the addition, the system was supplemented with 10 g of caustic soda flakes and incubated for 20 min. Hydrochloric acid was dropwise added to adjust the mixture to pH 1-2, and the mixture was filtered.

### Example #33

500 g of water, 390 g of acetone, 49 g of alanine, and 100 g of caustic soda flakes were added into a reaction flask and stirred uniformly. 500 g of lauroyl chloride was dropwise added with the reaction temperature controlled at 20-30 °C. After the addition, the system was incubated for 20 min. Hydrochloric acid was dropwise added to adjust the mixture to pH 1-2, and the mixture was filtered.

### Example #501

100 kg of water, 140 kg of acetone, 110 kg of alanine, and 120 kg of lye (32%) were added into a reaction kettle and stirred uniformly. 180 kg of lauroyl chloride was dropwise added with the reaction temperature controlled at 42 °C. After the addition, the pH value of the system was about 5. Hydrochloric acid was dropwise added to adjust the mixture to pH 1-2. The mixture was filtered and washed with pure water.

### Example #502

550 kg of water, 110 kg of alanine, and 125 kg of lye (32%) were added into a reaction kettle and stirred uniformly. 180 kg of lauroyl chloride was dropwise added with the reaction temperature controlled at 27 °C. After the addition, the pH value of the system was about 5. Hydrochloric acid was dropwise added to adjust the mixture to pH 1-2. The mixture was filtered and washed with pure water.

### Example #503

100 kg of water, 140 kg of acetone, 97 kg of alanine, and 150 kg of lye (32%) were added into a reaction kettle. 180 kg of lauroyl chloride was dropwise added with the reaction temperature controlled at 30 °C. After the addition, the pH value of the system was about 5-6. Hydrochloric acid was dropwise added to adjust the mixture to pH 1-2. The mixture was filtered and washed with pure water.

### Example #601

100 kg of water, 90 kg of acetone, 103 kg of alanine, and 160 kg of lye (32%) were added into a reaction kettle and stirred uniformly. 180 kg of lauroyl chloride was dropwise added with the reaction temperature controlled at about 25 °C. Hydrochloric acid was dropwise added to adjust the mixture to pH 1-2. The mixture was filtered and washed with pure water.

### Example #602

100 kg of water, 140 kg of acetone, 103 kg of alanine, and 145 kg of lye (32%) were added into a reaction kettle and stirred uniformly. 90 kg of lauroyl chloride was dropwise added with the reaction temperature controlled at 25 °C, and 90 kg of lauroyl chloride and 51.5 kg of lye (32%) were added. Hydrochloric acid was dropwise added to adjust the mixture to pH 1-2. The mixture was filtered and washed with pure water.

### Example #603

100 kg of water, 140 kg of acetone, 103 kg of alanine, and 145 kg of lye (32%) were added into a reaction kettle and stirred uniformly. 135 kg of lauroyl chloride was dropwise added with the reaction temperature controlled at 25 °C, and 45 kg of lauroyl chloride and 50 kg of lye (32%) were simultaneously and dropwise added. Hydrochloric acid was dropwise added to adjust the mixture to pH 1-2. The mixture was filtered and washed with pure water.

### Example #604

100 kg of water, 140 kg of acetone, 81 kg of alanine, and 150 kg of lye (32%) were added into a reaction kettle and stirred uniformly. 90 kg of an acid chloride was dropwise added, and after the addition, 90 kg of lauroyl chloride and 50 kg of lye (32%) were added. Hydrochloric acid was dropwise added to adjust the mixture to pH 1-2. The temperature was controlled at 20-30 °C throughout the process. The mixture was filtered and washed with pure water.

**Table I.**

| Examples | Alanine/sodium hydroxide Molar ratio | pH after addition of lye*¹ | pH after addition of lauroyl chloride | Monopeptide*² | Dipeptide*³ |
|---|---|---|---|---|---|
| 14# | 1.35 | 9-10 | 6-7 | 67.8% | 21.9% |
| 20# | 1.73 | 9-10 | 6-7 | 75.2% | 17.9% |
| 27# | 1.19 | 10 | 6-7 | 62.8% | 21.9% |
| 31# | 1.83 | Batched addition of base | 7 | 85.6% | 7.8% |
| 33# | 0.22 | 14 | 9 | 95.9% | 0.2% |
| 501# | 1.29 | 10 | 5 | 68.2% | 25.6% |
| 502# | 1.24 | 10 | 5 | 64.5% | 25.2% |
| 503# | 0.91 | 11-12 | 5-6 | 70.6% | 18.0% |
| 601# | 0.90 | 11-12 | 6-7 | 74.3 | 16.9% |
| 602# | 0.74 | Batched addition of base | 7 | 84.1% | 9.7% |
| 603# | 0.74 | Batched addition of base | 7 | 83.0% | 10.4% |
| 604# | 0.57 | Batched addition of base | 7-8 | 93.7% | 3.0% |

| | | | | | |
|---|---|---|---|---|---|
| *1. The pH value was tested by pH strips *2. Monopeptide refers to lauroyl alanine *3. Dipeptide refers to lauroyl alanyl alanine | | | | | |

It can be seen from the results that, for a lower amount of sodium hydroxide, a precipitate was gradually produced from the start of the reaction (e.g., #31), resulting in a reduced yield. When an excessive amount of sodium hydroxide was added (e.g., #33), the pH was above 8 throughout the reaction, which is unfavorable for dipeptide production. The reaction temperature is preferably 35 °C or lower, or otherwise, the acid chloride hydrolysis may increase, resulting in a reduced yield (e.g., #501). Water or a mixed solution of water and an organic solvent can be selected as the reaction solvent. The use of water as the reaction solvent (e.g., #502) may lead to a higher dipeptide content but a reduced yield.

### [Content assay of monopeptide and dipeptide]

The content assay adopts the "Method II. High-performance liquid chromatography" below, and the results are shown in Table I.

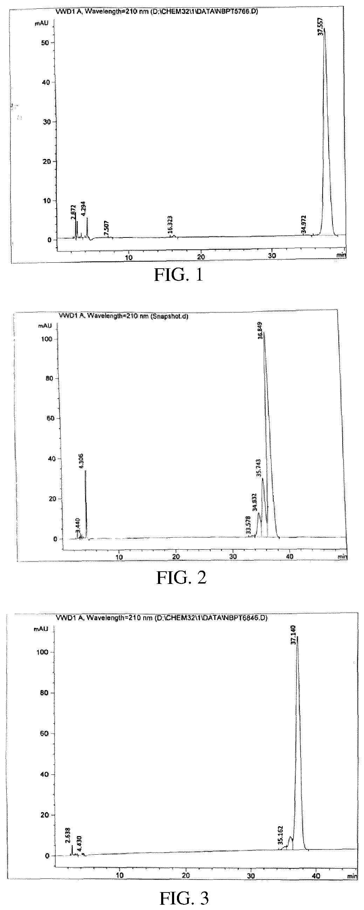

Typical chromatograms are shown in FIG. 1 (Example #33), FIG. 2 (Example #503), and FIG. 3 (Example #604). It can be seen that if the pH is controlled at 8 or higher after the lauroyl chloride is completely added (in a conventional process), only group peaks of 1-2 peaks may be found in the retention time range of 30-40 min in a high-performance liquid chromatogram (FIG. 1). If the pH is controlled at 8 or lower after the addition of lauroyl chloride, group peaks of 3-4 peaks may be found in the range of 30-40 min (FIGs. 2 and 3).

### Examples Removal of impurities and formation of amino acid supramolecules with specific structure

### Example 1

The crude N-lauroyl-L-alanine product obtained in Preparation Example 1 was mixed with water in a reaction kettle. The system was stirred uniformly, heated, controlled at 55 °C, and transferred into a filter centrifuge (equipped with a filter screen) for the first solid-liquid separation. The liquid phase was removed by centrifugation, and hot water at 55 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

Water was added into the reaction kettle before the solid residue of the centrifugation was transferred into the reaction kettle. The system was stirred, heated, controlled at 65 °C, and transferred into a filter centrifuge for the second solid-liquid separation. The liquid phase was removed by centrifugation, and hot water at 65 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

Water was added into the reaction kettle before the solid residue was transferred into the reaction kettle. The system was stirred, heated, controlled at 65 °C, and transferred into a filter centrifuge for the third solid-liquid separation. The liquid phase was removed by centrifugation, and hot water at 65 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up. The mixture was dried to give the amino acid supramolecule.

### [Content assay of lauric acid]

### Method 1. Ultra-performance liquid chromatography-mass spectrometry (ACQUITY I-Class PDA QDa)

### Chromatography

System: ACQUITY I-Class
□ Column: ACQUITY UPLC^{®} BEH, C18 2.1 × 50 mm, 1.7 µm
□ Mobile phase A: 10 mM NH4FA in water; mobile phase B: ACN
□ Column temperature: 40 °C; sample chamber temperature: 10 °C; injection volume: 4 µL
□ Solution preparation: solvent: methanol

- Lauric acid standard solution: lauric acid, 1-50 µg/mL;
- Sample solution: amino acid supramolecule product of Example 1, 0.5 mg/mL.

### Mass spectrometry

### MS System: QDa

Ionization mode: ESI(-); Capillary voltage: 0.8 kV; Taper hole voltage: 20 V; Probe temperature: 600 °C; Scanning mode: -SIR: lauric acid: 199.28 (exact mass: 200.18); -Full scan: 50-500.

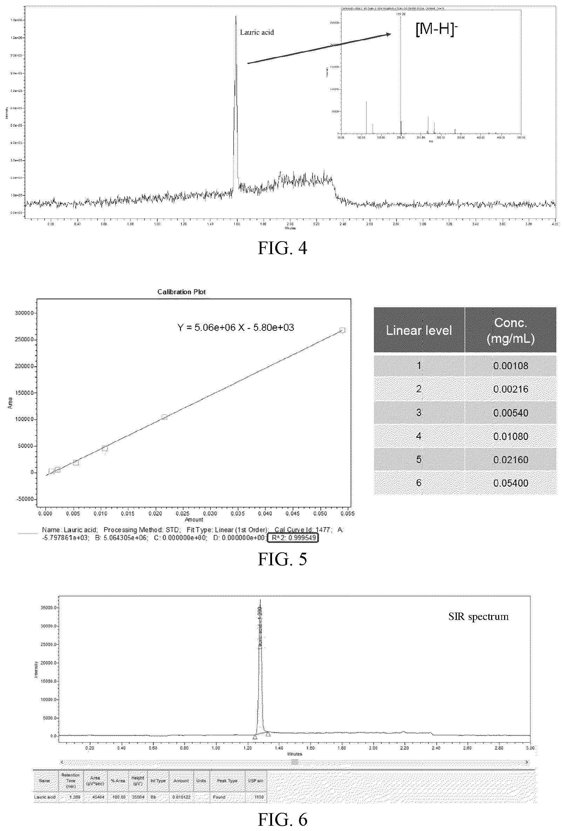

Confirmation of lauric acid: in the ESI(-) mode, a spectrum was acquired in the full scan mode (m/z = 50-500), as shown in FIG. 4. The lauric acid parent ion was [M-H]- with an m/z = 199.28. Rt = 1.59 min. In the subsequent quantitative analysis, the SIR chromatographic peak area was acquired according to the parent ion with m/z = 199.28.

Linearity and range: lauric acid has good linearity in the range of 1-50 µg/mL with an R² = 0.999549. The standard curve is shown in FIG. 5.

By a quantitative analysis through the external standard method using a standard curve, the lauric acid content in the sample solution of Example 1 was 0.010122 mg/mL. The relevant SIR spectrum is shown in FIG. 6.

### Method 2. Ultra-performance liquid chromatography

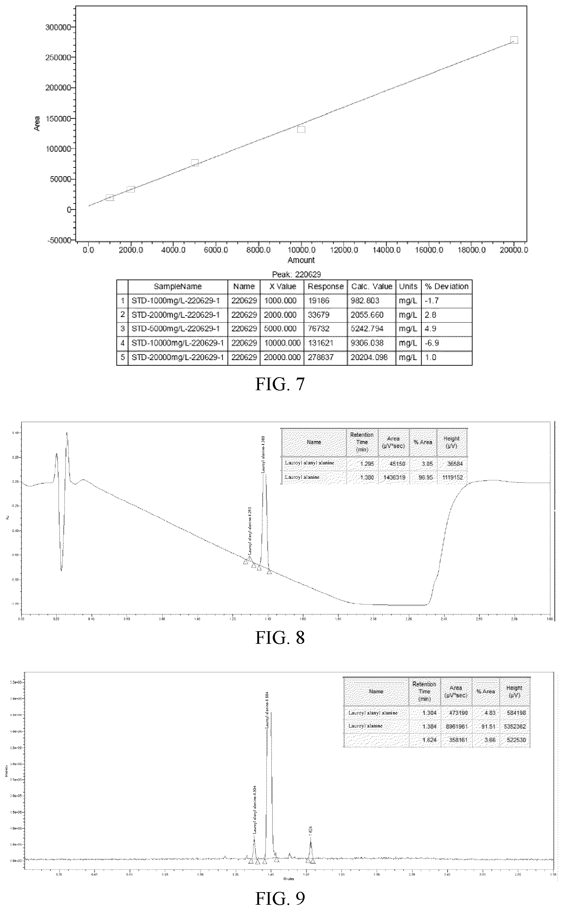

System: Waters UPLC H-Class
Detector: PDA detector
Column: Waters XBridge^{®}, C18 3.0 × 100 mm, 3.5 µm
Mobile phase: methanol:0.1% H₃PO₄ = 80:20
Column temperature: 35 °C; sample chamber temperature: not available; injection volume: 1 µL
Solution preparation: solvent: methanol
Lauric acid standard solution, lauric acid, 1000-20000 mg/L; the standard curve is shown in FIG. 7.

The test results are shown in Table II.

**Table II.**

| Test method | Lauric acid content (percentage content by weight) |
|---|---|
| Method 1 | 1.9% |
| Method 2 | 1.4% |

### [Content of lauroyl alanine and lauroyl alanyl alanine]

### Method I. Ultra-performance liquid chromatography-ultraviolet-mass spectrometry (ACOUITY I-Class PDA QDa)

### Chromatography

System: ACQUITY I-Class
□ Column: ACQUITY UPLC^{®} BEH, C18 2.1 × 50 mm, 1.7 µm
□ Wavelength: 210 nm
□ Mobile phase A: 0.1% FA and 5 mM NH4FA in water; mobile phase B: ACN
□ Column temperature: 40 °C; sample chamber temperature: 10 °C; injection volume: 2 µL
□ Solution preparation: solvent: methanol

- Sample solution: amino acid supramolecule product of Example 1, 3 mg/mL.

### Mass spectrometry

MS System: QDa
Ionization mode: ESI(-); Capillary voltage: 0.8 kV; Taper hole voltage: 20 V; Probe temperature: 600 °C; Scanning mode: -Full scan: 50-500

In the ESI(-) mode, signals of full scan mode were acquired (m/z = 50-500). At a wavelength of 210 nm, only two distinct chromatographic peaks could be seen; while in the QDa full scan mode, three distinct chromatographic peaks were visible.

The spectrum of the PDA channel was integrated, as shown in FIG. 8. The calculation by Empower suggested that the content in the sample was 3.05% for lauroyl alanyl alanine (dipeptide) and 96.95% for lauroyl alanine. It should be noted that in the normalized contents, only the compounds having an absorbance at the wavelength of 210 nm were considered.

The spectrum of the QDa full scan channel was integrated, as shown in FIG. 9. The calculation by Empower suggested that the content in the sample was 4.83% for lauroyl alanyl alanine (dipeptide) and 91.51% for lauroyl alanine. It should be noted that in the normalized contents, the difference in ionization efficiency between different compounds is not considered.

### Method II. High-performance liquid chromatography

In high-performance liquid chromatography, an ultraviolet detector was used to identify and determine lauroyl alanine and lauroyl alanyl alanine. Lauroyl alanine and lauroyl alanyl alanine in the samples were identified by comparing the retention times with those of the lauroyl alanine and lauroyl alanyl alanine standards, and were quantified by area normalization.
System: high-performance liquid chromatograph equipped with an ultraviolet detector
□ Column: ODS-2 HYPERSIL C18 250 × 4.6 mm, 5 µm
□ Wavelength: 210 nm
□ Mobile phase: methanol:20 mmol/L potassium dihydrogen phosphate buffer (pH 3.0) = 70:30 (v/v)
□ Column temperature: 30 °C; injection volume: 2 µL
Sample detection: The system parameters were adjusted according to chromatographic conditions. When the baseline of the system was stabilized, standard solutions and sample solutions were separately injected at 20 µL to the chromatographic column. Chromatograms of the lauroyl alanine standard solutions and the sample solutions were recorded. The chromatographic peaks of lauroyl alanine and lauroyl alanyl alanine in the sample were identified according to the retention time of the standard solution. The percentage contents of the test substances were determined by area normalization according to the peak areas of the sample.

The results are shown in Table III.

**Table III.**

| Test method | Lauroyl alanine content | Lauroyl alanyl alanine content |
|---|---|---|
| Method I - Integration of PDA channel spectrogram | 96.95% | 3.05% |
| Method I - Integration of QDa full scan channel spectrogram | 91.51% | 4.83% |
| Method II | 94.22% | 2.53% |

### [DSC analysis]

System: differential scanning calorimeter DSC2500;
Conditions: A certain amount of dry sample was weighed and transferred to a container with a punctured cap, without compression;
Temperature range: -50 °C to 150 °C;
Temperature ramp: 10 °C per minute.

The DSC analysis results of the product in Example 1 are shown in FIG. 10.

### Example 2

The crude N-lauroyl-L-alanine product obtained in Preparation Example 2 was mixed with water in a reaction kettle. The system was stirred uniformly, heated, controlled at 50 °C, and transferred into a filter centrifuge (equipped with a filter screen) for the first solid-liquid separation. During the separation, hot water at 50 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

The solid residue was transferred into the reaction kettle and water was added. The system was stirred, heated, controlled at 60 °C, and transferred into a filter centrifuge for the second solid-liquid separation. During the centrifugal separation, hot water at 60 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

The solid residue was transferred into the reaction kettle and water was added. The system was stirred, heated, controlled at 68 °C, and transferred into a filter centrifuge for the third solid-liquid separation. During the separation, hot water at 68 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up. The mixture was dried to give the amino acid supramolecule.

### Example 3

Hot water controlled at about 60 °C was added to the crude N-lauroyl-L-alanine product obtained according to the method of Preparation Example 3. The system was stirred, heated, controlled at 60 °C, and transferred into a filter centrifuge (equipped with a filter screen) for the first solid-liquid separation. The liquid phase was removed by centrifugation, and hot water at 60 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

Water was added into the reaction kettle before the solid residue was transferred into the reaction kettle. The system was stirred, heated, controlled at 70 °C, and transferred into a filter centrifuge for the second solid-liquid separation. The liquid phase was removed by centrifugation, and hot water at 70 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up. The mixture was dried to give the amino acid supramolecule.

### Example 4

At room temperature, in a 1000-L reaction kettle, 89 kg (1 kmol) of L-alanine and 40 kg (1 kmol) of sodium hydroxide were dissolved in a mixed solution of 150 L of distilled water and 150 L of acetone, and the mixture was uniformly stirred to give a sodium L-alaninate solution.

At 20 °C, 175 kg (0.8 kmol) of lauroyl chloride was slowly added dropwise to the L-alanine salt solution, and a 50% potassium hydroxide solution was added dropwise to adjust the reaction system to pH 9. After the addition, the system was stirred at 20 °C for 1.5 h to give a pasty N-lauroyl-L-alanine salt.

Hydrochloric acid was added into the pasty N-lauroyl-L-alanine salt for acidification to pH 3-4. A white solid was gradually precipitated. The system was incubated for 3 h in an ice bath.

The system was then heated, controlled at 50 °C, stirred, and transferred into a filter centrifuge (equipped with a filter screen) for the first solid-liquid separation. The liquid phase was removed by centrifugation, and hot water at 50 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

Water was added into the reaction kettle before the solid residue was transferred into the reaction kettle. The system was stirred, heated, controlled at 60 °C, and transferred into a filter centrifuge for the second solid-liquid separation. The liquid phase was removed by centrifugation, and hot water at 60 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

Water was added into the reaction kettle before the solid residue was transferred into the reaction kettle. The system was stirred, heated, controlled at 65 °C, and transferred into a filter centrifuge for the third solid-liquid separation. The liquid phase was removed by centrifugation, and hot water at 65 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up.

Water was added into the reaction kettle before the solid residue was transferred into the reaction kettle. The system was stirred, heated, controlled at 65 °C, and transferred into a filter centrifuge for the fourth solid-liquid separation. During the separation, hot water at 65 °C was sprayed on the solid via a spraying device for treatment while the centrifuge was still operating, namely, the centrifugation and the treatment were conducted simultaneously. The total amount of the hot water was 0.5 tons, and the centrifugation was stopped after the hot water was used up. The mixture was dried to give the amino acid supramolecule.

### Example 5

Hot water at 50 °C was added to a proper amount of the crude product containing the dipeptide obtained in Example #503. The system was controlled at 50 °C and transferred into an industrial centrifuge for the first solid-liquid separation. The liquid phase was removed by centrifugation, and hot water at 50 °C was sprayed on the solid via a spraying device for treatment while the centrifuge was still operating, namely, the centrifugation and the treatment were conducted simultaneously.

The solid residue from the centrifuge was transferred into the reaction kettle and hot water at 60 °C was added. The system was stirred, controlled at 60 °C, and transferred into an industrial centrifuge for the second solid-liquid separation. The liquid phase was removed by centrifugation, and hot water at 60 °C was sprayed on the solid via a spraying device for treatment while the centrifuge was still operating.

The solid residue was transferred into the reaction kettle and hot water at 70 °C was added. The system was stirred, controlled at 70 °C, and transferred into an industrial centrifuge for the third solid-liquid separation. The liquid phase was removed by centrifugation, and hot water at 70 °C was sprayed on the solid via a spraying device for treatment while the centrifuge was still operating. The centrifugation was stopped after the hot water was used up, and the mixture was dried to give the amino acid supramolecule.

### Example 6

Hot water at 50 °C was added to a proper amount of the crude product containing the dipeptide obtained in Example #503. The system was controlled at 50 °C and transferred into an industrial centrifuge for the first solid-liquid separation.

The solid residue from the centrifuge was transferred into the reaction kettle and hot water at 60 °C was added. The system was stirred, controlled at 60 °C, and transferred into an industrial centrifuge for the second solid-liquid separation.

The solid residue was transferred into the reaction kettle and hot water at 70 °C was added. The system was stirred, controlled at 70 °C, and transferred into an industrial centrifuge for the third solid-liquid separation. The centrifugation was stopped after the hot water was used up, and the mixture was dried to give the amino acid supramolecule.

### Example 7

The crude product containing the dipeptide obtained in Example #502 was treated similarly to the method of Example 6, with the exception that the temperature for sample #502 was 46 °C in the first solid-liquid separation, 50 °C in the second solid-liquid separation, and 60 °C in the third solid-liquid separation.

### Example 8

The crude products containing the dipeptide obtained in Examples #501 and #601-604 were treated similarly to the method of Example 5 or 6.

### [Content assay of lauric acid]

The assay was conducted using method 2 (ultra-performance liquid chromatography) described above, and the results are shown in Table IV.

**Table IV. Percentage content by weight of lauric acid in samples**

| | Product of Example 5 | Product of Example 6 |
|---|---|---|
| After the first solid-liquid separation* | 4.6% | 8.2% |
| After the second solid-liquid separation* | 3.1% | 5.6% |
| After the third solid-liquid separation* | 2.5% | 4.8% |
| | Product of Example 7 | |
| Crude product of #502 before treatment* | 25.4% | |
| Crude product of #502 after three treatments* | 10.8% | |

All test samples were dried before the lauric acid content was determined.

As seen from the above results, the lauric acid content was less than 5% in the conventional samples after the three treatments (three solid-liquid separations). The lauric acid removal effect is superior if the separation is promoted with a solvent having a certain temperature as a medium (as in Example 5).

The crude product of #502 was treated at a lower temperature in the first solid-liquid separation due to higher impurity content, and the lauric acid content was still greater than 5% after the three solid-liquid separations. However, the lauric acid content was significantly reduced after the treatments, and additional solid-liquid separations and the use of a solvent having a certain temperature as a medium to promote the separation can be introduced to control the lauric acid content below 5%.

### [DSC analysis]

The results of the DSC analysis using the same method as described above for the product in Example #502 are shown in FIG. 11.

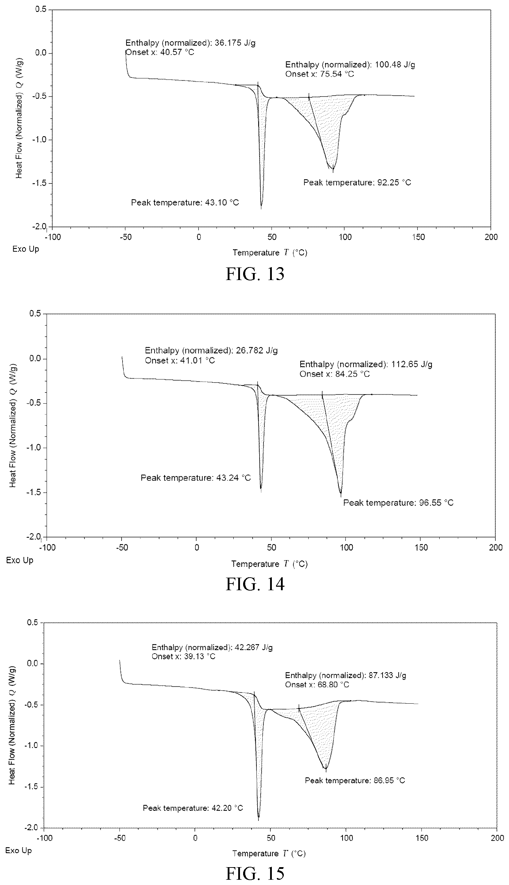

The results of the DSC analysis of the product in Example #501 after 1-3 hot water treatments (solid-liquid separations) are shown in FIGs. 12-14, respectively. The results of the DSC analysis of the product in Example #503 after 1-3 hot water treatments (following the treatment method of Example 6) are shown in FIGs. 15-17, respectively.

As seen from the results, for an untreated sample with high lauric acid impurity content (as in Example #502), the DSC demonstrated a peak temperature at about 78 °C. For a treated product (as in Examples #501 and #503), the DSC demonstrated a peak temperature at 86 °C or higher, and the peak gradually shifts to the right side with the increase of the number of treatments (number of solid-liquid separations), and the peak temperature becomes higher. In contrast, a higher dipeptide content leads to a higher peak temperature after the treatments.

### [Melting point analysis]

After the three solid-liquid separations, samples were to test the melting point by a capillary tube method. The results are shown in Table V.

**Table V.**

| Test sample | Determined melting point after three solid-liquid separations*¹ |
|---|---|
| 501# | 82-95°C |
| 503# | 80-94°C |
| 601# | 80-93°C |
| 604# | 80-87 °C |
| Control: a sample from WO2019233375A1 | 82-84°C*² |

| | |
|---|---|
| *¹: recorded in the table are temperatures from the start to the end of melting; *²: the melting point of the sample from WO2019233375A1 should be 82-84 °C, which was incorrectly described as 86-88 °C in a previous application CN108752228A (201810562220.1) (deviation due to thermometer misalignment) and was amended by the inventors in WO2019233375A1. | |

As seen from the test results, a higher dipeptide content leads to a higher melting point. The melting points (final melting temperatures) of the products with a high dipeptide content were above 87 °C.

### [Morphological analysis]

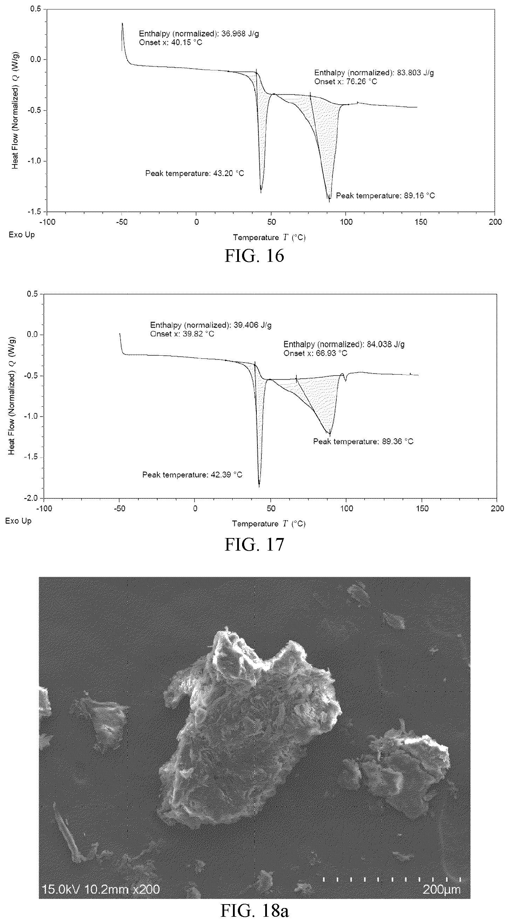

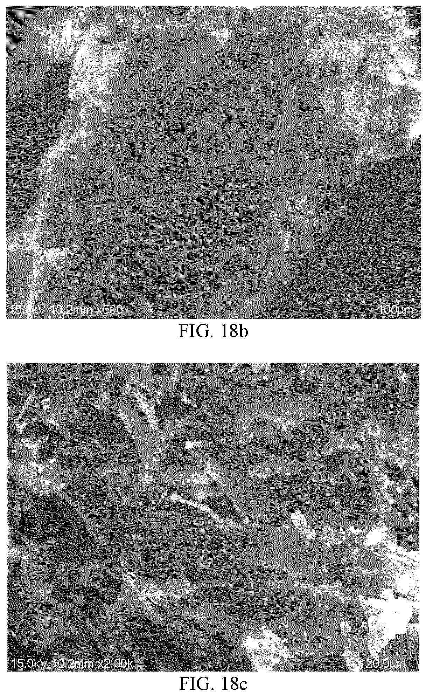

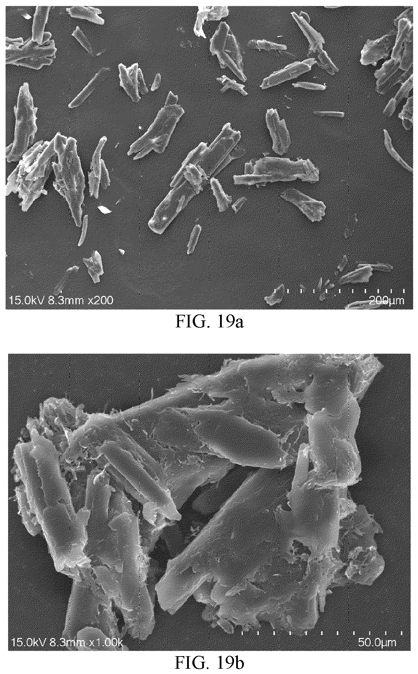

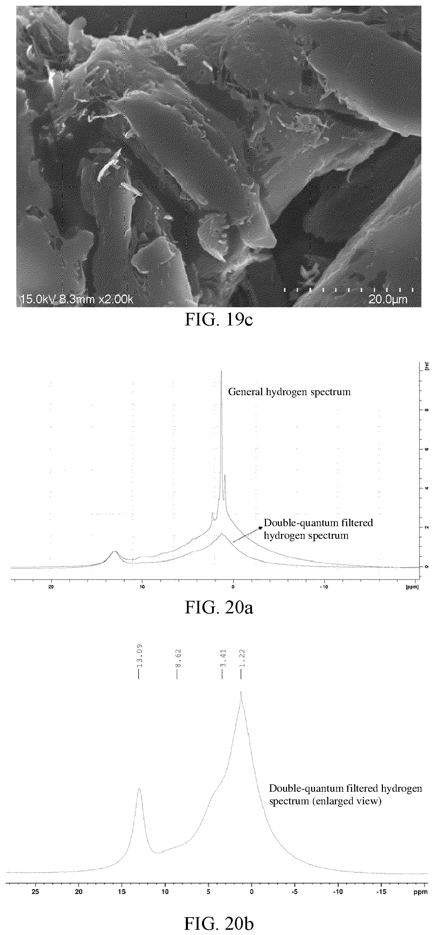

FIG. 18 illustrates the micromorphology of the crude product in Example #502, where the resulting product was formed by the aggregation of columnar, rod-shaped, thread-like or rope-shaped units when water was used as the solvent. FIG. 19 illustrates the micromorphology of the crude product in Example #501 after washing, which demonstrated rod-shaped basic units.

### [Solid nuclear magnetic resonance analysis]

System: Bruker AVANCE III HD WB 400 solid-state NMR spectrometer.

Methodology: carbon cross-polarization. The cross-polarization contact time was 1.5 ms, the sampling time was 25 ms, the relaxation time was 5 s, and the accumulation number was 1024. The sample was the product in Example 6.

In a double-quantum filtered (DQ-filtered) hydrogen spectrum experiment (FIGs. 20a and 20b), a carboxyl hydrogen signal was retained with high intensity, which indicates that the local motion of the corresponding group is limited and a hydrogen bond is formed; a broad peak at 8.6 ppm is presumed to be an amino peak.

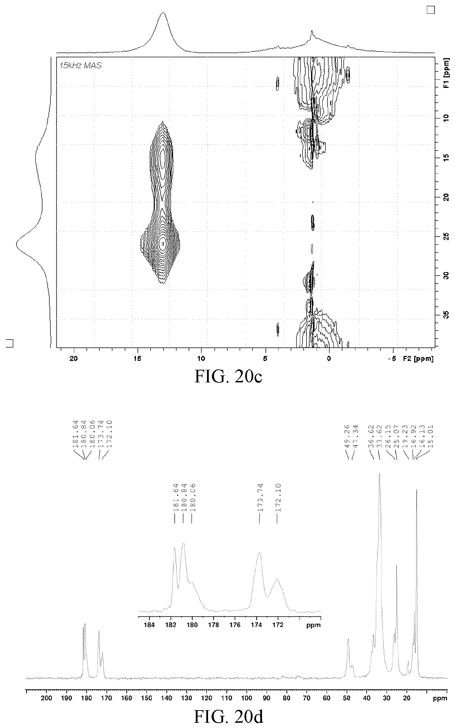

In the two-dimensional 2D ¹H-¹H DQ-SQ spectrum (FIG. 20c), the carboxyl hydrogen has a strong autocorrelation peak, which indicates that other carboxyl groups around interact with the carboxyl group, and the existence of hydrogen bonding between the carboxyl groups is inferred.

In the ¹³C CP spectrum (FIG. 20d), five C=O peaks were detected, corresponding to three carboxyl groups and two amide structures. From the peak pattern, it is presumed that the aggregation state of the structural molecule of the 1:1 reaction is more regular.

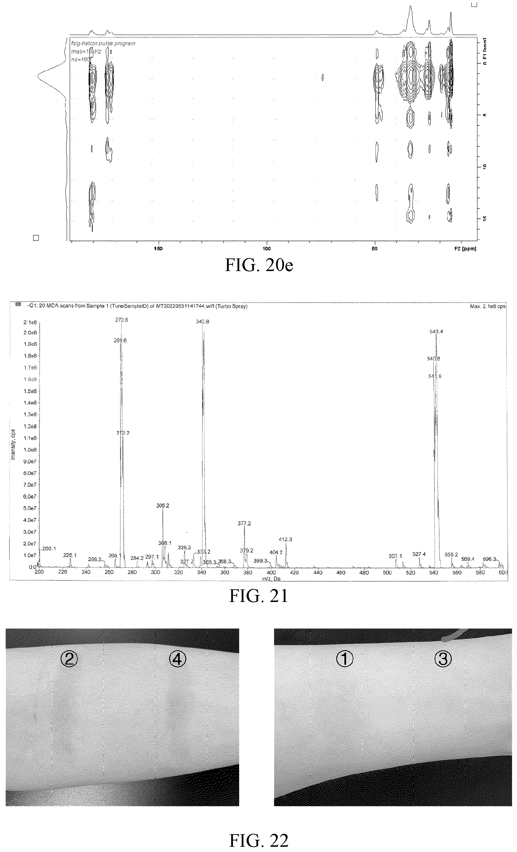

2D ¹³C-¹H FSLG-HETCOR spectrum (FIG. 20e) can help to identify the carbon spectra and confirm the relationship of amino groups to carboxyl groups.

### [Mass spectrometry]

System: AB4500
Scanning mode: Q1SCAN
Ionization mode: ESI(-); scanning range: m/z = 200-600
Sample: the product in Example 6.

As can be seen in FIG. 21, a characteristic ion peak is present at 543, indicating that two lauroyl alanine molecules are associated together (the molecular weight of lauroyl alanine is 271.4, and the characteristic ion peak at 543 presumably represents two associated lauroyl alanine molecules). The research team of the present invention proposes that if lauric acid is present, it may disturb the association of two lauroyl alanine molecules. When the lauric acid is removed or the content is reduced to a certain level, the carboxyl groups of the two lauroyl alanine are connected through hydrogen bonds to form alkane structures with a chain of eleven carbon atoms arranged at each of the two ends. According to the "like dissolves like" principle, the lipophilic ends are matched with the lipophilic ends in a chain manner and are connected to form a ring. The rings are infinitely superposed due to hydrogen bonding and the "like dissolves like" principle to form columnar molecular clusters. The columnar molecular clusters are infinitely superposed to form a special spatial structure, i.e., the amino acid supramolecule.

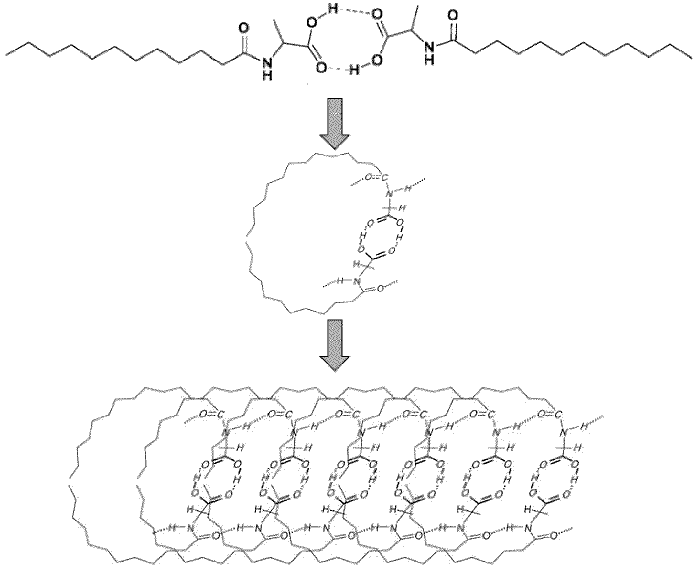

### [GPC analysis]

The samples were the sodium salt of the product in Example 6 and the sodium salt of the crude product in Example #502 before treatment.

### Instruments and conditions:

Pump: Waters 1515
Detector: Waters 2414
Column: PL aquagel-OH MIXED-H
Mobile phase: sodium acetate
Flow rate: 0.5 mL/min
Standard: PEG/PEO

After weighing, an aqueous sodium hydroxide solution was dropwise added, and the mixture was well stirred to give the sodium salt.

### Results:

The sodium salt of the product in Example 6 demonstrated a number-average molecular weight of 28,000.

The sodium salt of the crude product in Example #502 (before treatment) demonstrated no peaks, indicating the absence of macromolecules. This result was attributed to the fact that the crude product of #502 contained more than 25 wt% of lauric acid.

### Comparative Example 1

At room temperature, in a 1-L reaction kettle, 89 g (1 mol) of L-alanine and 40 g (1 mol) of sodium hydroxide were dissolved in a mixed solution of 150 mL of distilled water and 150 mL of acetone, and the mixture was uniformly stirred to give a sodium L-alaninate solution.

At 20 °C, 175 g (0.8 mol) of lauroyl chloride was slowly added dropwise to the L-alanine salt solution, and a 50% potassium hydroxide solution was added dropwise to adjust the reaction system to pH 9. After the addition, the system was stirred at 20 °C for 3 h to give a pasty N-lauroyl-L-alanine salt.

Hydrochloric acid was added into the pasty N-lauroyl-L-alanine salt for acidification to pH 1-2. The mixture was eluted with water and petroleum ether multiple times, filtered in vacuum and dried to give N-lauroyl-L-alanine as a white powdery solid.

### Comparative Example 2

Synthesis was performed with reference to: First report of phase selective gelation of oil from oil/water mixtures. Possible implications toward containing oil spills, Santanu Bhattacharya, Chem. Commun., 2001, 185-186.

Methyl lauroyl alaninate (as shown in the formula below) was hydrolyzed in methanol in the presence of 1 equivalent of 1 M NaOH at 5 °C for 2 h. The mixture was separated by centrifugation at a low temperature, and dried to give N-lauroyl-L-alanine.

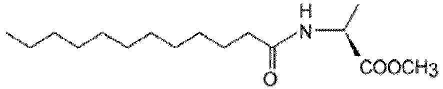

### Application of Amino Acid Supramolecule

Experiments such as microorganism inhibition, pesticide removal and deodorization and preparation of products such as toothpaste and laundry liquid can be found in prior Application No. WO2019/233375A1, with the exception that the long-chain acyl amino acid is replaced by the amino acid supramolecule of the present invention.

### Application Example 1 Evaluation of microorganism inhibitory effect of amino acid supramolecule

### a. Treatment of fruits

10 g of the N-lauroyl-L-alanine supramolecule synthesized by the method in Example 1 was added into water. A 10% aqueous sodium hydroxide solution was added to neutralize the mixture to pH 6-7. The volume of the aqueous solution was brought to 100 mL. Fruits pre-inoculated with common microorganisms such as *Staphylococcus aureus, Escherichia coli, Pseudomonas aeruginosa*, *Candida albicans*, and the like were soaked in 5 mL of the stock solution (the solution neutralized by sodium hydroxide) for a certain period of time, and washed with clean water. The microorganism residues on the fruits were determined. The results are shown in Table 1.

**Table 1. Analysis of microorganism inhibitory effect of N-lauroyl-L-alanine supramolecule**

| No. | Item | Unit | Criterion | Results | Evaluation |
|---|---|---|---|---|---|
| 1 | Inhibition of *E. coli* (8099), % (after 2 minutes) | -- | -- | 98.5 | -- |
| 2 | Inhibition of *E. coli* (8099), % (after 5 minutes) | -- | -- | 100 | -- |
| 3 | Inhibition of *S*. *aureus* (ATCC 6538), % (after 2 minutes) | -- | -- | 100 | -- |
| 4 | Inhibition of *S*. *aureus* (ATCC 6538), % (after 5 minutes) | -- | -- | 100 | -- |
| 5 | Inhibition of *C*. *albicans* (ATCC 10231), % (after 5 minutes) | -- | -- | 100 | -- |
| 6 | Viable cell count | CFU/mL | ≤1000 | <10 | Acceptable |
| 7 | *E. coli* count | MPN/100mL | ≤3 | <3 | Acceptable |

The data show that the N-lauroyl-L-alanine supramolecule solution synthesized by the method has a significant inhibitory effect on *E. coli, S. aureus,* and *C*. *albicans.* For *E. coli,* the inhibitory rate was 98.5% after 2 min and 100% after 5 min of treatment; for *S*. *aureus,* the inhibitory rate was 100% after 2 min of treatment; for *C*. *albicans,* the inhibitory rate was 100% after 5 min of treatment.

### b. Microorganism inhibition test

The N-lauroyl-L-alanine supramolecule synthesized according to the method of Example 1, was neutralized with sodium hydroxide or arginine to prepare 10% aqueous solutions (10% LA, formulated with sterilized deionized water). After standing for one month, no microorganisms were detected in the materials neutralized with sodium hydroxide or arginine, indicating that the 10% LA does not breed microorganisms and thus has certain microorganism inhibitory activity.

### Application Example 2 Evaluation of pesticide removal effect of amino acid supramolecule

Two 100-g aliquots of bok choy pre-sprayed with pesticides methamidophos and acephate were taken. One was directly soaked in 1 L of clean water and detected for the pesticide residues, denoted as "before washing". The other aliquot was washed with a solution formulated with the N-lauroyl-L-alanine supramolecule synthesized according to the method in Example 1, denoted as "after washing". The procedures are as follows:
10 g of the N-lauroyl-L-alanine supramolecule synthesized by the method in Example 1 was added into water. A 10% aqueous sodium hydroxide solution was added to neutralize the mixture to pH 6-7. The volume of the aqueous solution was brought to 100 mL. The second 100-g aliquot of bok choy pre-sprayed with pesticides methamidophos and acephate was shredded, soaked in 5 mL of the stock solution for 2 min, washed with 500 mL of clean water, and detected for the pesticide residues. Table 2 shows the comparison of pesticide residues before and after washing.

**Table 2. Analysis of pesticide removal effect of N-lauroyl-L-alanine supramolecule**

| No. | Item | Before washing, mg/kg | After cleaning, mg/kg | Percentage removal, % |
|---|---|---|---|---|
| 1 | Methamidophos | 16.42 | 3.91 | 76.19 |
| 2 | Acephate | 37.57 | 5.12 | 86.37 |

According to the data, the N-lauroyl-L-alanine supramolecule solution used in the present invention has a significant removal effect on methamidophos and acephate. After 2 min of treatment, the removal rate of methamidophos was 76.19%, and the removal rate of the acephate was 86.37%.

### Application Example 3 Evaluation of deodorization effect of amino acid supramolecule

### a. Deodorization experiment

10 g of the N-lauroyl-L-alanine supramolecule synthesized by the method in Example 1 was added into water. A 10% aqueous sodium hydroxide solution was added to neutralize the mixture to pH 6-7. The volume of the aqueous solution was brought to 100 mL. Cotton cloths of 10 square centimeters with peculiar smell (smell, engine oil smell, odor, etc.) were soaked in 5 mL of stock solution for 2 min, taken out, washed with water, and dried by airing. It was found that the smells on the cotton cloths were completely eliminated, suggesting that the N-lauroyl-L-alanine supramolecule synthesized by the method has a good deodorization effect.

### b. Deodorization experiment

10 g of the N-lauroyl-L-alanine supramolecule synthesized by the method in Example 1 was added into water. A 10% aqueous sodium hydroxide solution was added to neutralize the mixture to pH 6-7. The volume of the aqueous solution was brought to 100 mL (stock solution). The deodorization evaluation was performed by 5 designated subjects. The specific procedures are as follows: A small amount of cooked crab cream on the arms of the subjects. The arms of 3 subjects were washed with the stock solution (twice), and the arms of the other 2 were washed with clean water (twice). The residual smell on the arms was independently evaluated. Thick crab cream smell was scored 1; light smell was scored 2-4; basically no smell was scored 5.

**Table 3. Analysis of crab cream smell removal effect of N-lauroyl-L-alanine supramolecule**

| Wash solution | Stock solution | Stock solution | Stock solution | Clean water | Clean water |
|---|---|---|---|---|---|
| Wash effect | 5 | 5 | 4 | 1 | 1 |

### Application Example 4 Application of amino acid supramolecule in skin care

**Table 4. Materials and percentage contents by weight of skin care compositions**

| Material | Formula 1 | Formula 2 | Formula 3 | Formula 4 | Formula 5 | Formula 6 |
|---|---|---|---|---|---|---|
| Natural oil blend* | 57 | 80 | 64 | 64 | 65 | 51 |
| Corn starch | 40 | 15 | 25 | 25 | 0 | 25 |
| KTZ^{®} Classical White | 0 | 0 | 0.5 | 0 | 0 | 0 |
| Titanium dioxide | 0 | 0 | 0.2 | 0 | 0 | 0 |
| Micronized titanium dioxide | 0 | 0 | 0 | 6 | 0 | 0 |
| Boron nitride (CaressnBN02 ex. Kobo) | 0 | 0 | 0 | 0 | 5 | 0 |
| Glycerol | 0 | 0 | 0 | 0 | 25 | 10 |
| Glycerol monolaurate | 0 | 0 | 0 | 0 | 0 | 4 |
| Mss-500/20 | 0 | 0 | 0 | 0 | 0 | 0 |
| N-lauroyl-L-alanine supramolecule | 3 | 5 | 10 | 5 | 5 | 10 |
| (Example 1) | | | | | | |

The natural oil blend contains 40% of grape seed oil, 37.2% of sunflower seed oil, and 22.8% of aloe oil.

The experiment was repeated according to the formula of the prior Application No. WO2019/233375A1, with the major exception that the long-chain acyl amino acid was replaced by the amino acid supramolecule of the present invention. The specific preparation procedures of formula 1 shown in Table 4 include: 57% of the natural oil blend and 40% of corn starch were added to the mixer and homogenized to disperse the particles. The particles in the oil dispersion were then heated to 83-86 °C. 3% of the N-lauroyl-L-alanine supramolecule was added to the mixer during heating. The sample was heated at 73-86 °C, held for 5-10 min, and cooled to 65-72 °C while remaining miscible. The sample was then poured into a 30-mL tank to give the skin care composition, which was preserved for evaluation. The skin care compositions of formulas 2-6 were prepared similarly to formula 1, and the details are not repeated.

The skin care compositions were obtained according to the above examples, wherein particulate matters of different types and contents were added. The results show that other than increasing the viscosity of the oil, due to the addition of particles, the N-lauroyl-L-alanine supramolecule can also stably suspend solid organic/inorganic particles or oil mixture liquids such as glycerol in thickened natural oils for additional skin benefits.

Four oil-insoluble particles, i.e., starch, TiO₂, mica, and boron nitride particles (Caress BN02 from Kobo) and an oil-miscible liquid such as glycerol were used. The natural oil blend used in formulas 1-6 was identical in composition, i.e., containing 40% of grape seed oil, 37.2% of sunflower seed oil, and 22.8% of aloe oil. The results show that compositions obtained from formulas 1-6 shown in Table 4 were stable at room temperature and in the 48 °C oven without any particle separation problems.

### Application Example 5 Application of amino acid supramolecule in toothpaste

(1) The experiment was repeated according to the formulation of the prior Application No. WO2019/233375A1, with the major exception that the long-chain acyl amino acid was replaced by the amino acid supramolecule of the present invention (formulas 1-5).
(2) The amino acid supramolecule of the present invention was neutralized by arginine for the toothpaste preparation experiment (formula 6).

The components of the toothpaste and specific contents are shown in Table 5. The sodium salt of the N-lauroyl-L-alanine supramolecule was prepared by reacting the N-lauroyl-L-alanine supramolecule of Example 1 with sodium hydroxide.

**Table 5. Components and their contents in toothpaste**

| | Component (parts by weight) | | | | | |
|---|---|---|---|---|---|---|
| Material | Formula 1 | Formula 2 | Formula 3 | Formula 4 | Formula 5 | Formula 6 |
| Sodium salt of N-lauroyl-L-alanine supramolecule | 4.4 | 3 | 2 | 5 | 13.75 | |
| N-lauroyl-L-alanine supramolecule (Example 1) | | | | | | 3 |
| Arginine | | | | | | 2 |
| Carboxymethyl cellulose | 4 | 6 | 4 | 6 | 3 | 5 |
| Hydrated silica | 35 | 35 | 33.4 | 35 | 33 | 35 |
| Water | 10 | 10 | 10 | 10 | 8 | 10 |
| Sorbitol | 37.5 | 35.5 | 35.5 | 33.5 | 32 | 35 |
| Glycerol | 5 | 6 | 6 | 6 | 5 | 6 |
| Polyethylene glycol-400 | 2 | 2 | 2 | 2 | 3 | 2 |
| Edible essence | 1 | 1.5 | 1 | 1.5 | 1 | 1 |
| *Chondrus crispus* extract | 0.2 | 0.1 | 0.2 | 0.1 | 0.2 | 0.2 |
| Sodium saccharin | 0.2 | 0.2 | 0.2 | 0.2 | 0.2 | |
| Trichlorogalactose | | | | | | 0.2 |
| Sodium benzoate | 0.4 | 0.4 | 0.4 | 0.4 | 0.4 | 0.4 |
| *Glycyrrhiza uralensis* extract | 0.1 | 0.1 | 0.1 | 0.1 | 0.15 | |
| *Portulaca oleracea* extract | 0.1 | 0.1 | 0.1 | 0.1 | 0.15 | |
| CI42090 | 0.1 | 0.1 | 0.1 | 0.1 | 0.15 | 0.1 |
| CI19140 | | | | | | 0.1 |

The toothpaste was prepared according to formula 1, and the specific procedures include: 10 g of water, 37.5 g of sorbitol, 0.2 g of sodium saccharin, 2 g of polyethylene glycol-400, 5 g of glycerol, and 0.4 g of sodium benzoate were formulated into an aqueous solution and added to a paste-making machine. Then 4 g of carboxymethyl cellulose, 35 g of hydrated silica, 0.2 g of a *Chondrus crispus* extract, 0.1 g of a *Glycyrrhiza uralensis* extract, and 0.1 g of a *Portulaca oleracea* extract were mixed and added into a paste-making machine, stirred and milled for 20-30 min until the paste was uniform, and the mixture was defoamed in vacuum. 4.4 g of the sodium salt of the N-lauroyl-L-alanine supramolecule, 1 g of an edible essence (mint flavor), and 0.1 g of CI42090 were sequentially added into the paste-making machine, stirred and milled for 10-15 min until paste is uniform, and the mixture was defoamed to give the amino acid-based toothpaste. Formulas 2-5 were prepared similarly.

The toothpaste was prepared according to formula 6, and the specific procedures include: 10 g of water, 35 g of sorbitol, 0.2 g of trichlorogalactose, 2 g of polyethylene glycol-400, 6 g of glycerol, and 0.4 g of sodium benzoate were formulated into an aqueous solution and added to a paste-making machine. Then 5 g of carboxymethyl cellulose, 35 g of hydrated silica, and 0.2 g of a *Chondrus crispus* extract were mixed and added into a paste-making machine, stirred and milled for 20-30 min until the paste was uniform, and the mixture was defoamed in vacuum. 3 g of the N-lauroyl-L-alanine supramolecule, 2 g of arginine, 1 g of an edible essence, 0.1 g of CI42090, and 0.1 g of CI19140 were sequentially added into the paste-making machine, stirred and milled for 10-15 min until paste is uniform, and the mixture was defoamed to give the amino acid-based toothpaste.

The amino acid toothpaste of the present invention demonstrated good safety with all measurements meeting the criteria. The toothpaste prepared by neutralizing the N-lauroyl-L-alanine supramolecule with arginine is milder and safer, with excellent mouthfeel.

### Application Example 6 Detergency test of amino acid supramolecule

### a. Detergency evaluation

Three sets of color cosmetics (lipstick + eyeliner) were applied to the arms of 5 subjects with similar areas and thicknesses. The cosmetics were washed with the aqueous solutions in Table 6 (1 g of each) and then rinsed with tap water (the detergency was graded by the 5 subjects comprehensively).

**Table 6. Detergency analysis**

| | N-lauroyl-L-alanine supramolecule (Example 1) | N-lauroyl-L-alanine (Comparative Example 1) | N-lauroyl-L-alanine (Comparative Example 2) |
|---|---|---|---|
| Water | To 100 | To 100 | To 100 |
| Various N-lauroyl-L-alanine | 4 | 4 | 4 |
| Sodium hydroxide | 0.54 | 0.54 | 0.54 |
| pH value | 7.25 | 8.07 | 6.56 |

| Appearance | Transparent | Transparent | Transparent |
|---|---|---|---|
| Detergency* | 5 | 3 | 1 |

| | | | |
|---|---|---|---|
| * 5 denotes the best, 1 denotes the worst, and 3 denotes moderate. | | | |

### b. Application of amino acid supramolecule in laundry liquid

**Table 7. Amino acid supramolecule laundry liquid**

| | Percentage by weight | | | |
|---|---|---|---|---|
| Component | Formula 1 | Formula 2 | Formula 3 | Formula 4 |
| Water | 78 | 78 | 78 | 78 |
| N-lauroyl-L-alanine supramolecule (Example 1) | 10 | 0 | 0 | 0 |
| Amino acid supramolecule (Example 6) | 0 | 0 | 0 | 10 |
| Sodium hydroxide | 1.4 | 1.4 | 1.4 | 1.4 |
| N-lauroyl-L-alanine (Comparative Example 1) | 0 | 10 | 0 | 0 |
| N-lauroyl-L-alanine (Comparative Example 2) | 0 | 0 | 10 | 0 |
| Decyl glucoside | 5 | 5 | 5 | 5 |
| Cocamidopropyl betaine | 5 | 5 | 5 | 5 |
| GPL | 0.5 | 0.5 | 0.5 | 0.5 |
| DMDM | 0.1 | 0.1 | 0.1 | 0.1 |
| Essence | 0.05 | 0.05 | 0.05 | 0.05 |

According to the test, for the laundry liquids prepared according to formulas 1 and 4, the results of JB01, JB02, and JB03 were acceptable (the detergency of the sample to the stained cloths JB01, JB02, and JB03 polluted were greater than those of the standard laundry liquid to the stained cloths JB01, JB02, and JB03); for the laundry liquid prepared according to formula 2, the result of JB01 was acceptable, while the results of JB02 and JB03 were unacceptable; for the laundry liquid prepared according to formula 3, the results of JB01, JB02, and JB03 were unacceptable.

### Application Example 7 Neutralization assay of amino acid supramolecule

### a. Test for product of Example 1

Test process (① denotes the amino acid supramolecule of Example 1 neutralized by arginine; ② denotes the amino acid supramolecule of Example 1 neutralized by sodium hydroxide; ③ denotes the amino acid supramolecule of Example 2 neutralized by arginine; ④ denotes the amino acid supramolecule of Example 2 neutralized by sodium hydroxide)

The preparation procedures are as follows: Two 500-g beakers were weighed on an electronic balance, and the weights of the beakers were recorded; a proper amount of deionized water was weighed and added to the beakers according to the formula, and the beakers were heated to 75-80 °C in a water bath. The temperature of the water in the beaker was measured by a thermometer. The amino acid supramolecule of Example 1 was weighed and added after the temperature was confirmed to be 75-80 °C. At the start of the stirring, a proper amount of arginine was added to beaker ① and a proper amount of 10% aqueous sodium hydroxide solution was added to beaker ②. The mixtures were stirred for 1 min. The temperature of the beakers ① and ② was reduced to 25 °C by running tap water. The evaporated water in the two beakers in the preparation process was compensated by cooled deionized water to 100% on an electronic balance. The pH of the products in the beaker ① and ② was measured, the appearance was observed, and the smell was confirmed.

The preparations of ③ and ④ were conducted according to the method described above, with the exception that the amino acid supramolecule of Example 1 was replaced with the amino acid supramolecule of Example 2.

### Detergency test

1) Four sets of color cosmetics were applied to the arms of a subject with similar areas and thicknesses, including 4 strokes of lipstick cosmetics and 4 strokes of eyeliner cosmetics;
2) The cosmetics were then washed with the aqueous solutions of ①, ②, ③, and ④ (1 g of each);
3) The cosmetics were rinsed with tap water. The results are shown in FIG. 22.

All test results are recorded in Table 8.

**Table 8. Results of amino acid supramolecule neutralization assay**

| | ① Neutralization with arginine | | ② Neutralization with sodium hydroxide | |
|---|---|---|---|---|
| | W% | *2 (trial, 200 g) | W% | *2 (trial, 200 g) |
| Deionized water | 93.6 | 187.2 | 90.6 | 181.2 |
| Amino acid supramolecule | 4 | 8 | 4 | 8 |
| Arginine | 2.4 | 4.8 | 0 | 0 |
| Sodium hydroxide (10% aqueous solution) | 0 | 0 | 5.4 | 10.8 |
| In total | 100% | 200g | 100% | 200g |
| Evaluation | | | | |
| pH | 7.59 | | 7.25 | |
| Appearance | Clear and transparent | | Clear and transparent | |
| Smell | Characteristic smell of amino acid supramolecule | | Characteristic smell of amino acid supramolecule | |
| Detergency performance | ① is superior to ②. Under the same conditions, ① can completely remove all the cosmetics, while ② demonstrated residual color cosmetics. | | | |
| Feeling of use | ① is superior to ②: In the same container, ① produces more foam with higher density, better durability, softer feel, and elasticity. The foam of ② exhibited insufficient density, moderate durability, and no elasticity. | | | |

### b. Comparison test of products of Example 1 (LA-I) and Example 6 (LA-II)

### Sample preparation:

| No. | Name | pH |
|---|---|---|
| ① | 5%LA-II+NaOH | 6.85 |
| ② | 5% LA-II + arginine | 6.99 |
| ③ | 5% LA-II + lysine | 6.64 |
| ④ | 5%LA-I+NaOH | 8.18 |
| ⑤ | 5% LA-I + arginine | 6.64 |
| ⑥ | 5% LA-I + lysine | 6.14 |

| | | |
|---|---|---|
| Note: The neutralizations in ①-⑥ were conducted in equimolar ratios. For example, 5% LA-II + NaOH refers to that LA-II was neutralized by equimolar NaOH, and 5% LA-II refers to that the percentage content by weight of LA-II was 5%. Lysine neutralization: Lysine hydrochloride was first neutralized with NaOH, and this aqueous solution was used to neutralize LA. | | |

### Foaming test

The foaming test was conducted with reference to the procedures in 6.2.6 of [GB/T 29679-2013 Hair shampoo, cream shampoo], with the exception that 5 g of the sample (the neutralized 5% LA solution) was diluted with 445 g of distilled water before 50 g of hard water was added. The percentage concentration by weight of the amino acid supramolecule in the final test solution was 0.05%. The results are shown in Table 9.

**Table 9. Amino acid supramolecule foaming test results**

| Name | 0min | 5min | Notes |
|---|---|---|---|
| 5%LA-II+NaOH | 80-90 | 65-75 | Significant collapse was observed in the foam in general |
| 5% LA-II + arginine | 85-95 | 75-80 | Defoaming was observed in the upper portion, while the rest of the foam was soft and dense |
| 5% LA-II + lysine | 95-105 | 85-90 | Defoaming was observed in the upper portion, while the rest of the foam was soft and dense |
| 5%LA-I+NaOH | 40-45 | 30-40 | Significant defoaming was observed |
| 5% LA-I + arginine | 60-70 | 50-60 | Significant defoaming was observed in the upper and middle portions, and the lower portion was soft and dense |
| 5% LA-I + lysine | 90-100 | 80-85 | Significant defoaming was observed in the upper and middle portions, and the lower portion was soft and dense |

From the above results, the neutralized aqueous solution of LA-II foams more easily and produces relatively stable and denser foam than LA-I. For the foaming property, the "lysine-neutralized" product foams better than the "arginine-neutralized" product, and the two are superior to the "NaOH-neutralized" product. The "NaOH-neutralized" product defoams faster than the "arginine-neutralized" product, and the "lysine-neutralized" product foams most stably.

### Application Example 8 Application of salts of amino acid supramolecule and basic amino acid in facial cleanser

**Table 10. Amino acid supramolecule-based facial cleanser**

| Component | Percentage of components in the formula |
|---|---|
| Water | 82 |
| N-lauroyl-L-alanine supramolecule (Example 1) | 4 |
| Arginine | 0.7 |
| Cocamidopropyl betaine | 7.8 |
| Glycerol | 4.5 |
| Tetrasodium N,N-bis(carboxylatomethyl)-glutamate | 0.3 |
| GPL | 0.5 |
| DMDM | 0.1 |
| Essence | 0.05 |

The experiment was repeated according to the formula of the prior Application No. WO2019/233375A1, with the major exception that the long-chain acyl amino acid was replaced by the amino acid supramolecule of the present invention. The facial cleanser prepared according to the formula shown in Table 10 is mild, non-irritant, and more suitable for sensitive skin, since the salts of the N-lauroyl-L-alanine supramolecule and arginine, as the surfactants for cleansing, are non-irritant to human skin. This desirable property finally results in excellent performance of the facial cleanser.

### Application Example 9 Application of amino acid supramolecule in facial cleansing foam product

**Table 11a. Formula containing amino acid supramolecule of Example 1 (LA-I formula)**

| NO. | Name | Compounding amount | Purpose of use |
|---|---|---|---|
| 1 | Deionized water | TO 100 | Solvent |
| 2 | TEA cocoylglutamate | 3.5 | Surfactant |
| 3 | Glycerol | 10 | Humectant |
| 4 | Butanediol | 8 | Humectant |
| 5 | Lauryl hydroxysultaine | 2 | Surfactant |
| 6 | Amino acid supramolecule LA-I (Example 1) | 4 | Detergent |
| 7 | Cocamide DEA | 3 | Thickening agent |
| 8 | Decyl glucoside | 1.5 | Detergent |
| 9 | Arginine | 2.47 | Skin conditioner |
| 10 | Phenoxyethanol | 0.5 | Preservative |
| 11 | Disodium EDTA | 0.1 | Chelating agent |

**Table 11b. Formula containing amino acid supramolecule of Example 6 (LA-II formula)**

| NO. | Name | Compounding amount | Purpose of use |
|---|---|---|---|
| 1 | Deionized water | TO 100 | Solvent |
| 3 | Glycerol | 10 | Humectant |
| 4 | Butanediol | 8 | Humectant |
| 6 | Amino acid supramolecule LA-II (Example 6) | 4 | Detergent |
| 9 | Arginine | 2.47 | Skin conditioner |
| 10 | Phenoxyethanol | 0.5 | Preservative |
| 11 | Disodium EDTA | 0.1 | Chelating agent |

The LA-II formula can achieve the same effect as the LA-I formula without the need for surfactants or detergents other than LA due to the excellent detergency and foaming property.

### Application Example 10 Effect evaluation of amino acid supramolecule-based body wash foam product

### Evaluation: Water content in the stratum corneum

The inner side of the arm was washed with the body wash foam product. Before use of the product, the water content in the uncoated stratum corneum was measured. After that, the sample product was used, and the water content in the skin was measured before washing and 5 min, 15 min, 30 min and 60 min after washing by the following method. The change in water content before and after washing was used as the index for evaluating the moisturizing property. Before the test in the uncoated state, 5 subjects were acclimatized for 20 min or more at room temperature (22 °C)/RH 50%.

Use of sample product: The foaming sample was applied to acclimate to the inner side of the arm for 1 min, and then rinsed 10 times with water.

Instrument: Corneometer CM 825 (Courage + Khazaka, Germany).

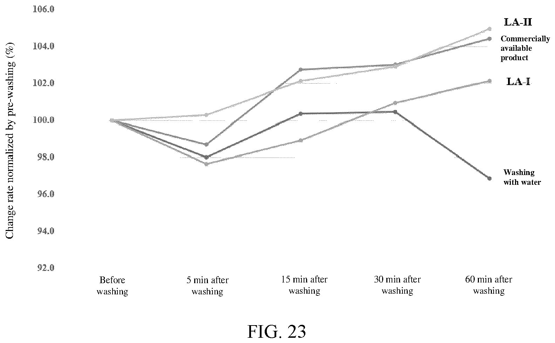

The formulas are shown in Table 12 and the results are shown in FIG. 23 (means of 5 subjects).

**Table 12.**

| Materials | Formula of commercially available products* | Formula of LA-I* | Formula of LA-II* |
|---|---|---|---|
| Deionized water | 59.85 | 86.8 | 87.1 |
| TEA cocoylglutamate | 10.5 | | |
| Amino acid supramolecule LA-I (Example 1) | | 8 | |
| Amino acid supramolecule LA-II (Example 6) | | | 8 |
| Arginine | | 5.2 | 4.9 |
| Butanediol | 8 | | |
| Cocamide DEA | 5 | | |
| Diglycerol | 5 | | |
| Glycerol | 5 | | |
| Lauryl hydroxysultaine | 3 | | |
| Sorbitol | 3 | | |
| Phenoxyethanol | 0.3 | | |
| Decyl glucoside | 0.25 | | |
| Skin conditioner | 0.1 | | |

| | | | |
|---|---|---|---|
| *The addition amounts of the materials are percentage content by weight. | | | |

The commercially available product comprises TEA cocoylglutamate as the amino acid-based surfactant, and further comprises various humectants such as butanediol, glycerol, diglycerol, sorbitol, and the like. The LA-I formula and the LA-II formula of the present invention are aqueous solutions neutralized by arginine with no additional humectant, but demonstrated excellent moisturizing performance. The LA-II formula showed even superior performance to the commercially available product with additional humectants, and the performance advantage of the LA-II formula became more significant over time.

In addition, the same amounts of butanediol, glycerol, diglycerol and sorbitol were added into the LA-I formula as in the formula of the commercially available product, and significantly improved moisturizing performance was observed, which indicates that the product provided by the present invention has excellent moisturizing performance, and the performance can be further improved if additional humectants are included.

### Application Example 11 Qualitative application test

Due to the special structure, the amino acid supramolecule can be combined with grease to give a non-sticky "solid"/paste which is easy to remove and has good cleaning capacity within a pH range of 5-14 and a broad application range.

The sodium salts of the amino acid supramolecule (LA-I) of Example 1 and the amino acid supramolecule (LA-II) of Example 6 were formulated into 10% and 12% aqueous solutions for household use: the surface of electric rice cookers, the surface of kitchen hoods, oil collectors in kitchen hoods, kitchen benches, dish washing, clothes washing, deodorization, closestools, and the like.

The 12% LA aqueous solution produced more foam with higher density and better detergency than the 10% LA solution.

For the surface of the electric rice cookers, especially the sticky bottoms, the greasy stains on the surface were wiped several times by using rags or paper towels soaked in the solutions and then wiped by using a clean cloth. After the wipe, the surface was clean with no stickiness, no odor and no foam residue.

The common dish washing detergent exhibited poor effects on the surfaces of cooking benches and kitchen hoods, while the 12% aqueous LA-I or LA-II solution demonstrated excellent detergency and no irritation to hands, with no need for additional rinsing. For the drawers washed with the aqueous LA-I or LA-II solution, the mobility of the oil was enhanced (the oil was easier to pour), and the drawer could be cleaned more easily.

When the 10% LA solution was used for washing dishes, the solution produced a greater amount of foam, with no stickiness in hands and no residual detergent. The dishes were easy to wash, demonstrating water-saving and environment-friendly effects.

The use of the 10% LA solution for cleaning stains and dirt on the inner wall of a closestool provided a good user experience. By adding a proper amount of LA solution and several brushes, the odor could be removed.

Moreover, those skilled in the art will appreciate that although some embodiments described herein include some features included in other embodiments, the combinations of features in different embodiments shall fall within the scope of the present invention and form different embodiments. For example, in the Claims, any of the claimed embodiments may be used in arbitrary combinations.

## Claims

1. A method for preparing an N-long-chain acyl amino acid dipeptide and/or a salt thereof or a composition comprising the N-long-chain acyl amino acid dipeptide and/or the salt thereof, wherein comprising: reacting an amino acid and/or a salt thereof with a long-chain acid halide, wherein the pH value of the system after the reaction is less than 8, preferably 7.5 or less, more preferably 7 or less, and most preferably 5-6.5.

2. A method for preparing an N-long-chain acyl amino acid dipeptide and/or a salt thereof or a composition comprising the N-long-chain acyl amino acid dipeptide and/or the salt thereof, wherein comprising: reacting an amino acid and/or a salt thereof with a long-chain acid halide in the presence of a base, wherein throughout the reaction, the molar ratio of the amino acid to the base is 3:1 to 1:2, preferably 2:1 to 1:1.8, more preferably 1.7:1 to 1:1.7, and most preferably 1.5:1 to 1:1.5.

3. The method according to claim 1 or 2, wherein the long-chain acid halide is added to the amino acid and/or the salt thereof without controlling the reaction solution at an alkaline pH, preferably without controlling the reaction solution at pH 8 or greater;
alternatively, the long-chain acid halide is added without simultaneously adding the base or controlling the dripping speed and amount of the base to maintain the pH value of the system; or
alternatively, the difference between the pH values of the system before and after the addition of the long-chain acid halide is 2 or higher, preferably 3 or higher, and more preferably 4 or higher.

4. The method according to any one of claims 1-3, wherein after the reaction of the amino acid and/or salt thereof with the long-chain acid halide, the percentage content by weight of the N-long-chain acyl amino acid dipeptide and/or the salt thereof in the product is 3% or greater, preferably 5% or greater, more preferably 8% or greater, and most preferably 10% or greater.

5. The method according to any one of claims 1-4, wherein comprising: (1) reacting a raw material comprising the amino acid and the base to give an amino acid salt solution; and (2) adding the long-chain acid halide into the resultant amino acid salt solution or adding the long-chain acid halide and the base into the resultant amino acid salt solution, wherein the method meets one or more of the following conditions:
a, the pH value of the amino acid salt solution prepared in step (1) is 7.5-14, preferably 8-12, and more preferably 9-11, and the pH value of the system after the reaction in step (2) is less than 8, preferably 7.5 or less, more preferably 7 or less, and most preferably 5-6.5;
b, the molar ratio of the amino acid to the base in the reaction system throughout step (1) and step (2) is 3:1 to 1:2, preferably 2:1 to 1:1.8, more preferably 1.7:1 to 1:1.7, and most preferably 1.5:1 to 1:1.5;
c, the pH value of the amino acid salt solution prepared in step (1) is greater than that of the system prepared after the reaction of the amino acid salt and the long-chain acid halide in step (2), and the difference between the two is 2 or greater, preferably 3 or greater, and more preferably 4 or greater.

6. The method according to any one of claims 1-5, wherein the reaction of the amino acid and/or the salt thereof with the long-chain acid halide meets one or more of the following conditions:
a, the reaction is conducted in the presence of water or a mixed solution of water and a hydrophilic organic solvent; the hydrophilic organic solvent is selected from one or more of acetone, methanol, ethanol, isopropanol, sec-butyl alcohol, tert-butyl alcohol, acetonitrile, and tetrahydrofuran, and preferably acetone; preferably, the volume ratio of the water to the hydrophilic organic solvent is 1:(0-2);
b, the temperature of the reaction is 35 °C or lower, and preferably 30 °C or lower;
c, the molar ratio of the amino acid and/or the salt thereof to the long-chain acid halide is greater than 1, preferably 2:1 to 1.1:1, and more preferably 1.5:1 to 1.2:1.

7. The method according to any one of claims 1-6, wherein further comprising: acidifying a product prepared by the reaction of the amino acid and/or the salt thereof with the long-chain acid halide to give a crude N-long-chain acyl amino acid product; preferably, the pH value after the acidification is 1 to 4, and more preferably 1 to 2.

8. The method according to any one of claims 1-7, wherein the method meets one or more of the following conditions:
a, the amino acid is selected from one or more of glycine, alanine, glutamic acid, sarcosine, aspartic acid, leucine, isoleucine, valine, threonine, proline, phenylalanine, arginine, and lysine;
b, the long-chain acyl in the long-chain acid halide is derived from a saturated or unsaturated linear or branched fatty acid with 8-22 carbon atoms;
c, the base is selected from one or more of sodium hydroxide, potassium hydroxide, sodium carbonate, potassium carbonate, and ammonia.

9. The method according to claim 8, wherein the method meets one or more of the following conditions:
a, the amino acid is selected from alanine, glycine, glutamic acid, sarcosine, arginine, or lysine, and preferably L-alanine;
b, the long-chain acid halide is selected from one or more of octanoyl chloride, caprinoyl chloride, undecanoyl chloride, lauroyl chloride, myristoyl chloride, pentadecanoyl chloride, palmitoyl chloride, stearoyl chloride, oleoyl chloride, linoleoyl chloride, isostearoyl chloride, coconut oil fatty acid chloride, or palm oil fatty acid chloride, preferably coconut oil fatty acid chloride or lauroyl chloride, and most preferably lauroyl chloride;
c, the base is selected from sodium hydroxide or potassium hydroxide.

10. A method for removing an impurity from a crude N-long-chain acyl amino acid product, wherein comprising: mixing the crude N-long-chain acyl amino acid product with a solvent, optionally stirring, and controlling the temperature T of the mixture system in a range of the melting point of a long-chain fatty acid to the melting point of the N-long-chain acyl amino acid, wherein the solvent is water, an organic solvent, or a mixed solution of water and an organic solvent; and after the temperature control in the system, separating the solid and liquid phases.

11. The method according to claim 10, wherein the solid-liquid separation is conducted under the action of centrifugation or pressure; preferably, the solid-liquid separation is promoted by using a solvent having a certain temperature as a medium that allows temperature T to be controlled in a range of the melting point of the long-chain fatty acid to the melting point of the N-long-chain acyl amino acid, and the solvent is water, an organic solvent, or a mixed solution of water and an organic solvent.

12. The method according to claim 11, wherein the solid-liquid separation meets one or more of the following conditions:
a, during the solid-liquid separation, the solvent used as the medium is in contact with the crude product, and under the action of centrifugation or pressure, the solvent removes the impurity to promote the separation;
b, the solvent used as the medium in the solid-liquid separation is provided by spraying;
c, the amount of the solvent used as the medium during the solid-liquid separation is greater than 0.5 times the mass of the crude N-long-chain acyl amino acid product;
d, the solid-liquid separation is conducted using an industrial centrifuge or a filter press, and preferably a filter centrifuge equipped with a filter screen or a filter cloth.

13. The method according to claim 11 or 12, wherein the temperature T of the solvent as the medium is present in a plurality of temperature stages, and preferably the temperature in a next stage is equal to or higher than the temperature in a previous stage;
preferably, the temperature in the first stage is controlled in a range of the melting point of the long-chain fatty acid to the melting point of the long-chain fatty acid + 15 °C, and the temperature in at least one subsequent stage is controlled in a range of the melting point of the long-chain fatty acid + 15 °C to the melting point of the N-long-chain acyl amino acid;
more preferably, the temperature in the first stage is controlled in a range of the melting point of the long-chain fatty acid to the melting point of the long-chain fatty acid + 10 °C, and the temperature in at least one subsequent stage is controlled in a range of the melting point of the long-chain fatty acid + 20 °C to the melting point of the N-long-chain acyl amino acid.

14. The method according to claim 11 or 12, wherein if water is used as the solvent in the preparation of the crude N-long-chain acyl amino acid product or the content of the long-chain fatty acid impurity in the crude N-long-chain acyl amino acid product is 10 wt% or greater, the temperature T of the solvent as the medium is present in a plurality of temperature stages, and the temperature in the first stage is controlled in a range of the melting point of the long-chain fatty acid to the melting point of the long-chain fatty acid + 6 °C, and the temperature in at least one subsequent stage is controlled in a range of the melting point of the long-chain fatty acid + 15 °C to the melting point of the N-long-chain acyl amino acid;
more preferably, the temperature in the first stage is controlled in a range of the melting point of the long-chain fatty acid to the melting point of the long-chain fatty acid + 3 °C, and the temperature in at least one subsequent stage is controlled in a range of the melting point of the long-chain fatty acid + 20 °C to the melting point of the N-long-chain acyl amino acid.

15. The method according to any one of claims 10-14, wherein after the first solid-liquid separation, n solid-liquid separations are conducted, wherein n is not less than 1, and preferably the temperature in a next solid-liquid separation is equal to or higher than the temperature in a previous solid-liquid separation;
each solid-liquid separation comprises: mixing a solid obtained from a previous solid-liquid separation with a solvent, optionally stirring, controlling the temperature Tn of the mixture system in a range of the melting point of the long-chain fatty acid to the melting point of the N-long-chain acyl amino acid, and conducting the solid-liquid separation, wherein the solvent is water, an organic solvent, or a mixed solution of water and an organic solvent; or
alternatively: mixing a solid obtained from a previous solid-liquid separation with a solvent, optionally stirring, controlling the temperature Tn of the system in a range of the melting point of the long-chain fatty acid to the melting point of the N-long-chain acyl amino acid, and conducting the solid-liquid separation, wherein the solid-liquid separation is promoted by using a solvent having a certain temperature as a medium that allows temperature Tn to be controlled in a range of the melting point of the long-chain fatty acid to the melting point of the N-long-chain acyl amino acid, and the solvent is water, an organic solvent, or a mixed solution of water and an organic solvent.

16. The method according to claim 15, wherein the temperature T in the first solid-liquid separation is controlled in a range of the melting point of the long-chain fatty acid to the melting point of the long-chain fatty acid + 15 °C, and the temperature Tn in at least one solid-liquid separation of the n subsequent solid-liquid separations is controlled in a range of the melting point of the long-chain fatty acid + 15 °C to the melting point of the N-long-chain acyl amino acid;
more preferably, the temperature T in the first solid-liquid separation is controlled in a range of the melting point of the long-chain fatty acid to the melting point of the long-chain fatty acid + 10 °C, and the temperature Tn in at least one solid-liquid separation of the n subsequent solid-liquid separations is controlled in a range of the melting point of the long-chain fatty acid + 20 °C to the melting point of the N-long-chain acyl amino acid.

17. The method according to claim 15, wherein three or more solid-liquid separations are conducted; the temperature T in the first solid-liquid separation is controlled in a range of the melting point of the long-chain fatty acid to the melting point of the long-chain fatty acid + 8 °C, the temperature Tn in at least one intermediate solid-liquid separation is controlled in a range of the melting point of the long-chain fatty acid + 8 °C to the melting point of the long-chain fatty acid + 18 °C, and the temperature Tn in the last solid-liquid separation is controlled in a range of the melting point of the long-chain fatty acid + 24 °C to the melting point of the N-long-chain acyl amino acid.

18. The method according to claim 15, wherein if water is used as the solvent in the preparation of the crude N-long-chain acyl amino acid product or the content of the long-chain fatty acid in the crude N-long-chain acyl amino acid product is 10% or greater, the temperature T in the first solid-liquid separation is controlled in a range of the melting point of the long-chain fatty acid to the melting point of the long-chain fatty acid + 6 °C, and the temperature Tn in at least one solid-liquid separation of the n subsequent solid-liquid separations is controlled in a range of the melting point of the long-chain fatty acid + 15 °C to the melting point of the N-long-chain acyl amino acid;
more preferably, the temperature T in the first solid-liquid separation is controlled in a range of the melting point of the long-chain fatty acid to the melting point of the long-chain fatty acid + 3 °C, and the temperature Tn in at least one solid-liquid separation of the n subsequent solid-liquid separations is controlled in a range of the melting point of the long-chain fatty acid + 20 °C to the melting point of the N-long-chain acyl amino acid.

19. The method according to any one of claims 10-18, wherein the crude N-long-chain acyl amino acid product is a commercially available N-long-chain acyl amino acid product;
alternatively, the crude N-long-chain acyl amino acid product described in any one of claims 7-9;
alternatively, an N-long-chain acyl amino acid product with a long-chain fatty acid percentage content by weight of 5% or greater;
alternatively, a crude N-long-chain acyl amino acid product prepared by a method comprising: (1) reacting a raw material comprising an amino acid and a base to give an amino acid salt solution; (2) adding a long-chain acid halide and optionally a base into the resultant amino acid salt solution to give an N-long-chain acyl amino acid salt; and (3) acidifying the resultant N-long-chain acyl amino acid salt; or
alternatively, a crude N-long-chain acyl amino acid product prepared by a method comprising: reacting an amino acid and/or a salt thereof with a long-chain acid halide in the presence of a base to give an N-long-chain acyl amino acid salt, acidifying the resultant N-long-chain acyl amino acid salt, gradually precipitating a solid, standing, separating solid and liquid phases, and optionally washing and drying to give the crude N-long-chain acyl amino acid product.

20. The method according to any one of claims 10-19, wherein the long-chain fatty acid is a saturated or unsaturated linear or branched fatty acid having 8-22 carbon atoms; the N-long-chain acyl group in the N-long-chain acyl amino acid is derived from the saturated or unsaturated linear or branched fatty acid having 8-22 carbon atoms; the amino acid in the N-long-chain acyl amino acid is derived from one or more of glycine, alanine, glutamic acid, sarcosine, aspartic acid, leucine, isoleucine, valine, threonine, proline, phenylalanine, arginine, and lysine; the organic solvent is an organic solvent in which the long-chain fatty acid and the N-long-chain acyl amino acid are slightly soluble, practically insoluble, or insoluble, wherein slightly soluble, practically insoluble, or insoluble refers to that the solubility of the long-chain fatty acid and the N-long-chain acyl amino acid in the organic solvent at 20 °C is less than 1 g/100 g, preferably less than 0.01 g/100 g, and more preferably less than 0.001 g/100 g.

21. The method according to claim 20, wherein the long-chain fatty acid is selected from one or more of octylic acid, capric acid, undecanoic acid, lauric acid, myristic acid, pentadecanoic acid, palmitic acid, stearic acid, oleic acid, linoleic acid, isostearic acid, coconut oil fatty acid, or palm oil fatty acid, preferably coconut oil fatty acid or lauric acid, and most preferably lauric acid;
correspondingly, the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
the amino acid in the N-long-chain acyl amino acid is derived from alanine, glycine, glutamic acid, sarcosine, arginine, or lysine, and preferably L-alanine.

22. A method for separating components in a solid mixture using the difference in melting point, comprising: (a) adding a solvent to the solid mixture, (b) after the addition of the solvent, controlling the temperature T of the system in a range of the melting point of a low-melting-point component to the melting point of a high-melting-point component, (c) after the temperature control in the system, separating the solid and liquid phases, wherein the solvent is a solvent in which the components to be separated are slightly soluble, practically insoluble, or insoluble, wherein slightly soluble, practically insoluble, or insoluble refers to that the solubility of the components to be separated in the solvent at 20 °C is less than 1 g/100 g, preferably less than 0.01 g/100 g, and more preferably less than 0.001 g/100 g; the boiling point of the solvent is greater than the melting point of the low-melting-point component, and the temperature T of the system is not higher than the boiling point of the solvent.

23. The method according to claim 22, wherein the difference between the melting points of the components to be separated is 10 °C or greater, preferably 20 °C or greater, and more preferably 30 °C or greater;
and/or, the percentage content by weight of the low-melting-point component is 50% or less, preferably 40% or less, and more preferably 30% or less.

24. The method according to claim 22 or 23, wherein the solid-liquid separation is conducted under the action of centrifugation or pressure; preferably, the solid-liquid separation is promoted by using a solvent having a certain temperature as a medium that allows temperature T to be controlled in a range of the melting point of the low-melting-point component to the melting point of the high-melting-point component, and the solvent is a solvent in which the components to be separated are slightly soluble, practically insoluble, or insoluble.

25. The method according to any one of claims 22-24, wherein the solid-liquid separation meets one or more of the following conditions:
a, during the solid-liquid separation, the solvent used as the medium is in contact with the mixture to be separated, and under the action of centrifugation or pressure, the solvent removes the low-melting-point component to promote the separation;
b, the solvent used as the medium in the solid-liquid separation is provided by spraying;
c, the amount of the solvent used as the medium during the solid-liquid separation is greater than 0.5 times the mass of the mixture to be separated;
d, the solid-liquid separation is conducted using an industrial centrifuge or a filter press, and preferably a filter centrifuge equipped with a filter screen or a filter cloth.

26. The method according to claim 24 or 25, wherein the temperature T of the solvent as the medium is present in a plurality of temperature stages, and preferably the temperature in a next stage is equal to or higher than the temperature in a previous stage;
preferably, the temperature in the first stage is controlled in a range of the melting point of the low-melting-point component to the melting point of the low-melting-point component + 10 °C, and the temperature in at least one subsequent stage is controlled in a range of the melting point of the low-melting-point component + 10 °C to the melting point of the high-melting-point component; and
more preferably, the temperature in the first stage is controlled in a range of the melting point of the low-melting-point component to the melting point of the low-melting-point component + 10 °C, and the temperature in at least one subsequent stage is controlled in a range of the melting point of the low-melting-point component + 20 °C to the melting point of the high-melting-point component.

27. The method according to claim 24 or 25, wherein the percentage content by weight of the low-melting-point component is 10%-40%, and preferably 15%-30%; the temperature T of the solvent as the medium is present in a plurality of temperature stages, and the temperature in the first stage is controlled in a range of the melting point of the low-melting-point component to the melting point of the low-melting-point component + 6 °C, and the temperature in at least one subsequent stage is controlled in a range of the melting point of the low-melting-point component + 15 °C to the melting point of the high-melting-point component;
more preferably, the temperature in the first stage is controlled in a range of the melting point of the low-melting-point component to the melting point of the low-melting-point component + 3 °C, and the temperature in at least one subsequent stage is controlled in a range of the melting point of the low-melting-point component + 20 °C to the melting point of the high-melting-point component.

28. The method according to any one of claims 22-27, wherein after the first solid-liquid separation, n solid-liquid separations are conducted, wherein n is not less than 1, and preferably the temperature in a next solid-liquid separation is equal to or higher than the temperature in a previous solid-liquid separation;
each solid-liquid separation comprises: mixing a solid obtained from a previous solid-liquid separation with a solvent, optionally stirring, controlling the temperature Tn of the mixture system in a range of the melting point of the low-melting-point component to the melting point of the high-melting-point component, and conducting the solid-liquid separation, wherein the solvent is a solvent in which the components to be separated are slightly soluble, practically insoluble, or insoluble; or
alternatively: mixing a solid obtained from a previous solid-liquid separation with a solvent, optionally stirring, controlling the temperature Tn of the system in a range of the melting point of the low-melting-point component to the melting point of the high-melting-point component, and conducting the solid-liquid separation, wherein the solid-liquid separation is promoted by using a solvent having a certain temperature as a medium that allows temperature Tn to be controlled in a range of the melting point of the low-melting-point component to the melting point of the high-melting-point component, and the solvent is a solvent in which the components to be separated are slightly soluble, practically insoluble, or insoluble.

29. The method according to claim 28, wherein the temperature T in the first solid-liquid separation is controlled in a range of the melting point of the low-melting-point component to the melting point of the low-melting-point component + 10 °C, and the temperature Tn in at least one solid-liquid separation of the n subsequent solid-liquid separations is controlled in a range of the melting point of the low-melting-point component + 10 °C to the melting point of the high-melting-point component;
preferably, the temperature T in the first solid-liquid separation is controlled in a range of the melting point of the low-melting-point component to the melting point of the low-melting-point component + 10 °C, and the temperature Tn in at least one solid-liquid separation of the n subsequent solid-liquid separations is controlled in a range of the melting point of the low-melting-point component + 20 °C to the melting point of the high-melting-point component.

30. The method according to claim 28, wherein three or more solid-liquid separations are conducted; the temperature T in the first solid-liquid separation is controlled in a range of the melting point of the low-melting-point component to the melting point of the low-melting-point component + 8 °C, the temperature Tn in at least one intermediate solid-liquid separation is controlled in a range of the melting point of the low-melting-point component + 8 °C to the melting point of the low-melting-point component + 18 °C, and the temperature Tn in the last solid-liquid separation is controlled in a range of the melting point of the low-melting-point component + 24 °C to the melting point of the high-melting-point component.

31. The method according to claim 28, wherein the percentage content by weight of the low-melting-point component is 10%-40%, and preferably 15%-30%; the temperature T in the first solid-liquid separation is controlled in a range of the melting point of the low-melting-point component to the melting point of the low-melting-point component + 6 °C, and the temperature Tn in at least one solid-liquid separation of the n subsequent solid-liquid separations is controlled in a range of the melting point of the low-melting-point component + 15 °C to the melting point of the high-melting-point component;
more preferably, the temperature T in the first solid-liquid separation is controlled in a range of the melting point of the low-melting-point component to the melting point of the low-melting-point component + 3 °C, and the temperature Tn in at least one solid-liquid separation of the n subsequent solid-liquid separations is controlled in a range of the melting point of the low-melting-point component + 20 °C to the melting point of the high-melting-point component.

32. A method for preparing an amino acid supramolecule, comprising the step of removing an impurity from a crude N-long-chain acyl amino acid product described in any one of claims 10-21, wherein a structural reconstruction occurs during the removal of the impurity.

33. An amino acid supramolecule prepared by the method according to claim 32.

34. The amino acid supramolecule according to claim 33, wherein the percentage content by weight of a long-chain fatty acid is 5% or less, preferably 3% or less, and most preferably 0.5%-3%;
and/or, the percentage content by weight of an N-long-chain acyl amino acid dipeptide is 3% or greater, preferably 5% or greater, more preferably 8% or greater, and most preferably 10% or greater.

35. An amino acid supramolecule, comprising a supramolecular structure self-assembled by an N-long-chain acyl amino acid and an N-long-chain acyl amino acid dipeptide, wherein the percentage content by weight of the N-long-chain acyl amino acid dipeptide is 3% or greater, preferably 5% or greater, more preferably 8% or greater, and most preferably 10% or greater.

36. The amino acid supramolecule according to claim 35, wherein the amino acid supramolecule is an amino acid supramolecule having a medium content of the dipeptide, and the percentage content by weight of the N-long-chain acyl amino acid dipeptide is 5% or greater and preferably 10% or greater and less than 15% by weight; or
alternatively, the amino acid supramolecule is an amino acid supramolecule having a high content of the dipeptide, and the percentage content by weight of the N-long-chain acyl amino acid dipeptide is 15% or greater, and preferably 20% or greater.

37. The amino acid supramolecule according to claim 35 or 36, wherein the percentage content by weight of the long-chain fatty acid is 5% or less, preferably 3% or less, and most preferably 0.5%-3%.

38. The amino acid supramolecule according to any one of claims 33-37, wherein the amino acid supramolecule has a characteristic ion peak in a range of 541-545 in a mass spectrum detected on a mass spectrometer AB4500 with a scanning mode Q1SCAN, an ionization mode ESI (-), and a scanning range m/z = 200-600;
and/or, the amino acid supramolecule has 3 or 4 group peaks in the retention time range of 30-45 min in a chromatogram detected on a high-performance liquid chromatograph equipped with an ultraviolet detector with a column of ODS-2 HYPERSIL C18 250 × 4.6 mm 5 µm, a wavelength of 210 nm, and a mobile phase of methanol:20 mmol/L potassium dihydrogen phosphate buffer (pH 3.0) = 70:30 (v/v).

39. The amino acid supramolecule according to any one of claims 33-38, wherein the amino acid supramolecule meets one or more of the following conditions: a, the solid powder of the amino acid supramolecule has a columnar, rod-shaped, thread-shaped, or rope-shaped micromorphology; b, the amino acid supramolecule has an initial melting temperature of 78 °C or higher and a final melting temperature of 87 °C or higher, and preferably an initial melting temperature of 80 °C or higher and a final melting temperature of 90 °C or higher, as measured by a capillary method; c, the amino acid supramolecule has a DSC peak temperature of 86 °C or higher, preferably 88 °C or higher, and more preferably 90 °C or higher; d, the sodium salt of the amino acid supramolecule has a number-average molecular weight of 5,000-250,000, preferably 10,000-150,000, and more preferably 15,000-100,000.

40. The amino acid supramolecule according to any one of claims 33-39, wherein the amino acid supramolecule meets one or more of the following conditions:
a, the N-long-chain acyl group in the N-long-chain acyl amino acid and the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
b, the amino acid in the N-long-chain acyl amino acid and the N-long-chain acyl amino acid dipeptide is derived from one or more of glycine, alanine, glutamic acid, sarcosine, aspartic acid, leucine, isoleucine, valine, threonine, proline, phenylalanine, arginine, and lysine, preferably alanine, glycine, glutamic acid, sarcosine, arginine, or lysine, and most preferably L-alanine;
c, the long-chain fatty acid is selected from one or more of octylic acid, capric acid, undecanoic acid, lauric acid, myristic acid, pentadecanoic acid, palmitic acid, stearic acid, oleic acid, linoleic acid, isostearic acid, coconut oil fatty acid, or palm oil fatty acid, preferably coconut oil fatty acid or lauric acid, and most preferably lauric acid.

41. The amino acid supramolecule according to any one of claims 33-40, wherein the N-long-chain acyl amino acid is N-lauroyl-L-alanine, the N-long-chain acyl amino acid dipeptide is N-lauroyl-L-alanyl-L-alanine, and the long-chain fatty acid is lauric acid.

42. An amino acid supramolecule salt, formed from the amino acid supramolecule according to any one of claims 33-41 and a base.

43. The amino acid supramolecule salt according to claim 42, wherein the base is selected from one or more of inorganic bases, organic amines, and basic amino acids; wherein the inorganic base is selected from one or more of sodium hydroxide, potassium hydroxide, sodium carbonate, and potassium carbonate, and preferably sodium hydroxide or potassium hydroxide; the organic amine is selected from amine and alkanolamine; the basic amino acid is selected from one or more of arginine, lysine, and histidine, preferably arginine or lysine.

44. Use of the amino acid supramolecule and/or the salt thereof according to any one of claims 33-43, in a cleaning composition, a washing composition, a cosmetic composition, or a healthcare composition;
or, as a surfactant or emulsifier.

45. Use of the amino acid supramolecule and/or the salt thereof according to any one of claims 33-43, in adsorbing oil stains or microorganisms, or in sterilizing, deodorizing or removing pesticide residues.

46. A cleaning composition, comprising the amino acid supramolecule and/or the salt thereof according to any one of claims 33-43, and preferably comprising an arginine salt or a lysine salt of the amino acid supramolecule.

47. The cleaning composition according to claim 46, wherein the cleaning composition is a detergent, a laundry liquid, a soap, a laundry powder, a dish detergent, a facial mask, a shampoo, a body wash, a facial cleanser, a makeup remover, a mouth wash, a shaving product, a hand sanitizer, a cleaning lotion, or a cleansing cream.

48. A toothpaste, comprising the amino acid supramolecule and/or the salt thereof according to any one of claims 33-43, and preferably comprising an arginine salt or a lysine salt of the amino acid supramolecule.

49. A cosmetic composition, comprising the amino acid supramolecule and/or the salt thereof according to any one of claims 33-43, and preferably comprising an arginine salt or a lysine salt of the amino acid supramolecule.
